# EUROPEAN PATENT SPECIFICATION

(11) **EP 3 665 140 B1**
(45) Date of publication and mention of the grant of the patent: **07.06.2023**
(21) Application number: 18844261.0
(22) Date of filing: 13.08.2018
(51) Int. Cl.: C05F 11/00, C05F 17/00, A23K 10/37, C05F 5/00, C05F 7/00, C02F 9/00, C02F 11/12, C02F 11/18, C02F 11/02, C05F 17/20, C05F 9/00, C05F 17/40, C05F 17/50, A23K 10/14, A23K 50/75, A23K 50/60, A23K 10/38, A23K 40/10, A23K 50/30, C05F 17/90

(54) **AGRICULTURAL ADMIXTURES**
LANDWIRTSCHAFTLICHE BEIMENGUNGEN
MÉLANGES AGRICOLES

(30) Priority: 11.08.2017 US 201762544579 P
(43) Date of publication of application: 17.06.2020
(73) Proprietor: California Safe Soil, LLC, McClellan, California 95652 (US)
(72) Inventor: MORASH, Daniel, McClellan California 95652 (US); LEJEUNE, Mark, McClellan California 95652 (US); ZICARI, Steve, McClellan California 95652 (US)
(74) Representative: J A Kemp LLP
(86) International application number: PCT/US2018/046571
(87) International publication number: WO 2019/033124

(56) References cited:
- WO-A1-2004/021797
- WO-A2-01/88961
- CN-A- 101 961 120
- CN-A- 104 892 037
- KR-A- 20140 054 732
- US-A1- 2015 315 466
- US-A1- 2016 159 705

## Description

### FIELD OF THE INVENTION

This invention relates to processes for manipulating biological recyclable streams and for blending recyclable streams and recyclable minerals to obtain agricultural admixtures, and the compositions produced thereby as disclosed in the claims.

### BACKGROUND

In the United States, food production uses approximately 50% of the land, and utilizes 80% of the total fresh water consumed. About 40% of total food production, however, goes as waste (Gunders, D., "Wasted: How America Is Losing Up to 40 Percent of Its Food from Farm to Fork Landfill," NRDC Issue Paper IP: 12-06-B (August 2012)), which is equivalent to $200 billion each year. While maximizing the efficiencies in the U.S. food system from the farmto-table draws much public attention, productive uses of food waste are poorly developed.

Soil organic matter is negatively impacted by cultivation and/or extended periods without a vegetative cover, which can decrease the content of organic matter below the natural or virgin levels for a given locality. The exhaustion of organic matter in the soil is a serious threat to limited agricultural resources. Global food production relies on fertile soils (Lal, et al., "Climate Strategic Soil Management," Challenges, 5:43-74 (2014); Blanco-Canqui, et al. "Principles of Soil Conservation and Management," Springer, Netherlands (2008)), which are a finite resource, requiring protection and efficient use by farmers producing food sources for animal and human consumption. Typical animal feed is sourced from corn, hay, alfalfa, soy, rice, sorgum, wheat, and oats. Animal feed is typically supplemented with peanuts, soybeans, corn gluten, and cottonseed to increase the feed protein content.

WO 2004/021797 describes a method for recovering peptides/amino acids and oil/fat from one or more protein-containing raw materials.

US 2015/315466 and US 2016/159705 describe processes and systems for converting fresh food waste into nutrient rich hydrolysates and particulate compositions.

CN 104 892 037 describes method for producing water-soluble fish protein powder organic fertilizer by using fish as a raw material.

WO 01/88961 describes a process for recovering bone and oil from animal byproducts.

KR 2014 0054732 describes a method and arrangement for manufacturing functional feed and liquid fertilizer from food waste by using saccharization-decomposition processing apparatus.

CN 101 961 120 describes a production method of concentrated sweet potato clear juice with high dietary fiber.

### SUMMARY

The scope of the invention is defined by the claims. Any aspects and examples of the present disclosure which do not fall under the scope of the appended claims do not form part of the invention and are merely provided for illustrative purposes.

The ability to process and to manage processing of one or more biological recyclable streams and to combine the processed products to yield agricultural admixtures affords numerous benefits. This disclosure features methods and processes for producing agricultural admixtures by combining biological recyclable streams, or by combining selected processed (*e.g*., incubated) biological hydrolysates and/or processed particles obtained from the one or more biological recyclable streams. In some aspects, the biological recyclable streams can include or exclude: fresh food recyclables (including fruits, vegetables, meat, fish, delicatessen, bakery and diary recyclables), fish processing recyclables, blood meal, bakery recyclables, distiller's grain, spent poultry, eggs, orange peels, spent tea leaves, banana peels, pomace, hulls, and culled fruits and/or vegetables. In some aspects, the biological recyclable stream does not include spent poultry and/or poultry recyclable products. This disclosure features an effective way to combine two or more selected biological recyclable streams and/or the hydrolysates and/or particles from processed streams to obtain agricultural admixtures which enhance plant or animal health and growth. In some aspects, this disclosure relates to systems, methods, and compositions for processing selected biological recyclable streams before they become putrescent and/or toxic, and converting those selected biological recyclable streams into valuable compositions for nourishment of plants and animals. In some aspects, the compositions produced by the methods of this disclosure are in liquid form, in concentrated liquid form, or in solid form. In some aspects of this disclosure, the compositions may be produced from multiple biological recycled streams, introduced at different steps in the production, including different steps of the enzymatic digestion process. In some aspects of this disclosure, the compositions may be produced from multiple by-products, thereby recycling al1 the biological recyclable feedstocks into useful components including plant fertilizer and animal feed products.

The present invention provides a process for producing an agricultural admixture from a selected biological recyclable stream, the process comprising the steps of:
(a) providing a biological recyclable stream using a collection system;
(b) grinding the biological recyclable stream using a first grinder and optionally a second grinder to produce a ground biological slurry;
(c) adding to said ground biological slurry one or more selected enzymes;
(d) increasing the temperature of the ground biological slurry from ambient temperature to at least one temperature between about 35 °C and about 60 °C (about 95 °F and about 140 °F) and incubating the first ground biological slurry under constant agitation and shear at two or more temperatures between about 35 °C and about 60 °C (about 95 °F and about 140 °F), thereby producing an incubated first biological slurry comprising first incubated biological particles and a first incubated biological hydrolysate;
(e) pasteurizing the incubated ground biological slurry to kill pathogens;
(f) separating the first incubated hydrolysate into a first incubated biological hydrolysate and first incubated biological particles using one or a plurality of size-based separation methods; and
(g) reducing the fat content of the pasteurized first incubated hydrolysate to a range of between 1 to 4 wt% by tricanter centrifugation to form a centrifuged biological hydrolysate.
The present invention further provides the centrifuged biological hydrolysate produced by said process.

The process of the present invention may further comprising the steps of: (i) stabilizing the centrifuged biological hydrolysate to form a stabilized aqueous hydrolysate; and (ii) emulsifying the stabilized aqueous hydrolysate to form an emulsified agricultural admixture, and optionally adding a dispersant to the emulsified agricultural admixture. Said process may further comprise the step of concentrating the emulsified agricultural admixture; optionally wherein concentrating the liquid agricultural admixture is performed using a vibratory filter, vacuum drum, vacuum evaporator, drum dryer, spray dryer, paddle dryer, rotary dryer, or extruder. The present invention also provides the emulsified agricultural admixture, optionally a concentrated emulsified agricultural admixture, prepared by said processes.

The process of the present invention may further comprise the step of separating the first incubated biological particles into dewatered biological particles and a recycled liquid fraction. The present invention also provides the dewatered biological particles formed by said process.

The present invention further provides an agricultural admixture of the present invention, wherein the agricultural admixture is produced by grinding a biological recyclable stream to produce a ground biological slurry, heating and incubating the ground biological slurry with one or more selected enzymes with constant agitation and shear, pasteurizing the incubated mixture to produce a biological hydrolysate, reducing the fats content in the biological hydrolysate aqueous phase to a range of 1 to 4 wt%, and stabilization of the aqueous phase by adding a stabilizer selected from an inorganic acid, an organic acid, an inorganic preservative, or an organic preservative, to produce a stabilized agricultural admixture.

The process of the present invention may further comprise the steps of: (h) drying the centrifuged biological hydrolysate to form a dried, solid biological slurry; (i) milling the solid biological slurry to form a powdered, dried biological slurry or pelletizing the dried, solid biological slurry to form dried biological slurry pellets; and (j) optionally combining or blending the powdered, dried biological slurry or dried biological slurry pellets with a carbohydrate recyclable stream to form animal provender. The present invention further provides a method of increasing animal weight, or increasing the conversion rate of animal provender into animal weight, the method comprising providing to the animal a formulation comprising the animal provender produced by said process.

In some aspects of this disclosure the stabilized aqueous hydrolysate may also be concentrated and/or blended with an additive.

In some aspects of this disclosure it has been found that Animal Provender (I), in addition to being made from sustainable biological recyclables surprisingly results in a higher mass conversion rate of feed to animal weight compared to a standard feed product, with an observed increase in animal weight when used as a feed relative to control. The inventors have further surprisingly discovered that recycling food processed with the methods described herein into animal provender administered to animals results in healthier animals (*e.g*., exhibiting reduced diarrhea, and/or lower glucose levels), and faster growing, compared to conventional animal diets.

In one aspect, concentrating the liquid hydrolysate is performed using filtration or evaporation. In some aspects, the steps (e), (f), and (g), described above can be performed in any order.

According to the present invention, the fat content of the pasteurized first incubated hydrolysate is reduced by centrifugation to a range of between 1 to 4 wt% by tricanter centrifugation to form a centrifuged biological hydrolysate. The centrifuged biological hydrolysate may be added to the biological slurry before drying to modulate the fat content in the resulting mixture. In some aspects, the centrifuged oil is further separated into a food unuseable oil stream and a food useable oil stream. The food useable oil stream can be used as Animal Provender (III), and the food unuseable oil stream can be used in the production of biofuels. When the food unuseable oil stream is used in the production of biofuels, the food unuseable oil stream can be refined by distillation into fuels. In some aspects, the centrifuged oil comprises fatty acids, triglycerides, triglycerol, and/or fatty acid esters.

In some aspects, when the first incubated hydrolysate is separated into a first incubated biological hydrolysate and first incubated biological particles using one or a plurality of size-based separation methods, the size-based separation method comprises the use of a screen, mesh or separator to remove undigested material, *e.g*., a coarse screen. In some aspects of this disclosure, a second screen, mesh or separator may be used alone or in combination with the first separation method, where the second separation method is used to remove particles too large to fit through drip lines or other liquid transport lines, *e.g.,* a fine screen, or both. The separation steps may, in some aspects, be performed using screens such as vibratory screens.

In some aspects, an anti-caking agent and/or antioxidant is added to any of the Animal Provenders described herein at or before the final step in the process. In some aspects, an anti-caking agent and/or antioxidant is added to the dried, solid biological slurry.

In some aspects, the stabilization step (D) comprises the addition of a stabilizer selected from: inorganic acid, organic acid, organic preservative, and inorganic preservative.

In some aspects, the emulsification step (E) comprises the use of a high-shear mixer.

In some aspects, the first incubated biological particles are dewatered. In some aspects, the first incubated biological particles are dewatered using a screw press, belt filter, or hydraulic press to form separated dewatered biological particles and a recycled liquid fraction. In some aspect, the recycled liquid fraction can be added to any of the liquid compositions described herein. The dewatered biological particles can be used as a compost, biofuel source, or as Animal Provender (IV). In some aspects, the compost can be composted with a mineral. In some aspects, the mineral can be mined basalt. The basalt compost can be used as a high mineral content fertilizer. In some aspects, the basalt compost can be combined with the agricultural admixtures described herein, to be used as a fertilizer.

In some aspects, the emulsified hydrolysate from one production batch can be stored and blended in one or more storage tanks with one or more circulation pumps to form an agricultural admixture.

In some aspects, the process can further include processing a second or more biological recyclable streams using the methods described for the first recyclable stream. The products formed from the second or more biological recyclable streams can be added to the products of the first recyclable stream at any point in the process.

In one aspect, the method of producing the agricultural admixtures comprises grinding a first selected biological recyclable stream to form a first ground biological slurry, heating and incubating the first ground biological slurry with one or more enzymes with constant agitation and shear, and pasteurizing the incubated first ground biological slurry to produce a first pasteurized ground biological slurry for use in an agricultural admixture. In some aspects, the method further comprises grinding a second selected biological recyclable stream to form a second ground biological slurry, heating and incubating the second ground biological slurry with one or more enzymes with constant agitation and shear, and pasteurizing the incubated ground biological slurry to produce a second pasteurized ground biological slurry for use in an agricultural admixture. In some aspects, the method also comprises mixing the first ground biological slurry and the second ground biological slurry to obtain a blended agricultural admixture. During incubation, the one or more enzymes release nutritional components from the biological recyclable stream by digesting proteins, carbohydrates (such as sugars, starches, pectin and/or cellulosic materials), and/or fats and oils in the biological recyclable stream to produce, in one aspect, an incubated biological hydrolysate rich in nourishment, comprising, for example, amino acids, simple sugars, fatty acids, triglycerides, antioxidants, vitamins, polypeptides, fertilizers, and minerals. In some aspects, the incubated biological hydrolysate can be emulsified or homogenized using an ultra-high shear grinder to produce a stably emulsified agricultural admixture, useful as a fertilizer and soil amendment, or animal provender. The incubated biological hydrolysate can be filtered or evaporated, to produce a concentrated liquid fertilizer or animal provender, or dried to yield a dry agricultural admixture which can be used as either fertilizer or animal provender.

In one aspect this disclosure describes methods of and systems for processing two or more selected biological recyclable streams to form incubated biological hydrolysates from each stream, and combining the incubated biological hydrolysates to obtain agricultural admixture compositions, *e.g*., combined incubated biological hydrolysates, concentrates, dried cakes, or combined incubated biological particles. The agricultural admixtures are useful for providing nourishment and minerals for plants and soil microbes, and/or for animals. This disclosure also describes the admixtures and hydrolysates obtained from those processes and systems. The methods of this disclosure permit recycling of biological recyclable streams which would otherwise be disposed of in landfills, or other similar facilities for wasting said biological recyclable streams.

In one aspect, the incubated biological hydrolysate can comprise one or more phases. In some aspects, the incubated biological hydrolysate can comprise an aqueous phase and an oil phase. In some aspects, the incubated biological hydrolysate oil phase can further comprise fatty acids, biodiesel oils, and/or food oils. The aqueous phase, oil phase, and optionally the biological particles can be separated by a three phase separator by the processes described herein. According to the present invention, the three phase separator is a tricanter centrifuge. In some aspects, the tricanter centrifuge is a Flottwegg Separator (Germany). In some aspects, the centrifugal three phase separator is a Peony (China) Centrifuge. In some aspects, the centrifugal three phase separator is an Alfa Laval (Sweden) centrifuge. In some aspects, the incubated biological hydrolysate can be separated using a hydrocyclone to separate the particles from the liquids. The hydrocyclone can be a Sand Separator from Netafim (USA), or a John Deer F 1000 Sand Separator (Deer, USA).

When used as a fertilizer and soil amendment, the agricultural admixtures of this disclosure provide higher crop yields by, for example, providing nourishment to plants in the form of nutrients and increasing organic matter in the soil and by supporting the growth of beneficial soil organisms. In some aspects, agricultural admixtures of this disclosure increase crop yields while also permitting reduction of the use of nitrate or ammonia based fertilizers, which lowers nitrate runoff into lakes and streams and lowers potent greenhouse emissions (according to the EPA, N₂O, or nitrous oxide, given off from nitrate or ammonia based fertilizer, is 300 times as powerful a greenhouse gas as carbon dioxide (IPCC (2007) Climate change 2007: The Physical Science Basis. S. Solomon et al., Eds. Cambridge University Press, Cambridge, UK)). Accordingly, the use of the agricultural admixtures of this disclosure to replace some or all nitrate or ammonia based fertilizers can mitigate problems associated with the use of chemical fertilizers, such as nitrate run-off, GHG emissions, and/or reduction of organic matter in the soil. In addition, the agricultural admixtures of this disclosure also increase plant vigor and root system growth, increasing uptake of nitrates by plants and thereby further reducing the runoff of nitrate or ammonia based fertilizers into the water supply and increasing water and fertilizer use efficiency for the farmer. (*See, e.g.,* Dara et al., Evaluating a Recycled Food Waste-Based Liquid Compost in Conventional California Strawberries, Agricultural Research & Technology Open Access Journal Vol. 12(2) (October 2017), 1-3)

As used herein, the term "crop yield" refers to a measurement of the amount of a crop that was harvested per unit of land area. Crop yield can also refer to the actual seed generation from the plant. The unit by which the yield of a crop is measured is kilograms per hectare, bushels per acre, or tons per acre.

Water use efficiency is of increasing concern, due to the impact of drought and climate change. The agricultural admixtures of this disclosure can also increase water retention through the build-up of organic matter in the soil, and improve soil tilth (including the formation and stability of aggregated soil particles, moisture content, degree of aeration, rate of water infiltration, and drainage). In addition, the agricultural admixtures of this disclosure produce high crop yields at relatively lower costs, improve the quality of crops, and promote crop resistance to pests, diseases and plant stresses (such as salt, poor soil, heat or drought).

Discarded biological recyclable streams are a waste of resources and a large source of greenhouse gas emissions (*e.g*., carbon in the form of CO₂ (carbon dioxide) or CH₄ (methane) which, according to the EPA, is 23 times as potent a greenhouse gas as carbon dioxide). Biological recyclable streams can quickly begin to decompose, creating a safety and public nuisance problem, thereby making it difficult, if not impossible to make valuable use of biological recyclable streams. This disclosure features the use of varied biological recyclable streams to make agricultural admixtures and animal provender, which reduce the greenhouse gas emissions associated with decomposing biological wastes. In some aspects, the processed biological recyclable stream yields lower methane emissions than unprocessed biological recyclable streams. In another aspect, the agricultural admixtures produced by the methods of this disclosure facilitate the growth of beneficial microbial populations in the soil. Increased microbial activity increases the sequestering of carbon in the soil, thereby improving the sustainability of farm practices. The nutrients in the agricultural admixtures described herein stimulate microbial life in the soil. Detritus from microbial life in the soil is the basis for long term carbon sequestration in the soil (Kallenbach, C. et al., Nature Comm., 7: 13630 (2016); Lehmann, J., Nature, 528:60-69 (2015)). In some aspects, this disclosure relates to systems, compositions, and methods for collecting and processing biological recyclable streams before they become putrescent, and converting the biological recyclable streams into valuable agricultural admixtures. In some aspects, this disclosure relates to measuring increased carbon sequestration in the soil following application of the admixture described herein. In some aspects, soil carbon sequestration can be measured by monitoring C¹³ or C¹⁴ in CO₂ respirated from the soil. In some aspects, the C¹³ or C¹⁴ in CO₂ can be detected by GC-MS. In some aspects, the GC-MS system can be an Agilent 5977B GC/MSD mass spectrometry system. In some aspects, long-term biological stability of soil organic carbon can be measured by adding a C¹³-labelled substrate mixture (e.g. 1:1 glutamic acid:glucose at 25 atom % and 50 mg C per g soil) to a sample of soil treated with the agricultural admixtures described herein, and then incubating for 3 months. Analysis of the labelled substrate enables analysis by a standard isotope mixing model (described in Ineson, P., Cotrufo, M. F., Bol, R., Harkness, D. D. & Blum, H. Quantification of soil carbon inputs under elevated CO2:C-3 plants in a C-4 soil, Plant Soil, 187, 345e350 (1996)) to determine the amount of previously formed carbon vulnerable to decomposition by an active microbial community. In some aspects, chemical stability of accumulated soil organic carbon can be measured with an acid hydrolysis fractionation.

By recycling biological recyclable streams that would otherwise rot and ferment, releasing prodigious amounts of greenhouse gases, as well as toxic liquids and gases (C₂H₅OH or ethanol, a plant pathogen, and H₂S, (hydrogen sulphide, a toxic gas) and other related effluent by-products of rotting and fermenting, the methods of this disclosure fully utilize the nutritional content of biological recyclable streams and drastically reduce waste organic matter and its attendant risk of harbored pathogens, while providing significant benefits to the soil or animal provender. The methods described herein avoid the possibility of contamination by preventing the introduction of disease-causing pathogens (including or excluding *salmonella, E. coli* and *listeria,* which may be present in the input biological recyclable stream) into the soil when used as a fertilizer. (Pandey, P. et al., J. Cleaner Prod., 1-9 (2015)).

In some aspects of this disclosure, the collection system of this disclosure captures the nutritional value of the biological recyclable stream using a system (which minimizes the final discarded waste) that does not allow biological recyclable streams to become putrescent (non-putrescent biological recyclable streams). Putrescence can be measured by smell-odor tests, or analysis by GC-MS (gas chromatography) of the headspace above the test sample, or by a handheld odor monitor (*e.g*., Kanomax OMX-TPM or Shinyei OMX-SRM handheld odor meter). In some aspects of this disclosure, supermarket staff separate some forms of biological recyclable streams (produce, meat, fish, delicatessen, bakery and dairy) from other recyclable streams. In some aspects, the biological recyclable stream can be from winemakers, olive oil manufacturers, vegetable processors, nut processors, fruit processors, coffee processors, yogurt manufacturers, supermarkets, food wholesalers, food processors, butcher shops, and institutional sources. In some aspects, the institutional sources can be where food is freshly prepared and excess food is discarded as a biological recyclable stream. In some aspects, the institutional source can be from sports arenas, hospitals, hotels, and cafeterias. In some aspects, the coffee processors can provide coffee grounds after preparation of coffee. In some aspects, yogurt manufacturers can provide whey recyclable product. The whey recyclable product can comprise lactic acid which can be used as an in-situ acid source during the incubation steps described herein. In some aspects of this disclosure, commercial bakeries provide isolated baked goods as a biological recyclable stream. In some aspects of this disclosure, winemakers and vineyards provide culled grapes and/or isolated grape pomace as a biological recyclable stream. In some aspects of this disclosure, olive oil manufacturers provide culled olives or isolated olive pomace as a biological recyclable stream. In some aspects of this disclosure, processed food manufacturers provide nut or legume hulls, isolated tomato and/or culled vegetable recyclable matter as a biological recyclable stream. In some aspects, the biological recyclable stream can comprise okara (soy pulp). The soy pulp can increase the relative nitrogen content in the hydrolysate product. In some aspects, the biological recyclable stream can comprise dairy products. Dairy products can be sourced from a diary or a supermarket as packaged dairy. The packaged dairy can be de-packaged before use as a biological recyclable stream.

In some aspects of this disclosure, rendering plants can provide poultry feathers, beaks, and feet (poultry recyclable stream) and/or bone meal. In some aspects, fish processing plants can provide fish products as a fish recyclable stream. Fish recyclables can include or exclude: skin, viscera, fish heads, fish tails, fish hydrolysate, and carcasses (fish bones). The fish recyclables can increase the relative amount of organic nitrogen in the hydrolysate. Ethanol plants can produce distiller's grains, which when added to the processes described herein can increase the carbohydrate content in the hydrolysate.

In some aspects the biological recyclable streams may include or exclude any of the foregoing biological recyclable streams.

In some aspects, the biological recyclable recyclable stream may include or exclude culled fruits, nuts or vegetables containing oils, for example, culled nut or, cucurbitaceae seeds. In some aspects the culled nuts may include or exclude almonds, beech nuts, brazil nuts, cashews, hazelnuts, macadamia nuts, mongongo nuts, pecans, pine nuts, pistachios, peanuts, and walnuts. In some aspects, the biological recyclable streams can include culled citrus containing oil, for example, it can include or exclude grapefruits, lemons, oranges, pomelos, and limes. In some aspects, the cucurbitaceae seeds can include or exclude bitter gourds, bottle gourds, buffalo gourds, butternut squash seeds, pumpkin seeds, and watermelons. In some aspects, the other culled recyclable plants containing oils can include or exclude amaranth, apricots, apple seeds, argan, avocados babassu, ben, borneo tallow nuts, cape chestnuts (also called yangu), carob pods (algaroba), cocoa, cocklebur, cohune coriander seeds, date seeds, dika, false flax, grape seed, hemp, kapok seeds, kenaf seeds, lallemantia, mafura, marula, meadowfoam seeds, mustard, niger seeds, poppyseeds, nutmeg, okra seeds, papaya seed ils perilla seeds, persimmon seeds, pequi, pili nuts, pomegranate seeds, poppyseeds, pracaxi, virgin pracaxi, prune kernels, quinoa, ramtils, rice bran, shea, sacha inchi, sapote, seje, tea seeds (camellia), thistle, tigernut (or nut-sedge), tobacco seeds, tomato seeds, and wheat germoil. In some aspects, the biological recyclable stream can include or exclude copaiba, jatropha, milk bush, nahor, paradise, petroleum nuts, or pongamia.

In some aspects, the agricultural admixtures described herein can be further mixed with organic fertilizers to produce a synergistic effect of the organic fertilizers and the agricultural admixtures described herein in improving crop yields and organic soil content. The organic fertilizers can include or exclude bone meal, blood meal, feather meal, or manure, for example, chicken manure, bird guano, biosolids (treated solids from wastewater treatment plants), cow manure, green waste compost, or combinations thereof. It was surprisingly discovered that the processed agricultural admixtures described herein when mixed with an organic fertilizer affords pelletization of the combined product and/or results in faster breakdown of the organic fertilizer into nutrients to enhance plant and/or crop growth rates and crop yield.

In some aspects of this disclosure, the biological recyclable stream is placed in insulated and/or airtight totes and/or buggies that keep cold the biological recyclable streams that supermarkets, food processors, food wholesalers, bakeries or other vendors or manufacturers no longer offer for sale. For example, non-putrescent food recyclables, culled vegetables, or pomace can be stored and transported in insulated and/or airtight totes and/or buggies.

In some aspects the insulated totes and/or buggies used to collect the food can be double-walled. These insulated containers improve store hygiene, and are easy for store staff to use, which promotes a high compliance rate among store staff and a low rate of contaminants in the biological recyclable stream. In some aspects, the insulated totes and/or buggies can be oxygen-deficient so as to decrease the rate of decomposition. In some aspects, the insulated totes and/or buggies can be vacuum sealed, and/or sealed under modified atmosphere packaging (MAP) where the CO₂ and O₂ and ethane levels are adjusted to prevent further spoilage of the enclosed product, to reduce the amount of oxygen in the totes and/or buggies. In some aspects of this disclosure, the collection system of this disclosure can include one or more of the following additional steps: collecting the biological recyclable stream at frequent intervals (*e.g*., once or twice per day or twice, three times, four times, five times, or six times per week); collecting the biological recyclable stream in refrigerated trucks; minimizing the distance the biological recyclable stream must travel to arrive at the processing facility described in this disclosure; and immediately processing or refrigerating the biological recyclable stream at the processing facility. The processing technology in this disclosure is modular, allowing the construction of facilities in urban areas and near sources of biological recyclable streams in addition to supermarkets, such as food processing facilities, fresh food distributors, institutional food preparation facilities, fresh green recyclables from farms, or other viable sources of biological recyclable streams (the "collection system").

In some aspects, the step of adding to the first ground biological slurry one or more selected enzymes is done before or during the step of increasing the temperature of the first ground biological slurry from ambient temperature to a temperature between about 35 °C and about 60 °C (about 95 °F and about 140 °F) and incubating the first ground biological slurry. In some aspects one or more selected enzymes may be added after the first ground biological slurry is heated to a temperature between about 35 °C and about 60 °C (about 95 °F and about 140 °F). In some aspects, the one or more selected enzymes can be added as powder or liquid form. In some aspects, the liquid form of the one or more selected enzymes can be pre-heated, and/or accelerated with the co-addition of one or more cofactors. In some aspects, the one or more selected enzymes is added with one or more cofactors. In some aspects, the cofactor can include or exclude metal cations and coenzymes. The metal cations can include or exclude: cupric, ferrous, ferric, catalase, magnesium, manganese, molybdenum, nickel, and zinc. the coenzymes can include or exclude vitamin and vitamin derivatives of: thiamine pyrophosphate, thiamine, NAD+ and NADP+, niacin, pyridoxal phosphate, pyridoxine, methylcobalamin, vitamin B12, cobalamine, biotin, coenzyme a, pantothenic acid, tetrahydrofolic acid, folic acid, menaquinone, vitamin K, ascorbic acid, flavin mononucleotide, riboflavin, and coenzyme F420.

In some aspects the first temperature of the incubated first ground biological slurry may be 35, 35.6, 36.1, 36.7, 37.2, 37.8, 38.3, 38.9, 39.4, 40, 40.6, 41.1, 41.7, 42.2, 42.8, 43.3, 43.9, 44.4, 45, 45.6, 46.1, 46.7, 47.2, 47.8, 48.3, 48.9, 49.4, 50, 50.6, 51.1, 51.7, 52.2, 52.8, 53.3, 53.9, 54.4, 55, 56.1, 56.7, 57.2, 57.8, 58.3, 58.9, or 59.4 °C (95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, or 139 °F), or any range in between any two of the recited temperatures. In some aspects a second temperature of the incubated first ground biological slurry may be 37.8, 38.3, 38.9, 39.4, 40, 40.6, 41.1, 41.7, 42.2, 42.8, 43.3, 43.9, 44.4, 45, 45.6, 46.1, 46.7, 47.2, 47.8, 48.3, 48.9, 49.4, 50, 50.6, 51.1, 51.7, 52.2, 52.8, 53.3, 53.9, 54.4, 55, 56.1, 56.7, 57.2, 57.8, 58.3, 58.9, 59.4, 60, °C (100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140°F), or any range in between any two of the recited temperatures.

In some aspects, the method in (h) (A) (which may or may not include steps (f) or (g)), may use a drum dryer (such as may be manufactured by Andritz, Drum Drying Systems, Buflovak, GL&V or Phoenix Drum Drying), a spray dryer (such as may be manufactured by Pulse Combustion Systems or GEA), extrusion dryers (such as may be manufactured by Diamond America or Coperion), or a rotary kiln (such as may be manufactured by Feeco) to produce a dried hydrolysate. In some aspects, in the method in (g) (B), the dried hydrolysate may be milled into a powdered form using a common fitz mill, or pelletized using a common pelletizer to form dried hydrolysate pellets for animal provender. The powder or pellets in (g) (B) may or may not include the addition of stabilizing agents and/or anti-caking agents. In some aspects, in the method in (g) (C), the animal provender may be blended with other animal feed ingredients, to be customized to specific applications.

In some aspects, step (f) results in reducing the number of particles in the liquid biological hydrolysate. In some aspects, step (f) is performed using selective size separation methods. In some aspects, selective size separation is performed using a centrifugal separation system. In some aspects, the selective size separation methods use a reusable filter or mesh. In some aspects the selective size separation is performed by serial filtration through a coarse screen followed sometime later by filtration through a fine screen. In some aspects the filter or mesh is made of metal, plastic, glass or ceramic.

In some aspects, the first incubated biological hydrolysate comprises one or more phases. The first incubated biological hydrolysate can comprise an oil phase, a particulate phase, and an aqueous phase. In some aspects, the method in (g) of separating is performed using a three phase separator. In some aspects, the three phase separator is a centrifugal separator. The three phase separator can separate all or part of a heavy liquid, light liquid and solid phase, per their different densities and mutually insolubility. The solid phase differentially sediments in a centrifugal force field or gravity force field, which causes the solid particles in the liquid to deposit. In some aspects, the centrifugal three phase separator is, for example, a Flottweg Separator. In some aspects, the centrifugal three phase separator is, for example, a Peony Centrifuge. The three phase separator operates at 1,000 - 7,000 RPM and processes 5 to 50 gallons per minute. In some aspects, the three phase separator processes 5 to 50 gallons per minute. In some aspects, the three phase separator processes 15 gallons per minute. In some aspects, multiple three phase separators can be placed in series or in parallel. When multiple three phase separators are placed in parallel, the first incubated biological hydrolysate can be processed faster with a lower process time per separator than if the first incubated biological hydrolysate were processed with a single three phase separator. In some aspects, the centrifugal three phase separator is, for example, an Alfa Laval centrifuge. In some aspects, the incubated biological hydrolysate can be separated using a hydrocyclone to separate the particles from the liquids. The hydrocyclone can be a Sand Separator from Netafim (USA), or a John Deer F1000 Sand Separator (Deer, USA).

In some aspects, the method further comprises (h) (D) stabilizing and preserving the incubated biological hydrolysate, using a stabilizer selected from: inorganic acid, organic acid, organic preservative, inorganic preservative. The stabilizing and preserving step may take place either before or after the separating step (f). In some aspects, the method comprises (h) (E) emulsifying the stabilized incubated biological hydrolysate using an ultra-high shear mixer and/or organic or inorganic emulsifiers to produce a stabilized emulsified hydrolysate. In some aspects, the emulsification step may include adding organic and/or inorganic dispersants to act as surface active ingredients in the stabilized emulsified hydrolysate. In some aspects, the method comprises (h)(E)(ii) blending the stabilized emulsified hydrolysate in one or more storage tanks using one or more circulation pumps with other liquid fertilizer ingredients which may include or exclude vitamins, pesticides, trace inorganic minerals, wood ash, gypsum salts, Epsom salts, worm castings, colorants, fragrances, and viscosity modifiers.

In some aspects, the method further comprises (h) (E)(i) concentrating the liquid hydrolysate through vibratory filtration equipment (such as may be manufactured by New Logic) or vacuum evaporation equipment (such as may be manufactured by Buflovak or Vobis). In some aspects, the method further comprises (h)(E)(ii), blending the concentrated liquid hydrolysate with other liquid fertilizer ingredients or liquid animal provender ingredients.

In some aspects of this disclosure, the method further comprises further processing the screened biological particles from step (f), using a separation method, e.g., using a screw press, belt press, or hydraulic press to produce an optionally recyclable liquid fraction, and a dewatered biological particle fraction comprising step. The dewatered biological particle fraction can be used as a compost feedstock for green waste compost, basalt compost, other composts, as well as biofuel or animal provender in an agricultural admixture. The liquid fraction can, in some aspects, be added to the biological hydrolysate from the biological slurry.

In some aspects of this disclosure, the finished product from the stabilized emulsified hydrolysate is homogeneous. In some aspects, the homogeneity can be measured by viscosity measurements using a rotational viscometer (*e.g*., Thermo Scientific^{™} HAAKE^{™} Viscotester). In some aspects, the viscosity of three samples of the finished product can be within experimental error of each other. In some aspects, the biological particles comprise bone, cellulose, solidified or semi-solidified fats, nut shells, fish scales, teeth, inorganic minerals, keratin-containing species, or combinations thereof. In some aspects, the keratin-containing species is selected from: beaks, feathers, claws, or hair. In some aspects the incubated hydrolysate is separated from the incubated particles using one or more screens. In some aspects, the incubated hydrolysate is separated from the incubated particles by centrifugation, settling, the use of a hydrocyclone, a rotaspiral drum screen, or a horizontal belt filter. In some aspects, the separated biological particles can be processed as a second biological recyclable stream.

In some aspects, the first grinder and second grinder are not in fluidic communication with each other. In some aspects, the first grinder and second grinder are in fluidic communication. In some aspects, the first grinder is not in fluidic communication with the incubation vessel.

In some aspects, the steps (a)-(b) can be done at a different site, physically separated, from the site where steps (c)-(h) are performed. In some aspects, the two different sites are more than 3.05 m, 30.48 m, 304.80 m, 1.61 km, 8.05 km, 16.09 km or 160.93 km (10 feet, 100 feet, 1000 feet, 1 mile, 5 miles, 10 miles or 100 miles) apart from each other. In some aspects, the steps (a)-(b) can be performed on a mobile platform.

In some aspects of this disclosure, steps (a)-(e), and optionally steps (f), (g) and/or (h), are repeated with at least a second, third or more selected recyclable stream(s), thereby producing at least a second incubated biological particles stream and at least a second incubated biological hydrolysate. Combining the first incubated hydrolysate with at least the second incubated hydrolysate yields an agricultural admixture. Combining the first incubated particles with at least the second incubated particles yields an agricultural admixture useful as a liquid plant fertilizer, a concentrated plant fertilizer or animal provender, or a dried animal provender. Accordingly, in some aspects of this disclosure, the process for producing an agricultural admixture from selected biological recyclable stream(s) further comprises (i) the step of incorporating the combined enzymatically digested hydrolysates from more than one recyclable stream through steps (a) - (h) to yield agricultural admixtures.

In some aspects, the biological hydrolysates described herein can comprise one or more liquid phases. In some aspects, the liquid phases can comprise an aqueous phase and an oil phase. In some aspects, the oil phase can be further separated into a usable food oil composition and a nonusable-as-food oil composition. As used herein, the term "nonusable-as-food oil" is an undistilled oil which is not suitable for animal provender. As used herein, the term "usable food oil" is an undistilled oil which can be incorporated into animal provender (or as a feedstock for biodiesel production, depending on market conditions). In some aspects, the usable food oil is a low-titer point oil from a plant or nut oil. In some aspects, the usable food oil composition can include or exclude plant oils, omega-3 fatty acids, omega-6 fatty acids, and combinations thereof. In some aspects, the plant oils can include or exclude nut oils, citrus oils, cucurbitaceae seed oils, vegetable oils, and/or other edible plant oils, or mixtures thereof. In some aspects, the nut oils can include or exclude almond oil, beech nut oil, brazil nut oil, cashew oil, hazelnut oil, macadamia oil, mongongo nut oil, pecan oil, pine nut oil, pistachio oil, peanut oil, and walnut oil. In some aspects, the citrus oils can include or exclude grapefruit seed oil, lemon oil, orange oil, pomelo oil, and lime oil. In some aspects, the cucurbitaceae seed oils can include or exclude bitter gourd oil, bottle gourd oil, buffalo gourd oil, butternut squash seed oil, pumpkin seed oil, and watermelon seed oil. In some aspects, the other edible plant oils can include or exclude amaranth oil, apricot oil, apple seed oil, argan oil, avocado oil, babassu oil, ben oil, borneo tallow nut oil, cape chestnut oil (also called yangu oil), carob pod oil (algaroba oil), cocoa butter, cocklebur oil, cohune oil, coriander seeds oil, date seed oil, dika oil, false flax oil, grape seed oil, hemp oil, kapok seed oil, kenaf seed oil, lallemantia oil, mafura oil, marula oil, meadowfoam seed oil, mustard oil, niger seed oil, poppyseed oil, nutmeg butter, okra seed oil, papaya seed oil, perilla seed oil, persimmon seed oil, pequi oil, pili nut oil, pomegranate seed oil, poppyseed oil, pracaxi oil, virgin pracaxi oil, prune kernel oil, quinoa oil, ramtil oil, rice bran oil, shea butter, sacha inchi oil, sapote oil, seje oil, tea seed oil (camellia oil), thistle oil, tigernut oil (or nut-sedge oil), tobacco seed oil, tomato seed oil, and wheat germ oil. In some aspects, the nonusable-as-food oil composition can comprise high titer-point oils. The high titer-point oils can congeal at ambient temperature. In some aspects, the high titer-point oils can act as coagulants. In some aspects, the nonusable-as-food oil composition can include or exclude copaiba oil, j atropha oil, milk bush oil, nahor oil, paradise oil, petroleum nut oil, pongamia oil, and animal fats and hydrolyzed lipids therefrom. In some aspects, the nonusable-as-food oil composition can be used for biodiesel production. The aqueous phase can include or exclude minerals, water-soluble amino acids, water-soluble peptides, sugars, and low molecular weight fatty acids. In some aspects, the aqueous phase can be concentrated. The concentrated aqueous phase can comprise increased levels of nitrogen, potassium, and phosphorous. In some aspects, the concentrated aqueous phase comprises 20-80% (by weight) of water.

Each of the steps recited above can feature any of the embodiments for that step featured in this disclosure, and the method can comprise processing of additional recyclable streams.

In some aspects, the biological recyclable stream can be selected from biological recyclable streams including: bone meal, feather meal, culled vegetable or fruit, grape pomace, tomato pomace, olive pomace, fruit pomace, culled grapes, culled tomatoes, culled olives, peanut hulls, walnut hulls, almond hulls, pistachio hulls, legume hulls, fresh food recyclables, and bakery recyclables. Fresh food recyclables can be provided by obtaining fresh food recyclables collected from, for example, one or more of fresh food waste or recyclables providers, for example, supermarkets, butcher shops, food processing facilities, fresh food distributors, fresh green waste from farms, restaurant grease traps, or other viable sources of fresh food recyclables. In some aspects, providing fresh food recyclables comprises collecting fresh food recyclables from for example, supermarkets, food wholesalers, food processing facilities, institutions (food preparation recyclables from such facilities as sports venues, schools, hospitals, hotels, cafeterias, and other institutions) fresh food distributors, fresh green recyclables from farms, or other viable sources of fresh food recyclables. In some aspects, fresh food recyclables are provided by collecting produce, meat, fish, delicatessen, and bakery organics culled by supermarket staff members from food offered for sale by supermarkets. In some aspects, the biological recyclable stream can be collected frequently. In some aspects, frequent collection intervals may be once, twice or multiple times per day, or once, twice, three times, four times, five times, six times, or seven times per week.

In some aspects, the agricultural admixtures described herein can be further mixed with organic fertilizers to produce a synergistic effect of the organic fertilizers and the agricultural admixtures described herein in improving crop yields and organic soil content. The organic fertilizers can include or exclude bone meal, blood meal, feather meal, chicken manure, and cow manure. The inventors have surprisingly discovered that the processed agricultural admixtures described herein when mixed with an organic fertilizer affords pelletization of the combined product. The inventors have also surprisingly discovered that the processed agricultural admixtures described herein when mixed with an organic fertilizer results in faster breakdown of the organic fertilizer into nutrients to enhance plant and/or crop growth rates and crop yield.

Blood meal is the liquid or dried blood from an animal after slaughter. Blood meal has a high nitrogen content, often up to 15% (wt.) owing to its high protein content. The inventors have determined that when blood meal is mixed with the agricultural admixtures described herein, the resulting agricultural admixture comprises a high protein, peptide and/or amino acid content which yields enhanced crop yields when administered to plants. Without being bound by theory, the protein and/or amino acids in the processed blood meal agricultural admixture enhances soil microbe colony expansion, which enables higher nutrient delivery to plants. In some aspects, the enzyme selected for processing the blood proteins can be a protease. In some aspects, the protease will degrade the blood proteins into peptides and/or amino acids. The agricultural admixture produced by combining incubated hydrolysate or particles ("blood meal agricultural admixture") produced from a blood meal biological recyclable stream can exhibit increased nitrogen content. The nitrogen content can be measured using the Kjeldahl method. In some aspects, the blood meal agricultural admixture comprises a final nitrogen concentration selected from 1 to 15% (wt.). In some aspects, the final nitrogen concentration (wt.) in a blood meal agricultural admixture is selected from: 0.5%, 0.6%, 0.7%, 0.8%, 0.9%., 1.0%, 1.1%, 1.2%, 1.3%, 1.4%, 1.5%, 1.6%, 1.7%,1.8%, 1.9%, 2.0%, 2.1%, 2.2%, 2.3%, 2.4%, 2.5%, 3.0%, 3.5%, 4.0%, 4.5%, 5.0%, 5.5%, 6%, 7%, 8%, 9%, 10%, 11%, 12%, 13%, or 14%, or any range between any two of the recited percentages. In some aspects, the final nitrogen concentration (weight percent) in the agricultural admixture produced by the methods described herein can range from 1-3.0%, 3.0-3.5%, 3.5-4.0%, 4.0-4.5%, 4.5-5.0%, 5.0-5.5%, or 5.5-6.0%, or any range between any two of the recited percentages. In some aspects, the nitrogen concentration (weight percent) in the blood meal agricultural admixture can be: 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1. 3.2, 3.3, 3.4, 3.5, 3.6, 3.7, 3.8, 3.9, 4.0, 4.1, 4.2, 4.3., 4.4, 4.5, 4.6, 4.7, 4.8, 4.9, 5.0., 5.1, 5.2, 5.3, 5.4, 5.5, 5.6, 5.7, 5.8, 5.9, or 6.0%, or any range between any two of the recited percentages. In some aspects, the agricultural admixture produced from a blood meal agricultural admixture can be mixed or blended with an agricultural admixture produced from a different biological recyclable stream as described herein to yield a final nitrogen content (weight percent) of: 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3., 4.4, 4.5, 4.6, 4.7, 4.8, 4.9, 5.0., 5.1, 5.2, 5.3, 5.4, 5.5, 5.6, 5.7, 5.8, 5.9, or 6.0%, or any range between any two of the recited percentages. In some aspects, the blood meal agricultural admixture can be mixed or blended with an agricultural admixture produced from a different biological recyclable stream as described herein to yield a final nitrogen content (weight percent) ranging from: 2.5-3.0%, 3.0-3.5%, 3.5-4.0%, 4.0-4.5%, 4.5-5.0%, 5.0-5.5%, or 5.5-6.0%, or any range between any two of the recited percentages. In some aspects, the blood meal agricultural admixture is a fertilizer. In some aspects, the blood meal agricultural admixture increases the nitrogen content in soil. In some aspects, the blood meal agricultural admixture enhances crop yield. In some aspects, the blood meal agricultural admixture is combined with an agricultural admixture produced from a different biological recyclable stream as described herein to yield a high nitrogen content fertilizer product.

In some aspects, the agricultural admixture produced by the methods described herein from soybean meal recyclable streams can be combined with an agricultural admixture produced from a different biological recyclable stream ("soybean product agricultural admixture") as described herein to yield a high protein fertilizer or animal provender. In some aspects, the soybean product agricultural admixture comprises a high protein and/or amino acid content in the form of amino acids and peptides in animal provender. In some aspects, the final nitrogen concentration (wt.) in a soybean product agricultural admixture is selected from: 0.5%, 0.6%, 0.7%, 0.8%, 0.9%., 1.0%, 1.1%, 1.2%, 1.3%, 1.4%, 1.5%, 1.6%, 1.7%,1.8%, 1.9%, 2.0%, 2.1%, 2.2%, 2.3%, 2.4%, 2.5%, 3.0%, 3.5%, 4.0%, 4.5%, 5.0%, 5.5%, 6%, 7%, 8%, 9%, 10%, 11%, 12%, 13%, or 14%, or any range between any two of the recited percentages. In some aspects, the final nitrogen concentration (weight percent) in the agricultural admixture produced by the methods described herein can range from 1-3.0%, 3.0-3.5%, 3.5-4.0%, 4.0-4.5%, 4.5-5.0%, 5.0-5.5%, or 5.5-6.0%, or any range between any two of the recited percentages. In some aspects, the nitrogen concentration (weight percent) in the soybean product agricultural admixture can be: 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1. 3.2, 3.3, 3.4, 3.5, 3.6, 3.7, 3.8, 3.9, 4.0, 4.1, 4.2, 4.3., 4.4, 4.5, 4.6, 4.7, 4.8, 4.9, 5.0., 5.1, 5.2, 5.3, 5.4, 5.5, 5.6, 5.7, 5.8, 5.9, or 6.0%, or any range between any two of the recited percentages. In some aspects, the agricultural admixture produced from a soybean product agricultural admixture can be mixed or blended with an agricultural admixture produced from a different biological recyclable stream as described herein to yield a final nitrogen content (weight percent) of: 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3., 4.4, 4.5, 4.6, 4.7, 4.8, 4.9, 5.0., 5.1, 5.2, 5.3, 5.4, 5.5, 5.6, 5.7, 5.8, 5.9, or 6.0%, or any range between any two of the recited percentages. In some aspects, the soybean product agricultural admixture can be mixed or blended with an agricultural admixture produced from a different biological recyclable stream as described herein to yield a final nitrogen content (weight percent) ranging from: 2.5-3.0%, 3.0-3.5%, 3.5-4.0%, 4.0-4.5%, 4.5-5.0%, 5.0-5.5%, or 5.5-6.0%, or any range between any two of the recited percentages. In some aspects, the soybean product agricultural admixture is a fertilizer. In some aspects, the soybean product agricultural admixture increases the nitrogen content in soil. In some aspects, the soybean product agricultural admixture enhances crop yield. In some aspects, the soybean product agricultural admixture is combined with an agricultural admixture produced from a different biological recyclable stream as described herein to yield a high nitrogen content fertilizer product.

In some aspects, a biological recyclable stream can include or exclude poultry recyclable products. In some aspects, the poultry can be selected, *e.g.,* from chickens (*e.g*., *Gallus gallus domesticus*), turkeys (*e.g., Meleagris gallopavo*), quail (*e.g., callipepla* genus), ostrich (*e.g., struthio camelus*), and emu (*e.g., dromaius novaehollandiae*). The poultry recyclable products can include or exclude the various components of poultry: feathers, beaks, feet, claws, bones, and feces. In some aspects, an enzyme selected to digest the poultry recyclables can include or exclude a protease or keratinase. The agricultural admixture produced by the methods described herein from biological recyclable streams which include poultry recyclable streams ("poultry agricultural admixtures") can exhibit high protein, peptide, and/or amino acid content, and can be used for enhancing crop yields or delivering digestible protein in animal provender. In some aspects, poultry agricultural admixtures can be used as a fertilizer. In some aspects, poultry agricultural admixtures increase the nitrogen content in soil. In some aspects, poultry agricultural admixtures enhance crop yield. In some aspects, poultry agricultural admixtures yield a high nitrogen fertilizer product. In some aspects, poultry agricultural admixtures are combined with an agricultural admixture produced from a different biological recyclable stream as described herein to yield a high protein animal provender.

In some aspects, the culled vegetable or fruit recyclables can be selected from: culled grapes, culled olives, culled corn (*e.g., zea mays Linn*), culled bottle groud (*e.g, lagenaria siceraria*), culled carrot (*e.g., daucus carota*), culled peas (*e.g., Pisum sativum*), culled potatoes (*e.g., Solanum tuberosum L*.), culled sugar beets (*e.g., Beta vulgaris var. altissima*), culled celery (*e.g., Apium graveolens*), culled tomatoes (*e.g., Lycopersicon esculentum Mill*.), culled members of the *brassica* genus (*e.g.,* culled broccoli (*e.g., Brassica oleracea*), culled radish (*e.g., Brassica oleracea B*), culled cauliflower (*e.g., Brassica oleracea C*.), culled Brussel sprouts (*e.g., Brassica oleracea*), culled cabbage (*e.g., Brassica oleracea*), culled collard greens (*e.g., Brassica oleracea A*), culled kale (*e.g., Brassica oleracea A*), culled mustard greens (*Brassica* juncea), culled turnips (*e.g., Brassica rapa* var. *rapa*), and culled rutabaga (*e.g., Brassica napus* subsp. *rapifera*)), culled lettuce (*e.g., Lactuca sativa*), culled spinach (*e.g.,* Spinacia oleracea), culled banana peels (*e.g., Musa acuminate*), culled watermelon (*e.g., Citrullus lanatus*), culled apples (*e.g., Malus domestica*), culled pineapples (*e.g.,* Ananas comosus), culled grapes (*e.g., vitis* species, including *Vitis californica*), culled olives (*e.g., Olea europaea*), culled citrus (including orange *(e.g.,* Raphanus sativus), squash (*e.g., Citrus x sinensis*), grapefruit (*e.g., Citrus* × *paradisi*), lemon (*e.g., Citrus* × *limon*), lime (*e.g., Citrus aurantifolia*), mandarin (*e.g., Citris reticulata*), and pomelo (*e.g., Citrus maxima*)), culled mangoes (*e.g., Mangifera indica*), culled members of the *fragaria* genus (*e.g.,* strawberries (*e.g., Fragaria* × *ananassa*)), culled members of the *Vaccinium* genus (*e.g.,* blueberries (*e.g., Vaccinium corymbosum sect. Cyanoccocus*), cranberries (*e.g., Vaccinium macrocarpon*), bilberries, whortleberries, lingonberries, cowberries, and huckleberries), culled sugar cane (*e.g., Saccharum officinarum*), culled members of the *Rubus* genus (*e.g.,* blackberries (*e.g., Rubus fruticosus species aggregate*), boysenberries (*e.g., Rubus ursinus* × *R. idaeus*), raspberries (*e.g., R. idaeus* and *R. strigosus,* and hybrids thereof)), culled members of the Prunus genus (*e.g.,* cherries (*e.g., Prunus avium*), plums (*e.g., P. domestica*), apricots (*e.g., P. armeniaca, P. brigantina, P. mandshurica, P. mume, or P. sibirica*), pluots (*e.g.,* hybrids of *P. salicina* and *P. cerasifera*), peaches (*e.g., Prunus persica*)), culled pears (*e.g., Pyrus communis* subsp. *Communis,* the Chinese white pear (*bai li*) *Pyrus* × *bretschneideri,* and the Nashi pear *Pyrus pyrifolia* (also known as Asian pear or apple pear)), or mixtures or combinations thereof. The culled vegetable or fruit recyclables can be the entire plant or components thereof. The culled vegetable plant components can include or exclude: roots, leaves, stems, fruits, peels, seeds, flowers, tubers, pollen, and stalks.

In some aspects, the biological recyclable stream can be soybeans or a soybean product. The soybeans can be hydrolyzed by the methods described herein to produce a soy protein. By the term "soy protein" as used herein is meant any form of soy concentrate or soy isolate, which may for example be a commercial soy concentrate or soy isolate or the soy concentrate or soy isolate intermediate produced in a plant adopted to conversion of defatted soy meal to polypeptides. In some aspects, the soybean product can include or exclude soy meal. Soy meal is the leftover product after crushing soy beans using a physical press to extract soy oil. In some aspects, soy meal comprises 10 to up to 45% (by weight) protein. The soy protein concentration referred to above with reference to the proteolytic activity of the enzymes described herein and to the substrate concentration is calculated as the percentage of nitrogen measured according to Kjeldahl multiplied by 6.25. In some aspects, biological recyclable stream can comprise soybeans and lettuce. The inventors have surprisingly discovered that the hydrolysis of soybeans requires a water source, and that lettuce provides a high-water content plant. In some aspects, hydrolysis of lettuce is performed by using a cellulase at an acidic pH in the incubation steps described herein. In some aspects, the hydrolysis of lettuce and/or soybean product can be performed using a whey biological recyclable stream comprising lactic acid.

In some aspects, the biological recyclable stream processed by the methods described comprises culled fruits or vegetables ("fruit/vegetable agricultural admixture"). The fruit/vegetable agricultural admixture can exhibit selected properties. Admixtures blended with a fruit/vegetable agricultural admixture can exhibit selected properties. The selected properties result from the selection of the culled fruit or vegetable recyclable stream processed by the methods described herein. In some aspects, the culled fruits can include or exclude: citrus molasses, fruit skins, fruit juice, and fruit pulp. In some aspects, the culled vegetables can be lettuce. The lettuce content can increase the water content, as necessary, to increase nutrient solubility when the lettuce cull recyclable stream is blended with other biological recyclable streams. When the other biological recyclable stream comprises soy recyclable products, the water from the lettuce can solubilize the nutrients from the soy waste.

In some aspects, the agricultural admixtures prepared from culled vegetable recyclable streams (*e.g., brassicas* (*e.g.,* broccoli, radish, cauliflower, Brussel sprouts, kale, collard greens, mustard greens, turnips, and/or rutabaga)) are useful as natural pesticides. The inventors have discovered that agricultural admixtures produced by the methods described herein from recyclable streams comprising brassicas can comprise glucosinolates, which hydrolyze to isothiocyanate. Isothiocyanate is produced from brassicas when the brassica cell walls are compromised from the grinding and cellulases described herein. In some aspects, the agricultural admixtures comprising isothiocyanate can be concentrated (*e.g*., by filtering, evaporation, lyophilization, nebulization, spray-drying) to increase the concentration of isothiocyanate. In some aspects, the concentration of isothiocyanate can range from 0.1 to 15,000 mg/kg of the agricultural admixture before concentrating the admixture, or any value in that range, depending on the recyclable stream composition. In some aspects, the agricultural admixtures with natural pesticide properties are applied to plants in part for natural pesticide, nematode, and/or weed protection. In some aspects, the natural pesticide agricultural admixtures can be applied to the leaves, stems, or roots of plants. In some aspects, the form of isothiocyanate is allyl isothiocyanate. In some aspects, the natural pesticide agricultural admixtures can be used to replace all or part of a crop fumigant. The crop fumigant to be replaced can include or exclude methyl bromide. In some aspects, the natural pesticide agricultural admixtures are blended with a fertilizer to yield an enhanced fertilizer which also provides natural pesticide protection. In some aspects, the fertilizers comprise agricultural admixtures produced from a second biological recyclable streams as described herein. The second biological recyclable stream can include or exclude: blood meal, fresh food recyclables, culled poultry, bakery recyclables, and vegetable recyclable stream other than culled *brassicas.* In some aspects, the agricultural admixtures prepared from culled *brassicas* (*e.g.,* broccoli, radish, cauliflower, Brussel sprouts, kale, collard greens, mustard greens, turnips, and/or rutabaga) as the biological recyclable stream can comprise an increase in carotenoids compared to agricultural admixtures not selected for *brassicas* as the biological recyclable stream. In some aspects, the agricultural admixtures with increased carotenoids (increased relative carotenoid concentration) can be used as an animal provender. The animal provender agricultural admixture with increased carotenoids can be fed to egg-layers. The carotenoids fed to said egg-layers can result in egg yolks which are a darker orange due to the carotenoid and therefore more valuable as a consumer item.

In some aspects, the agricultural admixtures produced by the methods described herein can yield a compost product, including the separated solids, which are then run through a screw press (such as may be manufactured by Vincent, Doda, or Fan); a belt filter (such as may be manufactured by Westphalia, Andritz, or Westech); or a hydraulic press (such as may be manufactured by Pall or Flow Press) to extract liquids, which may be recycled into the biological hydrolysate, and a dewatered biological particles. The dewatered biological particles may be used as a source of cellulosic material or fiber in the dried animal feed agricultural admixture. The dewatered biological particles may also be used in the production of biofuels. The dewatered biological particles may also be used to produce organic green waste compost, or an organic compost made with this material and basalt rock dust.

In some aspects, the culled fruit and/or vegetable biological recyclable stream can be selected to yield an agricultural admixture produced by the methods described herein with an increased sugar content compared to an agricultural admixture from a different biological recyclable stream. The biological recyclable stream used to produce agricultural admixtures with high sugar content can be culled fruits or vegetables with a high sugar (fructose, glucose, xylose, mannose, or sucrose) content. In some aspects, the high sugar content containing fruits or vegetables can include or exclude: apples, pears, cherries, blackberries, oranges, lemons, grapefruits, pomelos, papayas, watermelons, cantaloupes, honeydew melons, strawberries, blueberries, raspberries, bananas, grapes, boysenberries, blackberries, plums, apricots, nectarines, guava, pluots, pineapples, mangoes, and mixtures and combinations thereof. In some aspects, the agricultural admixture produced by the methods described herein with an increased sugar content can be used as an animal provender. In some aspects, the high sugar content animal provender agricultural admixture can be fed to animals to increase fat production and/or fat uptake. In some aspects, the high sugar content animal provender agricultural admixture can be mixed or blended with an agricultural admixture processed from a different biological recyclable stream at any of the process points of said different biological recyclable stream. The resulting mixture can be used as an animal provender with increased sugar content. The high sugar content animal provender can make the agricultural admixture more palatable to the animal resulting in a higher feed uptake amount.

In some aspects, the culled fruit and/or vegetable biological recyclable stream can be mixed or blended with a soybean product recyclable stream to yield an agricultural admixture produced by the methods described herein with an increased nitrogen content. In some aspects, the increased nitrogen containing agricultural admixture can be used as an organic fertilizer.

### Pesticide-binder

In some aspects, the agricultural admixtures produced by the methods described herein can be used to increase adhesion of pesticides to the surfaces of plants. In some aspects, the agricultural admixtures produced by the methods of this disclosure using biological recyclable streams comprising fats can exhibit a high oil content from the processed fats. The oils impart a tackiness to a liquid agricultural admixture. In some aspects, the agricultural admixture can be blended with a pesticide prior to application onto plants, and/or can comprise natural pesticides from admixtures obtained from culled *brassicas,* as disclosed herein. The oils in the agricultural admixture can form a complex between the pesticide and the plant surface to enhance adhesion of the pesticide to the plant or a plant component. In some aspects, the agricultural admixture with pesticide can be further increased in fat content by the addition of separated centrifuged oils from the processing of a portion or all of another biological recyclable stream. The plant component can be selected from: roots, leaves, stems, fruits, pollen, bark, or combinations thereof.

In some aspects, pesticides can be adhered to a plant using the agricultural admixtures of this disclosure by a method comprising the steps of:
(a) presenting an agricultural admixture produced by the methods described herein where the biological recyclable stream comprises a fat;
(b) blending the agricultural admixture with a pesticide to produce a blended pesticide-containing agricultural admixture; and
(c) contacting the blended pesticide-containing agricultural admixture with a plant or plant component.

In some aspects, the plant component can be selected from: roots, stems, leaves, fruits, or combinations thereof.

### Vitamins and Antioxidants in Agricultural Admixtures

In some aspects, the biological recyclable stream can comprise pomace from olives, grapes, tomatoes, cocoa, and/or apples. The pomace recyclable stream can include or exclude seeds, seed skins, fruit skins, shells, and residual juice from the fruit. The pomace recyclable stream from olives, grapes, cocoa, or tomatoes can comprise vitamins and antioxidants.

In some aspects, the vitamins and antioxidants can include or exclude: polyphenol compounds, tocopherol compounds, flavonoids, and vitamin C. In some aspects, the polyphenol compounds can include resveratrol. In some aspects, the flavonoids are anthocyanins. The inventors have determined that agricultural admixtures produced by the methods described herein from pomace recyclable streams ("pomace agricultural admixtures") exhibit high concentrations of vitamins and antioxidants. In some aspects, pomace agricultural admixtures can be mixed with agricultural admixtures processed from other biological recyclable streams as described herein to yield animal provender with a higher concentration of antioxidant compounds. The resulting agricultural admixtures with high concentrations of vitamins and antioxidant compounds can be fed to animals as a method of delivering vitamins and antioxidants to the animals.

In some aspects, antioxidants are added to animal provender or any process step preceding the formation of animal provender. The antioxidants are as described herein.

In poultry meats, the bright red or pink color associated with freshness fades to grey-brown as oxymyoglobin is converted to metmyoglobin by oxidation. Lipid oxidation may also occur, affecting both aroma and flavor acceptability. Growth of spoilage bacteria, such as *Pseudomonas spp.,* exacerbates these effects, while also influencing meat texture. The willingness of consumers to purchase meat is greatly reduced by these changes. In some aspects, the processes described herein include adding an antioxidant to the agricultural admixtures which are to be used as an animal provender. In some aspects, the antioxidant is added by using vitamin E as a fat-soluble antioxidant. In some aspects, the vitamin E is provided from a pomace. In some aspects, the pomace providing vitamin E is tomato pomace. In some aspects, the animal provender from agricultural admixtures described herein are useful for producing processed meat comprising antioxidants which decreases meat spoilage and increases the shelf-life of processed meat, including broiler chickens. In some aspects, the antioxidant animal provender when fed to egg-layers increases the egg-layer lifetime, resulting in an increase of eggs produced by the animal. In some aspects, chickens fed with the vitamins and antioxidant animal provender described herein produce darker color eggs. In some aspects, the eggs comprise beta-carotene.

In some aspects, pomace agricultural admixtures provide a source of vitamins and antioxidants in animal provender, by a method comprising:
(a) providing an agricultural admixture produced from a biological recyclable stream comprising pomace according to the methods herein; and
(b) introducing the agricultural admixture into an animal provender trough.

In some aspects, pomace agricultural admixtures provide antioxidants to processed meat, by a method comprising:
(a) providing an agricultural admixture produced from a biological recyclable stream comprising pomace according to the methods herein;
(b) introducing the agricultural admixture into an animal provender to feed an animal;
(c) slaughtering the animal;
(d) processing the slaughtered animal into processed meat,
where the processed meat comprises vitamins and antioxidants from the agricultural admixture.

In some aspects, the pomace agricultural admixtures extend the shelf-life of processed meat, by a method comprising:
(a) providing an agricultural admixture produced from a biological recyclable stream comprising pomace according to the methods herein;
(b) introducing the agricultural admixture into an animal provender to feed an animal;
(c) slaughtering the animal;
(d) processing the slaughtered animal into processed meat comprising at least 2% fat content,
where the vitamins and antioxidant from the agricultural admixture is present in the fat in the processed meat.

The methods of this disclosure for producing animal provender comprising a pomace agricultural admixture are further useful for reducing the cellulose content present in the pomace recyclable stream, for feeding to animals that would otherwise lack the ability to digest unprocessed pomace. In some aspects, the agricultural admixtures/animal provender produced from a biological recyclable stream comprising pomace can be used as chicken feed. In some aspects, the high antioxidant agricultural admixtures in the animal provender can extend the shelf life of animal meat products. In some aspects, animals fed the high antioxidant agricultural admixtures retain vitamins and antioxidants in lipids and fatty acids of the animal tissues. In some aspects, those antioxidants in the tissues of animals which consume the high vitamins and antioxidant agricultural admixture feed increase the shelf-life of meat from the processed animal. Without being bound by theory, the antioxidant in the fats and lipids in the processed meat can reduce decoloration, reduce rancidity, decrease decay, and/or prevent oxidation of the animal fats, resulting in a longer shelf-life of the processed meat. In some aspects, this disclosure includes a processed meat product with an extended shelf-life comprising antioxidants delivered by animal provender. In some aspects, the processed meat product can be selected from: poultry, pork, or fish.

### Anti-caking Agents

In some aspects, anti-caking agents can be added to dried forms of animal provender or any process step preceding the formation of dried forms of animal provender. Anti-caking agents are additives to powdered or granulated materials to prevent the formation of lumps. The anti-caking agents can include or exclude: tricalcium phosphate, powdered cellulose, magnesium stearate, sodium bicarbonate, sodium ferrocyanide, potassium ferrocyanide, calcium ferrocyanide, bone phosphate (*i.e*. Calcium phosphate), sodium silicate, silicon dioxide, calcium silicate, magnesium trisilicate, talcum powder, sodium aluminosilicate, potassium aluminium silicate, calcium aluminosilicate, bentonite, aluminum silicate, stearic acid, and polydimethylsiloxane.

### Antibiotic-free Animal Provender

In some aspects, the agricultural admixtures produced by the methods described herein can be used as a healthy food source in an antibiotic-free livestock program. In some aspects, the antibiotic-free livestock program can include weanling pigs and chicks. Animal provender from the agricultural admixtures described herein (which have large inputs of fruits and vegetables) can improve the health of weanling pigs or hatchling chicks, compared to conventional diets of corn and soy meal.

In some aspects, agricultural admixtures produced from a first biological recyclable stream can be mixed or blended with a second biological recyclable stream, which can be any other recyclable stream of this disclosure. In some aspects of this disclosure, for example, the first biological recyclable stream can be a pomace recyclable stream, and the second biological recyclable stream can be fresh food recyclables. In some aspects, the resulting mixed agricultural admixture from pomace recyclable streams and fresh food recyclable streams can comprise antioxidants and be used as digestible animal provender. In some aspects, the resulting mixed agricultural admixture from pomace recyclable streams and a second biological recyclable stream can be used a high protein and high antioxidant animal provender.

In some aspects of this disclosure, the biological recyclable stream can be a carbohydrate recyclable stream. The carbohydrate recyclable stream can include or exclude bakery recyclables. The bakery recyclables can include or exclude cooked products, expired ingredients, or expired dough. The bakery recyclable cooked products can include or exclude: cakes, tarts, donuts, cereals, pastas, breads, pastries, crackers, chips, pretzels, and the like. Expired ingredients can include or exclude: flour, sugar, icing, yeast, corn meal, and burnt or broken products. In some aspects, bakery agricultural admixtures can comprise a high concentration of carbohydrates compared to agricultural admixtures produced from recyclable streams other than bakery goods. In some aspects, the carbohydrates in bakery agricultural admixtures can decrease the dry time to convert a liquid form of the agricultural admixture to a solid form of the agricultural admixture. Agricultural admixtures produced using carbohydrate recyclable streams exhibit, for example a high sugar content and/or enhanced pelletization properties. The enhanced pelletization properties can be useful for manufacturing a desired form of animal provender. The appropriate form of an animal provender can include or exclude: pellets, flakes, pastes, cereals, and powders. In some aspects, carbohydrate agricultural admixtures can be blended with agricultural admixtures produced from other biological recyclable streams as described herein to enhance pelletization of the blended agricultural admixture. In some aspects, an agricultural admixture described herein can be mixed, blended, compounded, pulverized, ground, or dissolved with a carbohydrate recyclable stream which has not been processed by the enzymatic digestion methods described herein. The carbohydrate recyclable stream can be dried then added to a wet or dried form of an agricultural admixture described herein to produce a dry form of animal provender.

In some aspects, this disclosure also features a method of forming into pellets an agricultural admixture produced from a biological recyclable stream which includes carbohydrate recyclable streams, into pelleted fertilizer and feed products, by a method comprising the steps of:
(a) presenting the agricultural admixture produced from carbohydrate recyclable stream in liquid form;
(b) introducing the liquid form of the agricultural admixture into a drying apparatus;
(c) drying the agricultural admixture; and
(d) cutting the dried agricultural admixture into pellets.

In some aspects, this disclosure also features a method of forming into powder or granular form an agricultural admixture produced from a biological recyclable stream with a carbohydrate source which was not processed by enzymatic digestion. In some aspects, the carbohydrate source can include or exclude: bakery goods, bread crumbs, soymeal, distiller's grains, walnut hulls, and/or almond hulls. In some aspects, the hydrolysate can be in a dewatered (essentially dry) or liquid form when combined with the additional carbohydrate source. Agricultural admixtures produced by the methods as described herein from biological recyclable streams selected from blood meal, culled fruits or vegetables, pomace, or fresh food recyclables will be high in proteins and/or peptides, fats, and fiber, but lower in carbohydrates. In some aspects of this disclosure, the agricultural admixtures produced by the methods described herein from bakery recyclable streams can be blended or mixed with agricultural admixtures produced from other biological recyclable streams as described herein to yield a product high in proteins and/or peptides, fats, fiber, and carbohydrates. In some aspects, the agricultural admixture produced by the methods described herein comprising a constituent which can include or exclude: proteins and/or peptides, fats, fiber, and carbohydrates, can be used as a pre-digested feedstock for animals. The inventors have recognized that the pre-digested feedstock has a higher mass conversion rate of feed to animal weight compared to a standard feed product, with an observed increase in animal weight when used as a feed relative to control. As described in Example 10, broiler chickens fed with diluted animal provender in powdered form made by the processes described herein exhibited a 25% increase in weight after only 14 days of feeding relative to a control cohort.

The agricultural admixtures of this disclosure can be used as a high-conversion rate animal provender. Animals (pigs and/or chickens that are usually fed a diet of corn & soy meal) can be fed the liquid or dried agricultural admixtures of this disclosure to gain weight with increased food use efficiency (*i*.*e*., an increased conversion rate of food into animal weight). In some aspects, the animals produce less manure and have less diarrhea when fed the pre-digested composition. Accordingly, approximately 100% of the biological recyclable stream processed according to the methods of this disclosure can be efficiently utilized. In some aspects, the animals can include or exclude pigs, avians, rabbits, horses, insects, worms, and other nonruminants. In some aspects, avians can include or exclude chicken, turkey, quail, ostrich, and emu. In some aspects, insects can include or exclude crickets (*e.g., acheta domesticus*), and black soldier fly (*e.g., hermetia illucens*). In some aspects, worms can include or exclude earthworm (*e.g*., Oligochaeta), silk worm, moth worm, and mealworms (*e.g*., Tenebrio molitor).

### Systems for Producing Agricultural Admixtures

In some aspects, the systems described herein can include a heated feed tank. In some aspects, the heated feed tank can be configured to be between the incubation tank and the separation tank. In some aspects, the feed tank can be configured to be between the grinding tank and the incubation vessel. In some aspects, the feed tank can be configured to be between the incubation vessel and the drying equipment. The feed tank can be jacketed to afford temperature control. The jacketed feed tank can be steam sparged to increase the rate of temperature increase. In some aspects, the feed tank is heated to a temperature ranging from about 37.8 °C to about 104.4 °C (100 °F to about 220 °F). In some aspects, the feed tank is heated to around 71.1 °C (160 °F).

In some aspects, grinding of the biological recyclable streams may be carried out using a rotary knife grinder. In some aspects, the biological recyclable streams may be further ground with a low RPM/high torque grinder with shredding action may also be used to further grind the biological recyclable slurry. In some aspects, the agricultural admixtures described herein can be blended with a bread recyclable stream using a knife grinder to produce pelletized products.

In some aspects, the incubating ground biological slurry can be sheared with a high shear grinder with shearing action, which can comprise, for example, a high shear mixer with a disintegrating head, during all or a part of the incubating and pasteurizing steps. A high-shear mixer disperses or transports one phase or ingredient (liquid, solid, or gas) into a main continuous phase (liquid), with which it would normally be immiscible. A rotor or impeller, together with a stationary component known as a stator, or an array of rotors and stators, is used either in a tank containing the solution to be mixed, or in a pipe through which the solution passes, to create shear. The high shear grinder can impart a high shear rate onto the slurry. In some aspects, the high shear grinder can be, for example, the ARDE Dicon In-Line Dispersing Grinder, or a Silverson Mixer Homogenizer. As used herein, "shear" refers to a cutting action that reduces food particle size, increasing its surface area, and therefore, its interaction with enzyme molecules. In some embodiments, high shear is created by circulating the slurry through a high speed, high shear mixer throughout the digest at rates in the range of 10⁵-10⁶ sec⁻¹ or more.

This disclosure does not include a garbage disposal as the shearing means.

In some aspects, the incubation process can include or exclude a magnetic trap. The magnetic trap can pull out and/or trap metallic objects which may be present in the fresh food recyclable stream. In some aspects, the metallic objects can include or exclude coins, twistties, buttons, cans, and can parts. The magnetic trap can comprise a magnet. The magnet can be a permanent magnet or an electromagnet. The electromagnet can be configured to become magnetic upon the introduction of the ground fresh food recyclables into the incubation chamber.

In some aspects, the separation step described herein can be performed using a means to apply differential sedimentation. In some aspects, the means of applying differential sedimentation comprises a centrifuge. According to the present invention, the centrifuge can be a tricanter centrifuge. In some aspects, the tricanter centrifuge can be from Flottweg (Germany), U.S. Centrifuge (United States), or Peony (China). The centrifugation separation step can control the levels of high-titer point oils from the agricultural admixture. The high-titer point oils can clog fertilizer feed lines during the administration of agricultural admixtures to crops. It was surprisingly discovered that the control of the levels of high-titer point oils using a centrifugation separation step can improve administration of the agricultural admixtures described herein through fertilizer feed lines to crops. According to the present invention, the agricultural admixture fat content can be controlled using a tricanter centrifuge. In some aspects, the agricultural admixture fat content can be reduced from 5-12% to 0.2-4% (weight percent) using the centrifugal separation step. In some aspects, the fat content can be reduced from 5-12% to about 1-2 % (by weight) for the liquid phase using the centrifugal separation step. In some aspects, the fat content can be reduced from 5-12% or more to about 2-4% (by weight). In some aspects, the fat content can be reduced from 5-12% or more to about 3-4.5% (by weight). In some aspects, the fat content can be reduced from 5-12% or more to about 0.1-1.5% (by weight). In some aspects, the fat content can be reduced from 5-12% or more to about 0.05 to about 0.1% (by weight). According to the present invention, the fat content of the pasteurized first incubated hydrolysate is reduced to a range of between 1 to 4 wt% by tricanter centrifugation. In some aspects, animal provender comprises a lower fat content than the input biological recyclable stream. The inventors have surprisingly discovered that recycling food processed with the methods described herein into animal provender administered to animals results in healthier animals (*e.g*., exhibiting reduced diarrhea, and/or lower glucose levels), and faster growing, compared to conventional animal diets. The centrifugation step can be performed from 2000 rpm to 5000 rpm. The centrifugation step can be performed at a throughput 18.93 - 189.27 litres (5-50 gallons) per minute preferably from 49.21 to 56.78 litres (about 13 to about 15 gallons) per minute. The centrifuge step can be performed at from 1,000 to 9,000 rpm, preferably at a rate of 3,000 - 5,000 rpm, with the material at a temperature of 48.8 °C - 104.4 °C (120 °F - 220 °F), preferably at a temperature range of 60 °C to 82.2 °C (140 °F to 180 °F). As shown in Table 13, the inventors were able to selectively control the fats, dry ash, and crude protein content for a variety of hydrolysates produced from a variety of fresh food recyclable input streams using a tricanter centrifuge at selected operational parameters which can include or exclude: bowl speed, flow rate, and difference in core versus bowl centrifuge speeds, and the impeller spacing. The ability to selectively control the fats content in the aqueous (liquid) phase of the hydrolysate affords the ability to control the hydrophobicity and/or emulsion properties of the final processed product.

The tricanter centrifuge can separate the biological hydrolysates described herein into biological particles and liquid phases comprising one or more phases. In some aspects, the tricanter centrifuge can further separate the liquid phase into an oil-soluble phase and an aqueous-soluble phase. In some aspects, the solid particles can be blended with the dried agricultural admixtures described herein (or dried and pelleted without blending) to produce a high protein animal provender suitable for chickens, pigs, fish and pets. The tricanter centrifuge can be adjusted to separate oils from water-soluble phase from solid particles, and/or to adjust the oil level (which can include or exclude the total fats content) in the liquid phase. In some aspects, the oils can be further separated. The further separation of the oils can be performed using a system selected from a decanter, distillation apparatus, chromatography, and/or oil-water partitioner.

In some aspects, the aqueous phase can be concentrated. In some aspects, the dewatered aqueous phase can be blended with the separated biological particles into a dried solid. In some aspects, the dewatered aqueous phase can be blended with biological recyclable streams from bread to produce pelletized animal provender. The concentration of the aqueous phase can be performed using vacuum evaporation or vibrating filters. Vacuum evaporation removes water solvent, and therefore increases the relative concentration of the aqueous phase components relative to pre-concentrating. Vibrating filters can be used to remove water and salts from the aqueous phase. Fertilizers from the agricultural admixtures described herein require minimal salt content and therefore may be dewatered using vibrating filters which removes water and saline. Animal provender may require a higher saline content than fertilizer and therefore may be dewatered using vacuum evaporation which retains the saline content in the dewatered product. In some aspects, the aqueous phase can be dewatered by lyophilization. In some aspects, the aqueous phase can be dewatered by using a dewatering drum. In some aspects, the dewatering drum is a vacuum dewatering drum. In some aspects, the aqueous phase can be dewatered by azeoptropic removal by the addition of ethanol to form an azeotrope with the water, followed by evaporation of the azeotrope, ethanol, and water under atmospheric or vacuum conditions.

### Enzymes and Processes to Make Agricultural Admixtures

In some aspects, the selected enzymes involved in the incubation step can include or exclude: at least one enzyme to digest proteins, at least one enzyme to digest fats and lipids, or at least one enzyme to digest cellulosic material or at least one enzyme to digest other carbohydrates. The selected enzymes may include or exclude: xylanase, asparaginase, cellulase, hemicellulase, glumayase, beta-glumayase (endo-1,3(4)-), urease, protease, lipase, amylase, keratinase, alpha-amylase, phytase, phosphatase, aminopeptidase, amylase, carbohydrase, carboxypeptidase, catalase, chitinase, cutinase, cyclodextrin glycosyltransferase, deoxyribonuclease, esterase, alpha-galactosidase, beta-galactosidase, glucoamylase, alphaglucosidase, beta-glucosidase, haloperoxidase, invertase, laccase, keratinase (EC 3.4.99), mannosidase, oxidase, glucose oxidase, pectinolytic enzyme, pectinesterase, peptidoglutaminase, peroxidase, polyphenoloxidase, proteolytic enzyme, protease, ribonuclease, thioglucosidase, and transglutaminase. These enzymes may be selected, for example, from the group consisting of enzymes originating from microbial fermentation, enzymes derived from animal digestion, enzymes derived from a microorganism, and enzymes derived from plants.

In some aspects, the selected one or more enzymes may be added as individual enzymes or enzyme combinations to the slurry at various times, and incubated at selected temperatures. In one aspect, the selected one or more enzymes is added to the ground biological slurry in a first enzyme combination comprising at least two of the selected enzymes described herein, and incubated at a first temperature, followed by addition of a second enzyme combination comprising two or more selected enzymes, and incubation at a second temperature. In some aspects, a third enzyme combination can be added comprising two or more selected enzymes, and incubated at a temperature suitable for, or optimized for the activity of the enzymes in the enzyme combination. In some aspects the final enzyme or enzyme combination may comprise a protease, to avoid digestion of previously added enzymes.

In one aspect, a first enzyme combination of the selected enzymes is added during a first incubation step at a first temperature between about ambient temperature (*e.g*., 12.8 °C to about 32.2 °C (55 degrees F (Fahrenheit) to about 90 degrees F), including 13.3, 13.9, 14.4, 15, 15.6, 16.1, 16.7, 17.2, 17.7, 18.3, 18.9, 19.4, 20, 20.6, 21.1, 21.7, 22.2, 22.8, 23.3, 23.9, 24.4, 25, 25.6, 26.1, 26.7, 27.2, 28.3, 28.9, 29.4, 30, 30.6, 31.1, 31.7, or 32.2 °C (56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 degrees F)) to 60 °C (140 degrees F), to form an incubating mixture. In some aspects, the first temperature is selected from 13.3, 13.9, 14.4, 15, 15.6, 16.1, 16.7, 17.2, 17.7, 18.3, 18.9, 19.4, 20, 20.6, 21.1, 21.7, 22.2, 22.8, 23.3, 23.9, 24.4, 25, 25.6, 26.1, 26.7, 27.2, 28.3, 28.9, 29.4, 30, 30.6, 31.1, 31.7, 32.2, 32.8, 33.3, 33.9, 34.3, 35, 35.6, 36.1, 36.7, 37.2, 37.8, 38.3, 38.9, 39.4, 40, 40.6, 41.1, 41.7, 42.2, 42.8, 43.3, 43.9, 44.4, 45, 45.6, 46.1, 46.7, 47.2, 47.8, 48.3, 48.9, 49.4, 50, 50.6, 51.1, 51.7, 52.2, 52.8, 53.3, 53.9, 54.4, 55, 56.1, 56.7, 57.2, 57.8, 58.3, 58.9, and 59.4 °C (56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, and 139 degrees F), or any range between any two of the recited temperatures. In one aspect, the first enzyme combination can be added at an ambient first temperature, and enzymatic processes begin while the system is heated up to a second temperature. The incubation with the first enzyme combination can be carried out for the entirety of the heat ramp time to achieve the second temperature. The time for the heat ramp time can be between from about 20 minutes to about 6 hours, preferably 20 minutes to 1.5 hours, even more preferably 30 minutes to 1 hour. In some aspects, the time for the heat ramp time can is selected from: 20, 25, 30, 35, 40, 45, 50, 55, and 60 minutes. In some aspects, the time for the heat ramp time is selected from: 1, 1.25, 1.5, 1.75, 2, 2.25, 2.5, 2.75, 3, 3.25, 3.5, 3.75, 4, 4.25, 4.5, 4.75, 5, 5.25, 5.5, 5.75 and 6 hours, or any range in between any two heat ramp times. The first selected enzyme combination may in some aspects of this disclosure comprise at least one cellulase and at least one lipase. Preferably the first selected enzyme combination comprises enzymes for digesting complex carbohydrates from plants, for example endocellulase, exocellulase (or another cellulase formulation), and lipase. The first temperature may in some embodiments, preferably be about 35 °C to 60 to 60 °C (95 degrees F to about 140 degrees F), or any temperature described herein for the first temperature. In some embodiments, the incubating mixture is incubated at the first temperature for about 30 minutes. In some embodiments, an organic or inorganic chemical and/or buffer with a pKa enabling a pH above 7.0 may be added to the incubating mixture to increase the pH of the mixture and increase the effectiveness of the first enzyme combination.

In one aspect, at least a second combination of selected enzymes may be added to the incubating mixture, and a second incubation step may be carried out at a second temperature between about 35.6 °C to 62.8 °C (about 96 degrees F to 145 degrees F). In some aspects, the second temperature is selected from: 35.6, 36.1, 36.7, 37.2, 37.8, 38.3, 38.9, 39.4, 40, 40.6, 41.1, 41.7, 42.2, 42.8, 43.3, 43.9, 44.4, 45. 45.6, 46.1, 46.7, 47.2, 47.8, 48.3, 48.9, 49.4, 50, 50.6, 51.1, 51.7, 52.2, 52.8, 53.3, 53.9, 54.4, 55, 56,1, 56.7, 57.2, 57.8, 58.3, 58.9, 59.4, 60, 60.6, 61.1, 61.7, 62.2, or 62.8 °C (96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, or 145 degrees F), or any range between any two of the recited temperatures. The time of the second incubation may be, in some aspects, between about 1 to about 18 hours or more, preferably between 1.2 to 6 hours, more preferably about 1.5 hours to 2 hours. In some aspects, the second incubation time is selected from: 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, 10, 10.1, 10.2, 10.3, 10.4, 10.5, 10.6, 10.7, 10.8, 10.9, 11, 11.1, 11.2, 11.3, 11.4, 11.5, 11.6, 11.7, 11.8, 11.9, 12, 12.1, 12.2, 12.3, 12.4, 12.5, 12.6, 12.7, 12.8, 12.9, 13, 13.1, 13.2, 13.3, 13.4, 13.5, 13.6, 13.7, 13.8, 13.9, 14, 14.1, 14.2, 14.3, 14.4, 14.5, 14.6, 14.7, 14.8, 14.9, 15, 15.1, 15.2, 15.3, 15.4, 15.5, 15.6, 15.7, 15.8, 15.9, 16, 16.1, 16.2, 16.3, 16.4, 16.5, 16.6, 16.7, 16.8, 16.9, 17, 17.1, 17.2, 17.3, 17.4, 17.5, 17.6, 17.7, 17.8, 17.9, and 18 hours, or any range in between any two incubation times.

In some aspects, the second enzyme combination may comprise at least one pectinase, at least one protease, and alpha-amylase. In some aspects, one protease may be added after one pectinase and alpha-amylase in a third enzyme combination. In some aspects, the alpha-amylase can be 1,4-alpha-D-glucan glucanohydrolase (*e.g*., glycogenase).

In one aspect, when the biological recyclable stream comprises culled fruits or vegetables, the selected enzymes can be selected from: a cellulase, a pectinase, a ligninase, an amylase, and combinations thereof. In some aspects, the pectinase can be selected from: pectolyase, pectozyme, polygalacturonase, and combinations thereof. Without being bound by theory, a pectinase breaks down the pectin (*e.g*., polymethyl galacturonate) comprising the cell walls of the fruit or vegetable. The amylase can be selected from: alpha-amylase, beta-amylase (1,4-α-D-glucan maltohydrolase), gamma-amylase (glucan 1,4-α-glucosidase; amyloglucosidase; or exo-1,4-α-glucosidase), and combinations thereof. The amylase can catalyze the hydrolysis of starch into sugars. The cellulase can break down cellulose molecule into monosaccharides such as beta-glucose, or shorter polysaccharides and oligosaccharides. In some aspects, the cellulose can be selected from: endocellulases (EC 3.2.1.4), exocellulases or cellobiohydrolases (EC 3.2.1.91), cellobiases (EC 3.2.1.21), oxidative cellulases, cellulose phosphorylases, and combinations thereof. In some aspects, the cellulase can be selected from: endo-1,4-beta-D-glucanase (beta-1,4-glucanase, beta-1,4-endoglucan hydrolase, endoglucanase D, 1,4-(1,3,1,4)-beta-D-glucan 4-glucanohydrolase), carboxymethyl cellulase (CMCase), avicelase, celludextrinase,cellulase A, cellulosin AP, alkali cellulase, cellulase A 3, 9.5 cellulase, pancellase SS, and combinations thereof.

The temperature and pH of an incubation with one or more selected enzymes can be selected in order to optimize, or be suitable, for the activity of the enzymes in the reaction mixture. In some aspects, a first temperature and pH may be selected in order to optimize, or be suitable, for the activity of the first selected one or more enzymes in a first enzyme combination, while a second temperature and pH may be selected in order to optimize, or be suitable, for the activity of the selected enzymes in a second selected enzyme combination. In other aspects, the timing of an enzyme combination may be selected in order to minimize the impact of enzymes on each other. In one aspect, when a protease is added in combination with another selected enzyme, the protease would be added second, such that the protease would not degrade the other selected enzyme.

In some aspects, after incubating the ground biological slurry with the one or more selected enzymes, the incubated ground biological slurry can be heated to between about 65.6 °C to 82.2 °C (about 150 to 180 degrees F), preferably 65.6 °C to 76.7 °C (150-170 degrees F), for about 30 minutes to about 18 hours, preferably from about 30 minutes to 2 hours, to further pasteurize the ground biological slurry. In some aspects, the ground biological slurry is heated for at a temperature selected from: 65.6, 66.1, 66.7, 67.8, 68.3, 69.4, 70, 70.6, 71.1, 71.7, 72.8, 73.3, 73.9, 74.4, 75, 75.6, 76.1, 76.7, 77.2, 77.8, 78.3, 78.9, 79.4, 80, 80.6, 81.1, 81.7, and 82.2 °C (150, 151, 152, 153, 154, 155, 156, 157, 158, 159, 160, 161, 162, 163, 164, 165, 166, 167, 168, 169, 170, 171, 172, 173, 174, 175, 176, 177, 178, 179, and 180 degrees F), or any range between any two recited temperatures. In some aspects, the ground biological slurry is heated for about a time selected from: 30, 35, 40, 45, 50, 55, and 60 minutes; or 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, 10, 10.1, 10.2, 10.3, 10.4, 10.5, 10.6, 10.7, 10.8, 10.9, 11, 11.1, 11.2, 11.3, 11.4, 11.5, 11.6, 11.7, 11.8, 11.9, 12, 12.1, 12.2, 12.3, 12.4, 12.5, 12.6, 12.7, 12.8, 12.9, 13, 13.1, 13.2, 13.3, 13.4, 13.5, 13.6, 13.7, 13.8, 13.9, 14, 14.1, 14.2, 14.3, 14.4, 14.5, 14.6, 14.7, 14.8, 14.9, 15, 15.1, 15.2, 15.3, 15.4, 15.5, 15.6, 15.7, 15.8, 15.9, 16, 16.1, 16.2, 16.3, 16.4, 16.5, 16.6, 16.7, 16.8, 16.9, 17, 17.1, 17.2, 17.3, 17.4, 17.5, 17.6, 17.7, 17.8, 17.9, and 18 hours, or any range in between any two recited times.

While the incubation and constant agitation and shear steps are highly likely to reduce pathogen concentrations to non-detectible levels, a pasteurization step at a temperature range and duration commonly used in pasteurization processes further reduces the risk of pathogen contamination to levels that are undetectable under current pathogen detection technology. In some aspects, the pasteurization is performed for about 15 minutes to about 1 hour. In some aspects, the pasteurization step is performed for a time selected from: 15, 20, 25, 30, 35, 40, 45, 50, 55, and 60 minutes. In some aspects, the pasteurization step may be performed at various combinations of temperature, pressure, and duration, as commonly used in pasteurization processes. In these aspects, the pasteurization may be performed, for example, from about 15 minutes to about 12 hours, for any length of time at 15 minute intervals between 15 minutes to 12 hours (*e.g*., 15 minutes, 30 minutes, 45 minutes, etc.), or any pasteurization time described herein. In some aspects, the temperature can be from about 48.9, 49.4, 50, 50.6, 51.1, 51.7, 52.2, 52.8, 53.3, 53.9, 54.4, 55, 56.1, 56.7, 57.2, 57.8, 58.3, 58.9, 59.4, 60, 60.6, 61.1, 61.7, 62.2, 62.8, 63.3, 63.9, 64.4, 65, 65.6, 66.1, 66.7, 67.8, 68.3, 69.4, 70, 70.6, 71.1, 71.7, 72.8, 73.3, 73.9, 74.4, 75, 75.6, 76.1, 76.7, 77.2, 77.8, 78.3, 78.9, 79.4, 80, 80.6, 81.1, 81.7, or 82.2 °C (about 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, or 180 degrees F), or more, or any temperature or range falling between any two of those temperatures. In some aspects, the pasteurization may be performed at 1-10 atm (atmospheres) pressure. In some aspects, the pasteurization may be performed at 1, 2, 3, 4, 5, 6, 6, 7, 9, or 10 atm pressure.

The separated incubated biological hydrolysate may contain small incubated biological particles. In some aspects, the separating of step (d) produces a liquid hydrolysate that is about 90% to about 95% by weight relative to the weight of the input incubating biological recyclables, and particles having an average diameter of less than about 1000, 950, 900, 850, 841, 800, 750, 707, 700, 650, 600, 590-595, 550, 500, 450, 400, 354, 350, 300, or 271 microns (or any range between any of the recited diameters). In some aspects, the particles have an average diameter more than about 250, 210, 200, 177, 175, 150, 149, 125, 105, 100, 90, 88, 85, 75, 74, 63, 60, 53, 50, 44, 40, or 37 microns (or any range between any of the recited diameters). The average diameter of the particle can be measured using light scattering (*e.g*., multi-angle laser light scattering). In some aspects, average particle diameter is measured using a Wyatt Technologies Dawn Heleos II instrument (Wyatt Technologies, Inc., Santa Barbara, CA, USA).

The separated incubated biological hydrolysate can be emulsified using an ultra-high shear grinder. Emulsification can yield a homogeneous solution such that the viscosity of any three samples are measured to be within experimental error of each other. The ultra-high shear grinder may be designed for maximum shear and low flow. In some aspects, the ultra-high shear grinder may be, for example, a grinder suitable for polishing catchup. In some aspects, the ultra-high shear grinder may be, for example, an ultra-high shear multi stage mixer with maximum shear and low flow. In one aspect, the emulsified hydrolysate produced using an ultra-high shear mixer has an average particle size of less than about 70, 65, 60, 55, 50, 45, 40, 35, 30, 29, 28, 27, 26 or about 25 microns or less, or 24, 23, 22, 21, 20, 19, 18, 17, 16, 15, 14, 13, 12, 11 or 10 microns or less, or any range between any two recited sizes, preferably about 26 microns or less, or any emulsion mechanically created or created through the use of emulsifying agents. The size of the particles may be measured, for example, with laser light scattering as described herein.

In some aspects, the processes of this disclosure inactivate pathogens in the biological recyclable stream or the environment. The methods of this disclosure are therefore useful in eliminating pathogens present in biological recyclable streams during the production of compositions that can safely be used as fertilizers for the production of produce, other crops, fruits, nuts, flowers and turf, or as animal provender.

In some aspects, the grinder used to grind biological recyclable streams can be a rotary knife grinder, which produces particles in the particulate biological slurry with an average size of about 1/2 of an inch. In some aspects, the ground biological slurry is then pumped to an in-line low RPM/high torque grinder with shredding action to further ensure that the ground biological slurry has an average particle size of about 1/2 of an inch or less. The low RPM/high torque grinder may be used in any process for any system with even low levels of throughput, but is particularly suitable for use in a high throughput processing system, for example, a system capable of processing more than over 50 tons per day, *e.g.,* more than 90 tons/day, or up to 95 or 100 tons per day or more. The ground biological slurry produced by the first grinder, or the first grinder and the optional second grinder, is then pumped into a temperature controlled incubation vessel, where it undergoes constant mixing and incubation with enzyme combination(s) at desired temperatures.

In addition, the incubation vessel may contain a recirculating line connected to an in-line grinder with shearing action which is used during all or a part of the incubation and pasteurization. This may be the third grinder in aspects of the disclosure where an optional in-line grinder with shredding action is used to further grind the ground biological slurry, but it is the second grinder in aspects of the disclosure where the optional grinder is not used. In some aspects, the in-line grinder used during all or a part of the incubation with the enzyme combination comprises a high shear mixer. In some aspects the in-line grinder comprises a high shear mixer with a disintegrating head. In some aspects, the high shear in-line grinder is used beginning at about 30 minutes to about 1 hour after incubation begins and continues through the pasteurization step. In some aspects, the start and run times may vary, and still achieve the same particle size reduction objectives. In some aspects, the particles in the resulting incubated biological hydrolysate may be less than 1/16th and about 1/32nd of an inch. In some aspects, the particles in the resulting hydrolysate may be less than 3/32^{nd}, 1/8^{th}, or 3/8^{th} of an inch. In some aspects, the particles in the resulting incubated biological hydrolysate can be around 1/64^{th} of an inch.

The processes described herein can produce biological particles. In some embodiments, the biological particles comprise bone, cellulose, solidified or semi-solidified fats, nut shells, fish scales, teeth, inorganic minerals, keratin-containing species, or combinations thereof. In some aspects, the keratin-containing species is selected from: beaks, feathers, claws, or hair. Without being bound by theory, the solidified fats can result from incomplete fat hydrolysis, or fats which are soluble at the incubation temperature but become solid or semisolid upon cooling. In some embodiments, the levels of biological particles (*e.g*., solid or semisolid fats) in the can be controlled using controlled centrifugation processes. In some embodiments, the controlled centrifugation processes can include or exclude a fixed number of centrifuge speeds, one or more steps, a ramping centrifuge speed between two or more different centrifuge speeds, and one or more centrifuge times. The biological particles can be separated from the hydrolysate by a variety of methods. In some aspects, the biological particles can be separated by: screens, filters, sedimentation, centrifugation, the use of a hydrocyclone, a rotaspiral drum screen, and a horizontal belt filter. In some aspects, one or a plurality of screens is used to separate biological particles from the biological hydrolysate. In some aspects, screening or filtering of the pasteurized biological hydrolysate through one or more mesh screens may be used to separate the hydrolysate from particles that do not pass through the mesh. In some embodiments, the hydrolysate produced by incubating is then separated using a 30 mesh screen with an opening of 590 microns. In some embodiments, the 30 mesh screen is a vibrating screen. This separates the hydrolysate from particles too large to pass through the mesh, for example, particles having an average diameter larger than 590 microns. The hydrolysate passing through the first screen may then be further separated by filtering through a 200 mesh screen with an opening size of 74 microns. In some aspects, the incubated particles removed from the hydrolysate by screening through the 200 mesh screen have a diameter of greater than 74 microns. In some aspects the screen may be a vibrating screen. In some embodiments, a coarse screen and a fine screen can be used in two steps to separate and isolate the biological particles from the hydrolysate. In some embodiments, a mesh screen having 18-60 mesh may be used in a first screening step ("coarse screen"), for example 18 mesh screen with 1000 micron openings, 20 mesh screen with 841 micron openings, 25 mesh screen with 707 micron openings, 30 mesh screen with 590-595 micron openings, 35 mesh screen with 500 micron openings, 40 mesh screen with 400 micron openings, 45 mesh screen with 354 micron openings, 50 mesh screen with 297 micron openings, or 60 mesh screen with 250 micron openings, or other commercially available coarse screening technologies. The purpose of this screen is to separate solids or semisolids, which can be used to produce animal provender, from the liquid hydrolysate, and can be accomplished through a variety of known screening techniques. In some embodiments a mesh screen having 35 to 400 mesh may be used in the second screening step ("fine screen"), for example, 35 mesh screen with 500 micron openings, 40 mesh screen with 400 micron openings, 45 mesh screen with 354 micron openings, 50 mesh screen with 297 micron openings, or 60 mesh screen with 250 micron openings, 70 mesh screen with 210 micron openings, 80 mesh screen with 177 micron openings, 100 mesh screen with 149 micron openings, 120 mesh screen with 125 micron openings, 140 mesh screen with 105 micron openings, 170 mesh screen with 88 micron openings, 200 mesh screen with 74 micron openings, 230 mesh screen with 63 micron openings, 270 mesh screen with 53 micron openings, 325 mesh screen with 44 micron openings or 400 mesh screen with 37 micron openings, or other commercially available fine screening technologies. The purpose of this screen is: i) to increase particle surface area, thereby increasing the effectiveness of the enzymes used to produce the hydrolysate; ii) to assure the ability of the pasteurized hydrolysate to pass easily through the farmer's drip lines, or other similar equipment; iii) to ensure that the pasteurized hydrolysate is available for metabolism by soil organisms, once it is delivered to the root zone; and iv) to separate the desired level of animal provender from the liquid fraction. This purpose can be accomplished through a variety of known screening techniques. In some aspects, the particles separated by the fine screen, having a diameter between about 74 microns and about 590 microns, may be recycled as a feedstock to be digested in the next batch. This material will digest in the next batch, without accumulating.

The incubated biological particles which are filtered out by the coarse screen, having an average diameter of greater than about 590 microns may be suitable for use as animal provender and/or as a food supplement or other sources of nutrients for carnivorous or omnivorous mammals, such as pigs, chickens or pets. The incubated biological particle compositions are digestible, and have a high conversion rate of food to livestock weights, and/or high pet nutritional value. The incubated biological particles filtered out by the fine screen can be added to the next batch with a selected biological recyclable stream for additional processing. In some aspects, the agricultural admixtures of this disclosure can be used as animal provender with a high conversion rate of food to livestock weights.

In one aspect, the methods described herein can include a stabilization step following incubation of the ground biological slurry with the one or more selected enzymes. In one aspect, the stabilization step can occur after separation of the biological particles from the biological hydrolysate. In some aspects, the separated dewatered aqueous phase produced from the centrifuge separation step can be stabilized before dewatering or after dewatering by the addition of a stabilizer. In some aspects, the stabilizing step can also include a preserving step, for example, by using inorganic acid, organic acid, inorganic preservatives, or organic preservatives, emulsifiers or dispersants, including those which are allowed for use in the production of a certified organic hydrolysate. In some aspects, the separated particles can be stabilized by drying the particles. The drying can be performed by exposing the particles to heat, air, in a vacuum, vibrating filters, or a combination thereof. In some aspects, the separated particles can be stabilized when the particles are moist with the blending of a stabilizer or preservative as described herein. In some aspects, the stabilizing step of the processes of this disclosure comprises the addition and mixing of the liquid hydrolysate with an acid source consisting of hydrochloric, sulfuric, phosphoric, acetic, stearic, propionic, tartaric, maleic, benzoic, succinic acids, lactic, or citric acid, preferably phosphoric acid. Lactic acid, acetic acid, citric acid or other organic certified acids may also be preferably used to make certified organic fertilizer. For example, phosphoric acid or lactic acid may be added to lower the pH of the composition to inhibit microbial and/or pathogenic activity during the storage and transport of the composition which protects the nutrients from further digestion and/or degradation by microbes or pathogens. In some aspects, phosphoric acid may be tri-calcium phosphate. In some aspects, the pH of the stabilized liquid hydrolysate is less than about 3.5. In some aspects, the pH of the stabilized liquid hydrolysate agricultural admixture is from about 2.5 to about 3.5, preferably about 3.0. In some aspects, the pH of the liquid hydrolysate agricultural admixture is selected from: 2.4, 2.5, 2.6, 2.7, 2.8, 2.9, 3.0, 3.1, 3.2, 3.3, 3.4, 3.5, 3.6, 3.7, 3.8., 3.9, or 4.0 or between any of the aforementioned pH levels. The stabilized product may be quarantined overnight, while the contents are tested to assure the elimination of pathogens. Without being bound by theory, the stabilization step can produce a finished product that is shelf stable for at least two years, which can be accomplished through any of a variety of stabilization steps as described herein.

Although the pasteurization step inactivates any bacteria or other pathogens present in the biological recyclable stream or the processing plant, the stabilization prevents growth of pathogens from environmental sources after the pasteurization step. Without stabilization, microbes and pathogens could contaminate and degrade a liquid hydrolysate even after sterilizing the hydrolysate. The stabilized product is buffered in the soil to a pH similar to the soil pH, which, under normal circumstances, will cause the liquid pasteurized hydrolysate to become biologically active, which is the desired mode of action for the product.

A preservative ("stabilizer") such as sorbic acid, potassium sorbate, tocopherol, d-alpha-tocopherol acetate, resveratrol, rosemary oil, erythorbic acid, sodium erythorbate, sodium ascorbate, iso-ascorbic acid, sodium iso-ascorbate, potassium nitrate, ethyl lauroyl arginate, benzoic acid, ascorbyl palmitate, ascorbyl stearate, sulphurous acid, methyl-ρ-hydroxy benzoate, methyl paraben, potassium bisulphite, potassium lactate, sodium lactate, sodium diacetate, butylated hydroxyanisole (a mixture of 2-tertiarybutyl-4- hydroxyanisole and 3-tertiarybutyl-4-hydroxyanisole), butylated hydroxytoluene (3,5-ditertiarybutyl-4-hydroxytoluene), potassium metabisulphite, propyl-ρ-hydroxy benzoate, calcium propionate, calcium sorbate, citric acid esters of mono- and diglycerides, dimethyl dicarbonate, natamycin, propyl gallate, potassium sulfate, thyme extract, potassium benzoate or any other suitable food additive preservative may also optionally be added as a preservative during the stabilization step. For organic fertilizer, tocopherol, D-alpha- tocopherol acetate, natamycin, mined potassium sulfate, or any other food preservative certified for organic use may be added as a preservative. In some aspects, about the preservative is at a concentration from 0.1 to about 2.0%, preferably 0.25% (weight percent) in the agricultural admixture. In some aspects, the concentration of the preservative is 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0 (weight percent) or any weight percentage between any of the aforementioned weight percentages. In some aspects, Tocopherol or D-alpha-tocopherol acetate are added at levels ranging from 10 to 500 mg/kg, or any amount between those values. Natamycin may be added, in some embodiments, at levels, for example of 0.1 to 100 mg/mL, or any amount between those values. In another embodiment, other preservatives may be added (together with the preservatives listed above, "preservatives") and/or those preservatives approved for use in certified organic products ("organic preservatives"). In another aspect, this disclosure relates to agricultural admixtures made from biological recyclables, comprising nutrients released by grinding, shearing, homogenization and enzymatic digestion, and an acid stabilizer, wherein the emulsified hydrolysate has an average particle size of less than about 26 microns and a pH of between about 2.5 and about 3.5, preferably about 3.0.

In one aspect this disclosure relates to methods to collect biological recyclable streams in a manner of handling and timing that does not allow the biological recyclable stream to become putrescent. Putrescent biological waste is characterized by the presence of noxious odors emanating from the biological waste. The noxious odors can comprise methane (CH₄), dihydrogen sulfide (H₂S), carbon dioxide (CO₂) and dimethyl sulfide (CH₃SCH₃). Putrescent biological recyclable streams are typically hauled off to distant landfills, where they can emit greenhouse gases, noxious odors and toxic effluent. In the methods described herein, biological recyclable streams are kept fresh, so that there are no emissions, no effluent, no nuisance odors, and essentially no waste. The processes described herein can take place in an urban warehouse. Supermarket companies served in this method typically distribute food supplies to their supermarkets from a centrally located distribution center, in a warehouse in an urban area. The trucks typically return to the distribution center empty. In this method, the supermarkets use specially made, insulated and sealed totes and buggies to collect food recyclables, which is then returned to the distribution center on a frequent basis. In another aspect, the totes and buggies are double walled. Due to its environmental advantages, the processing facility described in this method can comply with all applicable laws and regulations and qualify to receive all necessary government and regulatory permits and approvals to locate in a warehouse that is at or near the supermarket distribution center. By the methods described herein, the biological recyclable stream can be transported over short distances between the urban processing plant and the supermarket distribution center, thereby eliminating the greenhouse gas emissions involved in otherwise transporting the biological recyclable stream to a landfill which is typically located far from urban centers.

In some aspects, the incubated biological particles produced in step (e) in the methods described herein may be used as animal provender or as a nutrient supplement. In some aspects, the agricultural admixtures described herein comprise energetic content for animal provender. The dry matter of the dried liquid agricultural admixtures can range from 16 to 30 wt%. The crude protein of the dried liquid agricultural admixtures can range from 18 to 40 wt%. The gross energy of the dried liquid agricultural admixtures can range from 5000 to 8000 kcal/kg. The ash percentage of the dried liquid agricultural admixtures can range from 3 to 10 wt%. The acid hydrolyzed ether extract of the dried liquid agricultural admixtures can range from 1 to 9 wt%. The nitrogen free extract of the dried liquid agricultural admixtures can range from 5 to 60 wt.%.

In some aspects, the incubated biological hydrolysate can be emulsified using an ultra-high-shear mixer to produce an emulsified biological hydrolysate. In some aspects, an emulsifying agent can be added to the incubated biological hydrolysate to form an emulsion.

In one aspect, the agricultural admixtures of this disclosure are suitable for use as fertilizer and soil amendment. The high nutrient concentration in the agricultural admixture provides nutrients directly to the plants (including amino acids) and also increases the organic matter in the soil by providing nutrients for soil organisms. These soil organisms which obtain nutrients from the agricultural admixtures of this disclosure grow and promote plant growth, through nitrogen fixation or by providing additional organic nutrients for plants and otherwise improving soil quality. For example, liquid hydrolysates comprising amino acids, fatty acids, sugars, and minerals not only make nutrients directly available to plants, but also improve the soil by sustaining soil organisms including earthworms and microorganisms, including, for example, nitrogen fixing organisms (*e.g*., bacteria and archaea) and aerobic bacteria and fungi (*e.g*., mycorrhizae), nematodes, protozoa, and a range of invertebrates. The amount of soil organisms increases after application to the soil of the biological hydrolysates described herein. The amount of soil organisms can be measured using carbon dioxide respiration, using the methods described in Kallenback et al. (Nature Comm., published on-line November 28, 2016, doi:10.1038/ncomms13630). In some aspects, application of the biological hydrolysates described herein to soil increases the amount of soil organic matter. The soil organic matter content can be measured by pyrolysis-GC/MS (as described in Grandy, et al., Geoderma, 150, 278-286 (2009)).

In some aspects, the agricultural admixtures described herein comprise nutrients. The nutrients can include or exclude amino acids (indispensable and dispensable amino acids), macro minerals, microminerals, carbohydrates, saturated fatty acids, and unsaturated fatty acids. The amino acids can include or exclude arginine, histidine, isoleucine, leucine, lysine, methionine, threonine, phenylalanine, tryptophan, valine, alanine, aspartic acid, cysteine, glutamic acid, glycine, proline, serine, and tryptophan. In some aspects, the range of arginine in the agricultural admixture can be from 0.5 to 5 wt%, preferably 1.0 to 1.5 wt%; the range of histidine can be from 0.2 to 5 wt%, preferably 0.5 to 1.0 wt%; the range of isoleucine can be from 0.2 to 5 wt%, preferably 0.5 to 1.5 wt%; the range of leucine can be from 0.5 to 10 wt%, preferably 1.3 to 2.0 wt%; the range of lysine can be from 0.2 to 5 wt%, preferably 1.0 to 2.0 wt%; the range of methionine can be from 0.2 to 5 wt%, preferably 0.4 to 1.0 wt%; the range of threonine can be from 0.2 to 5 wt%, preferably 0.7 to 1.5 wt%; the range of phenylalanine can be from 0.2 to 5 wt%, preferably 0.5 to 1.5 wt%; the range of tryptophan can be from 0.03 to 5 wt%, preferably 0.1 to 3.0 wt%; the range of valine can be from 0.1 to 5 wt%, preferably 0.7 to 1.5 wt%; the range of alanine can range from 0.1 to 5 wt%, preferably 0.7 to 1.8 wt%; the range of aspartic acid can be from 0.2 to 5 wt%, preferably 1.5 to 2.5 wt%; the range of cysteine can be from 0.03 to 5 wt%, preferably 0.1 to 0.3 wt%; the range of glutamic acid can be from 0.2 to 10 wt%, preferably 2.5 to 4.0 wt%; the range of glycine can be from 0.2 to 10 wt%, preferably 1.0 to 2.0 wt%; the range of proline can be from 0.01 to 5 wt%, preferably 0.03 to 1.5 wt%; the range of serine can be from 0.1 to 5 wt%, preferably 0.5 to 1.0 wt%; and/or the range of tryptophan can be from 0.1 to 5 wt%, preferably 0.4 to 1.0 wt%. In some aspects, the macro minerals can include or exclude: Ca, P, K, Mg, and Na. The range of Ca in the agricultural admixtures can be from 0.1 to 15 wt%, preferably 0.3 to 5.5 wt%; the range of P can range from 0.05 to 15 wt%, preferably 0.2 to 2.5 wt%; the range of K can range from 0.2 to 15 wt%, preferably 0.5 to 1.5 wt%; the range of Mg can range from 0.01 to 5 wt%, preferably 0.08 to 0.2 wt%; and/or the range of Na can range from 0.05 to 5 wt%, preferably 0.2 to 0.8 wt%. In some aspects, the microminerals can include or exclude Cu, Fe, Zn and Mn. In some aspects, the range of Cu in the agricultural admixtures can be from 0.1 to 100 ppm, preferably from 2 to 11 ppm; the range of Fe can be from 10 to 1000 ppm, preferably from 90 to 225 ppm; the range of Zn can be from 10 to 1000 ppm, preferably from 15 to 90 ppm; and/or the range of Mn can be from 0.1 to 200 ppm, preferably from 5 to 25 ppm. In some aspects the carbohydrates can include or exclude: fructose, glucose, sucrose, stachyose, starch, acid detergent fiber, neutral detergent fiber, acid detergent lignin, hemicellulose, and cellulose. In some aspects, the range of fructose can be from 0.5 to 20 wt%, preferably from 2 to 8 wt%; the range of glucose can be from 0.5 to 20 wt%, preferably from 2 to 11 wt%; the range of sucrose can be from 0.01 to 20 wt%, preferably from 0.02 to 0.08 wt%; the range of stachyose can be from 0 to 2 wt%, preferably from 0.01 to 0.12 wt%; the range of starch can be from 0.01 to 20 wt%, preferably from 0.3 to 6 wt%; the range of acid detergent fiber can be from 0.01 to 40 wt%, preferably from 0.8 to 23 wt%; the range of neutral detergent fiber can be from 0.5 to 45 wt%, preferably from 2 to 32 wt%; the range of acid detergent lignin can be from 0 to 20 wt%, preferably from 0.4 to 8 wt%; the range of hemicellulose can be from 0 to 20 wt%, preferably from 0 to 12 wt%; and/or the range of cellulose can be from 0.01 to 25 wt%, preferably from 0.6 to 14 wt%. In some aspects, the saturated fatty acids of the agricultural admixtures can include or exclude myristic (14:0), C15:0, palmitic (16:0), margaric (17:0), stearic (18:0), arachidic (20:0), behenoic (22:0), and lignoceric (24:0). In some aspects, the range of myristic acid can be from 1.0 to 15 wt%, preferably from 2 to 4 wt%; the range of C15:0 can be from 0.1 to 2 wt%, preferably from 0.2 to 0.5 wt%; the range of palmitic acid can be from 1.0 to 45 wt%, preferably from 20 to 30 wt%; the range of margaric acid can be from 0.1 to 15 wt%, preferably from 0.5 to 2 wt%; the range of stearic acid can be from 1.0 to 30 wt%, preferably from 9 to 15 wt%; the range of arachidic acid can be from 0 to 5 wt%, preferably from 0.1 to 0.5 wt%; the range of behenoic acid can be from 0 to 5 wt%, preferably from 0.05 to 0.25 wt%; and/or the range of lignoiceric acid can be from 0 to 5 wt%, preferably from 0.02 to 0.2 wt%. In some aspects, the unsaturated fatty acids can include or exclude myristoleic (9c-14:1), palmitoleic (9c-16:1), elaidic acid (9t-18:1), oleic acid (9c-18: 1), vaccenic acid (11c-18:1), linoelaidic acid (18:2t), linoleic acid (18:2n6), linolenic acid (18:3n3), stearidonic (18:4n3), gonodic acid (20:ln9), c20:2, homo-α-linolenic (20:3n3), arachidonic (20:4n6), 3n-archidonic (20:4n3), EPA (22:ln9), erucic (22:ln9), clupanodonic (22:5n3), DHA (22:6n3), and nervonic (24:ln9). In some aspects, the range of myristoleic can be from 0 to 5 wt%, preferably from 0.3 to 0.8% wt.%; the range of palmitoleic can be from 0.5 to 15 wt%, preferably from 2 to 4 wt.%; the range of elaidic can be from 0.5 to 15 wt%, preferably from 2 to 5 wt.%; the range of oleic can be from 33 to 43 wt.%; the range of vaccenic can be from 2 to 3 wt.%; the range of linoelaidic can be from 0 to 1.5 wt%, preferably from 0.01 to 0.03 wt.%; the range of linoleic can be from 0.5 to 45 wt%, preferably from 10 to 25 wt.%; the range of linolenic can be from 0.51 to 15 wt%, preferably from 1 to 2.5 wt.%; the range of gonodic can be from 0 to 5 wt%, preferably from 0.03 to 0.5 wt.%; the range of c20:2 can be from 0 to 5 wt%, preferably from 0.1 to 0.2 wt.%; the range of homo-a-linolenic can be from 0 to 1.5 wt%, preferably from 0.02 to 0.03 wt.%; the range of arachidonic can be from 0.05 to 1.5 wt%, preferably from 0.15 to 0.3 wt.%; the range of EPA can be from 0 to 5 wt%, preferably from 0.05 to 0.25 wt.%; the range of erucic can be from 0 to 5 wt%, preferably from 0.2 to 0.15 wt.%; the range of clupanodonic can be from 0 to 1 wt%, preferably from 0.2 to 0.08 wt.%; the range of DHA can be from 0 to 1.5 wt%, preferably from 0.05 to 0.15 wt.%; and/or the range of nervonic can be from 0 to 1 wt%, preferably from 0.01 to 0.05 wt.%. The range of any of the aforementioned nutrients can be a percentage between any two recited percentages.

When the soil organisms obtaining nutrients from the agricultural admixtures of this disclosure die they decay and in turn provide more organic nutrients for the soil organisms and the plants, providing additional organic matter and nutrients for plants over a sustained period of time and increasing soil organic matter. The increase in organic matter in the soil stimulates plant root growth, flowering, and fruiting, and increases crop yields. In one aspect, the agricultural admixtures of this disclosure may more than double soil organic matter. In some aspects, the agricultural admixtures of this disclosure may increase soil organic matter by up to 150% or more, preferably increasing soil organic matter by between about 10% and about 150%, depending on the initial level of soil organic matter.

Agricultural admixtures produced from selected biological recyclable streams contain more nutrients than compost mixtures obtained using standard composting processes which take up to 3 months, and result in degradation of organic nutrients resulting in reduction of the carbon content through conversion to carbon dioxide (CO₂), methane (CH₄), ethanol (C₂H₅OH), hydrogen sulfate (H₂S) and other related effluent by-products of rotting and fermenting.

In some aspects, the methods described herein are performed under aerobic conditions, with little decomposition. In some aspects, the methods described herein are performed in the presence of added oxygen during the incubation and/or pasteurization steps. The oxygen can be added by sparging the incubation solution with oxygen gas. The oxygen can be introduced at an amount between about 0.1 atm to 10 atm. In some aspects, the amount of added oxygen is selected from: 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, or 10 atm, or any range between the aforementioned values. In some aspects, the methods described herein are performed in the presence of ambient oxygen levels with no added oxygen.

In some aspects, the methods described herein are performed, for example, in less than about 2 to about 12 hours or more, for example, about 3 to about 4 hours, preferably about 3 hours.

### Use of agricultural admixtures to enhance crop and/or produce yields

In another aspect, application of the agricultural admixtures of this disclosure as a hydrolysate-based fertilizer can eliminate or reduce the use of conventional nitrate or ammonia based fertilizers such as urea nitrate, ammonium nitrate, calcium ammonium nitrate, or other nitrate or ammonia based fertilizers, while also improving crop yields relative to the use of nitrate fertilizers alone. The agricultural admixtures of this disclosure may promote faster initial growth after germination, increase root growth, increase canopy growth, increase field and/or greenhouse crop yields and/or increase the quality or flavor of the produce relative to the use of nitrate fertilizers alone, for example by increasing the levels of sugar and/or other flavor components. Moreover, when the fertilizers of this disclosure are used in combination with nitrate or ammonia based fertilizers, plant growth is improved, including, for example, more vigorous root growth to form more extensive root systems. This results in uptake of a higher percentage of nitrate or ammonia based fertilizers by more extensive root systems of the treated plants, thereby further decreasing the amount of nitrate run off beyond the reduction in the amount of nitrate or ammonia based fertilizer applied and increasing water and nitrate use efficiency. In addition to polluting ground water and causing aquatic life killing eutrophication in the nation's waterways, excessive use of nitrate or ammonia based fertilizers also causes the release of nitrous oxide (N₂O), a greenhouse gas that is over 300 times as damaging as carbon dioxide, according to the United States Environmental Protection Agency (EPA).

In one aspect the fertilizers of this disclosure may be applied using irrigation drip lines. In some aspects, the stock agricultural admixture fertilizers of this disclosure are diluted prior to use. For example, the agricultural admixtures may be diluted with water to 1/5, 1/6, 1/7, 1/8, 1/9, 1/10 or in some applications, to as little as 5%, 4%, 3%, 2%, or 1% or less prior to use. In some aspects, the agricultural admixtures may be presented in a dry powder form, and dissolved in water prior to use. Preferably the agricultural admixture is diluted to 1/10 or as low as 1% (wt.) or less prior to use. In some aspects, the suitability of the agricultural admixtures of this disclosure for use with drip irrigation without clogging drip lines results from grinding and emulsification of water and oil soluble particles in the hydrolysates. Flushing and/or cleaning of drip lines with water following may also be desirable to avoid microbe growth in drip lines following application of the hydrolysates of this disclosure. In some aspects, the agricultural admixture is applied to crops by spraying, preferably via a sprinkler. In some aspects, the agricultural admixture is blended with a soil amendment, e.g., manure or rendering byproducts, before application of the soil amendment to the soil before or during crop growth.

In another aspect, this disclosure relates to a method of increasing the yield of produce, the method comprising applying by drip line irrigation a composition comprising an agricultural admixtures made from selected biological recyclable streams, the agricultural admixture comprising nutrients released by grinding, shearing, homogenization and enzymatic digestion, and an acid stabilizer, wherein the agricultural admixture has an average particle size of less than about 30 microns and a pH of between about 2.5 and 3.5, wherein the yield of produce is increased by at least 10% in some crops, and over 40% in other crops compared to treatment with nitrate or ammonia based fertilizer alone. In some aspects, the (diluted) agricultural admixture is applied in combination with nitrate or ammonia based fertilizer, either through separate application on the same or different schedules, or by combining the admixture and nitrate or ammonia based fertilizer in a mixture. For example, the agricultural admixture may be applied in a 90:10, 85:15, 80:20, 75:25, 70:30, 65:35, 60:40, 55:45 50:50, 45:55, 40:60, 35:65, 30:70, 25:75, 20:80, 15:85, or 10:90 mixture (v/v) or any ratio between any of the aforementioned ratios, or in that ratio in combination with a nitrate or ammonia based fertilizer.

In some aspects, application of the fertilizers of this disclosure increase crop yield relative to the use of nitrate fertilizers alone, as described herein, even when the amount of nitrate or ammonia based fertilizer is decreased. Preferably the use of the hydrolysate-based fertilizers of this disclosure increase crop yield relative to nitrate fertilizer alone by at least 10%, 15%, 20%, 25%, 30%, 35% 40%, 45%, 50%, 60%, 70%, 80%, 90%, 100%, 200%, 300%, 400%, or at least 10% over a growing season.

In some aspects, the agricultural admixtures produced by the methods described herein can be blended or mixed with a dispersant to prevent fats and/or oils in the admixture from adsorbing to the delivery lines thus improving flow through the delivery lines when the agricultural admixture is applied as a fertilizer to crops. It was surprisingly discovered that adding a dispersant to the agricultural admixture also significantly improves emulsion formation of the agricultural admixture. Emulsion formation in low-fat content agricultural admixture liquids was found to be difficult without the addition of a dispersant because the fat provided the source of hydrolysable lipids which was the primary source of surfactant in a dispersant-free medium. The inventors recognized that when the fat and/or oil content is reduced in the agricultural admixture by the methods described herein, including the use of a tricanter centrifuge, the viscosity of the agricultural admixture increased before addition of a dispersant. In some aspects, the use of the tricanter centrifuge reduce the fat content from about 6-12 wt.% before centrifugation to a range of 0.2-1.4 wt.% after centrifugation, depending on the feedstock input and the centrifuge parameters. According to the present invention, the fat content of the pasteurized first incubated hydrolysate is reduced to a range of between 1 to 4 wt% by tricanter centrifugation. The inventors recognized that the levels of added dispersant were lower than the amount of fats removed from the agricultural admixture. In some aspects, addition of dispersant to reduced-fat content agricultural admixtures enables the administration of low-fat content agricultural admixtures to plants. In some aspects, the dispersant can be a product listed in the EPA Product Schedule, June 2016, which includes: ACCELL CLEAN^{®} DWD (D-16) (Advanced BioCatalytics Corporation, California), BIODISPERS (D-9) (Petrotech America Corporation, New York), COREXIT^{®} EC9500A (D-4) (Nalco Environmental Solutions LLC, Texas), COREXIT^{®} EC9500B (D-19) (Nalco Environmental Solutions LLC, Texas), COREXIT^{®} EC9527A (D-1) (Nalco Environmental Solutions LLC, Texas), DISPERSIT SPC 1000TM (D-5) (U.S. Polychemical Corp., New York), FFT-SOLUTION^{®} (D-17) (Fog Free Technologies, LLC, South Carolina), FINASOL^{®} OSR 52 (D-11) (TOTAL FLUIDES, France), JD-109 (D-6) (GlobeMark Resources Ltd., Texas), JD-2000^{™} (D-7) (GlobeMark Resources Ltd., Texas), MARE CLEAN 200 (D-3) (Ichinen Chemicals Co., Ltd, Japan), MARINE D-BLUE CLEAN^{™} (D-18) (AGS Solutions, Inc., Texas) , NEOS AB3000 (D-2) (NEOS Company Limited, Japan), NOKOMIS 3-AA (D-14) (Mar-Len Supply, Inc., Hayward, CA),NOKOMIS 3-F4 (D-8) (Mar-Len Supply, Inc., Hayward, CA), SAF-RON GOLD (D-12) (Sustainable Environmental Technologies, Inc., Atlanta, GA), SEA BRAT #4 (D-10) (B.R.A.T. Microbial Products Inc., Texas), SEACARE ECOSPERSE 52 (AKA of FINASOL^{®} OSR 52) (TOTAL FLUIDES, France), SEACARE E.P.A. (AKA of DISPERSIT SPC 1000^{™}) (TOTAL FLUIDES, France), ZI-400 (D-13) (Z.I. Chemicals, Los Angeles, CA), and/or ZI-400 OIL SPILL DISPERSANT (AKA of ZI-400) (Z.I. Chemicals, Los Angeles, CA).

In some aspects, the dispersant can be a surface active agent. The surface active agent can include or exclude: Polyethylene glycol alkyl ethers, Octaethylene glycol monododecyl ether, Pentaethylene glycol monododecyl ether, Glucoside alkyl ethers, Decyl glucoside, Lauryl glucoside, Octyl glucoside, Polyethylene glycol, Octylphenyl ethers, Polyethylene glycol alkylphenyl ethers, Nonoxynol-9, Glycerol alkyl esters, Glyceryl laurate, Polyoxyethylene glycol sorbitan alkyl esters, Sorbitan alkyl esters, Cocamide MEA, Dodecyldimethylamine oxide, Cetrimonium bromide (CTAB), Cetylpyridinium chloride (CPC), Benzalkonium chloride (BAC), Benzethonium chloride (BZT), Dimethyldioctadecylammonium chloride, Dioctadecyldimethylammonium bromide (DODAB), Docusate (dioctyl sodium sulfosuccinate), Perfluorooctanesulfonate (PFOS), Perfluorobutanesulfonate, Alkyl-aryl ether phosphates, Alkyl ether phosphates, Sodium Stearate, Sodium lauroyl sarcosinate, Perfluorooctanoate (PFOA or PFO), Ammonium lauryl sulfate, Sodium lauryl sulfate, Phosphatidylserine, Phosphatidylethanolamine, Phosphatidylcholine, Arkopal N-300 (C9H19C6H4O(CH2CH2O)30H), Brij 30 (polyoxyethylenated straight chain alcohol), Brij 35 (C12H25O(CH2CH2O)23H), Brij 56 (C16H33O(CH2CH2O)10H), Brij 58 (C16H33O(CH2CH2O)20H), EGE Coco (ethyl glucoside), Genapol X-150 (C13H27O(CH2CH2O)15H), Tergitol NP-10 (nonylphenolethoxylate), Marlipal 013/90 (C13H27O(CH2CH2O)9H), Pluronic PE6400 (), Sapogenat T-300 (C4H9)3C6H2O(CH2CH2O)30H), T-Maz 60K (ethoxylated sorbitan monostearate), T-Maz 20 (ethoxylated sorbitan monolaurate), Triton X-45 (C8H17C6H4O(CH2CH2O)5H), Triton X-100 (C8H17C6H4(OC2H4)10OH), Triton X-102(C8H17C6H4O(CH2CH2O)12H), Triton X-114 (C8H17C6H4O(CH2CH2O)7.5H), Triton X-165 (C8H17C6H4O(CH2CH2O)16H), Tween 80 (C18H37-C6H9O5-(OC2H4)20OH), Cocamidopropyl betaine, Ethoxylated nonylphenol, Diethanolamine, Propylene glycol, Oleic acid sorbitan monoester, Coconut oil monoethanolamide, Polyethylene glycol) monooleate, Polyethoxylated tallow amine, Dipropylene glycol methyl ether, and combinations thereof. In some aspects, the concentration (weight percent) of the dispersant in the blend with the agricultural admixture can be selected from 0.5%, 1.0%, 3%, 5%, 7%, and 9%. In some aspects, the dispersant concentration (weight percent) can range from: 0.1-1.0%, 1.0-3.0%, 3.0-5.0%, 5.0-7.0%, or 7.0-9.0%, or any percentage between any of the aforementioned percentages. In some aspects, the concentration (weight percent) of the dispersant can be selected from: 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1., 2.2., 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4., 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3., 9.4, and 9.5%, or any percentage between any of the recited percentages.

In some aspects, the dispersant can be added to the incubated mixture during the incubation to solubilize the fats and oils. In some aspects, the dispersant can be added before, or during, the emulsification step to produce an emulsified agricultural admixture comprising the dispersant.

In some aspects, the agricultural admixtures produced by the methods described herein can be processed according to the methods disclosed herein, blended or mixed with an inorganic mineral to create a high mineral content admixture. In some aspects, the inorganic mineral is blended with the processed agricultural admixture. The resultant blended admixture with inorganic mineral can feed microbes with organic and inorganic nutrients to synergistically enhance nutrient uptake directly and indirectly (from microbes) into the plant rootstock. In some aspects, the inorganic mineral can be selected from: basalt, granite, glauconite (greensand), and biotite. In some aspects, the inorganic mineral can be basalt. In some aspects, methods of increasing crop yields relative to nitrate fertilizers alone can comprise the steps of contacting the crop with an agricultural admixture comprising basalt. In some aspects, the blending or mixing of an inorganic mineral with the agricultural admixtures described herein can be exothermic. In some aspects, the agricultural admixtures described herein can be combined with a carbon source. In some aspects, the carbon source can include or exclude: ground corn meal, ash, charcoal, wood chips, mulch, and waste carbon. In some aspects, the carbon source can be the coarse-screened particles from the processes described herein, which may otherwise be difficult to incorporate into commercial organic fertilizer and feed products. In some aspects, the carbon source can be the screened particles from the enzymatic digested fresh food recyclables described herein. In some aspects, the screened particles from the processed described herein can be the screened particles obtained from the course screen and/or fine screen filters. The compost derived from such basalt rock dust and coarse-screened particles may stimulate the growth of organic crops and organic dairy and rangeland alfalfa and hay, and contribute to the viability of regenerative agriculture.

In some aspects, the agricultural admixtures described herein can be effective in increasing crop yields relative to nitrate fertilizers in high-stressed growing conditions. In some aspects, the high-stressed growing conditions can comprise high salinity soil. In some aspects, the high-stressed growing conditions can comprise reduced water irrigation amounts. In some aspects, the high-stressed growing conditions can comprise irrigation with high-salinity water. In some aspects, the high-stressed growing conditions can comprise low soil water content (including growing during or after a drought), high temperature (greater than 32.2 °C (90 °F)), and/or soils comprising low levels of micronutrients. The agricultural admixtures described herein increase soil microbe population, which in turn increases the uptake of saline from the irrigation water and/or soil, which reduces the saline exposure to the crops. The agricultural admixtures described herein can be used to increase the area of usable cropland.

In some aspects, the application of the fertilizers of this disclosure increase crop yields relative to nitrate fertilizers under high salinity conditions. The soil can comprise a high mineral content which is otherwise inhospitable for plant growth. Application of the fertilizer can promote crop yield when applied to the soil before or during plant growth. The inventors have determined that the agricultural admixtures produced by the methods described herein can be used to protect crops from irrigation with high salinity water. In some aspects, crops fertilized with agricultural admixtures produced by the methods described herein can be irrigated with high salinity water comprising up to 200 ppm (parts per million) sodium chloride (NaCl) with no reduction in crop yield compared to crops irrigated with normal water and a control nitrate fertilizer (e.g., obtaining "high crop yields" compared to the yields that would be obtained when irrigating with high salinity water when using standard fertilizers).

In some aspects, higher crop yields than nitrate fertilizers can be obtained in high salinity soil using the agricultural admixtures of this disclosure by a method comprising the steps of:
(a) providing an agricultural admixture produced by the methods described herein;
(b) applying the agricultural admixture to a plant; and
(c) irrigating the plant with water.

In some aspects, beneficial soil microbes or fungi can be added to the agricultural admixture before applying the admixture to crops. This may be done in a fermentation tank in water, where the temperature, pH, oxygen levels, and agitation are maintained at levels to maximize the increase in microbial colony counts prior to shipping the production from such fermentation tank to farms for application to crops through their irrigation system. The beneficial soil microbes, beneficial bacteria, and/or beneficial fungi can undergo colony expansion between the processing time and the presentation to the crops. Alternatively, the agricultural admixture may be applied in the soil through farm irrigation equipment, followed shortly thereafter by application of beneficial soil microbes or fungi, thus facilitating the increase of colony expansion for such microbes. In some aspects, when soil microbes are added to the agricultural admixture, the stabilization step may not be performed so as to ensure the viability of the microbes.

### BRIEF DESCRIPTION OF FIGURES

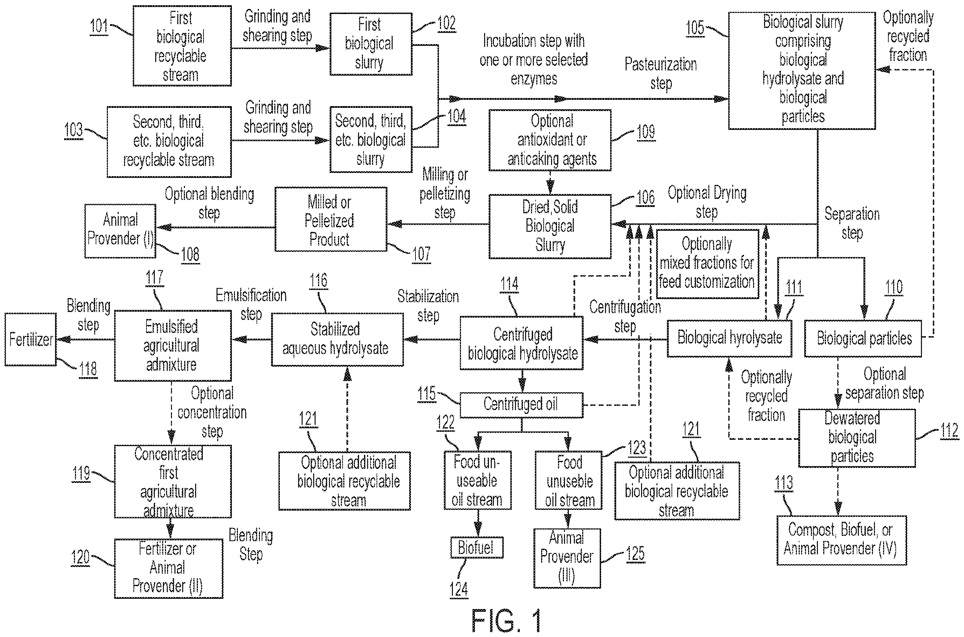

**FIG. 1** is a flowchart showing the process steps involved in one embodiment of this disclosure. A first biological recyclable stream is subjected to a grinding and shearing step to produce a first biological slurry. The first biological slurry is incubated with one or more selected enzymes. An optional second biological recyclable stream is subjected to a grinding and shearing step to produce an optional second biological slurry. The first and optional second biological slurries are then mixed to form a mixed first and second biological slurry. The mixture of the first and second biological slurry is then incubated with one or more selected enzymes, then pasteurized to produce a mixed biological slurry comprising mixed biological hydrolysate and mixed biological particles. The first biological slurry is then subjected to a separation step yielding first biological particles and first biological hydrolysate. The first biological hydrolysate is then subject to a stabilization step yielding a stabilized first biological hydrolysate. The stabilized first biological hydrolysate is then emulsified to form a first agricultural admixture. The first agricultural admixture is optionally subject to a drying step to produce a dried first agricultural admixture.

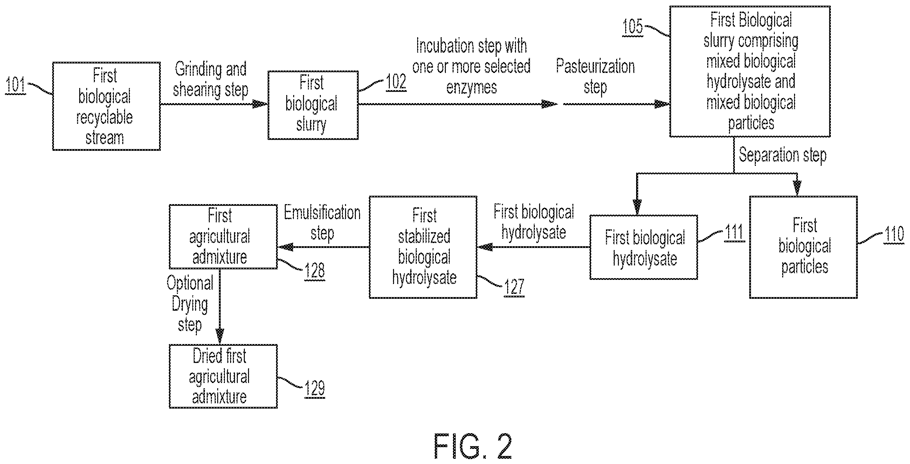

**FIG. 2** is a flowchart showing the process steps involved in one embodiment of this disclosure from a first recyclable stream as an example. Additional recyclable streams can be incorporated at any point of the process steps with a corresponding process output. The first biological slurry is then subjected to a separation step yielding first biological particles and first biological hydrolysate. The first biological hydrolysate is then subject to a stabilization step yielding a stabilized first biological hydrolysate. The stabilized first biological hydrolysate is then emulsified to form a first agricultural admixture. The first agricultural admixture is optionally subject to a drying step to produce a dried first agricultural admixture.

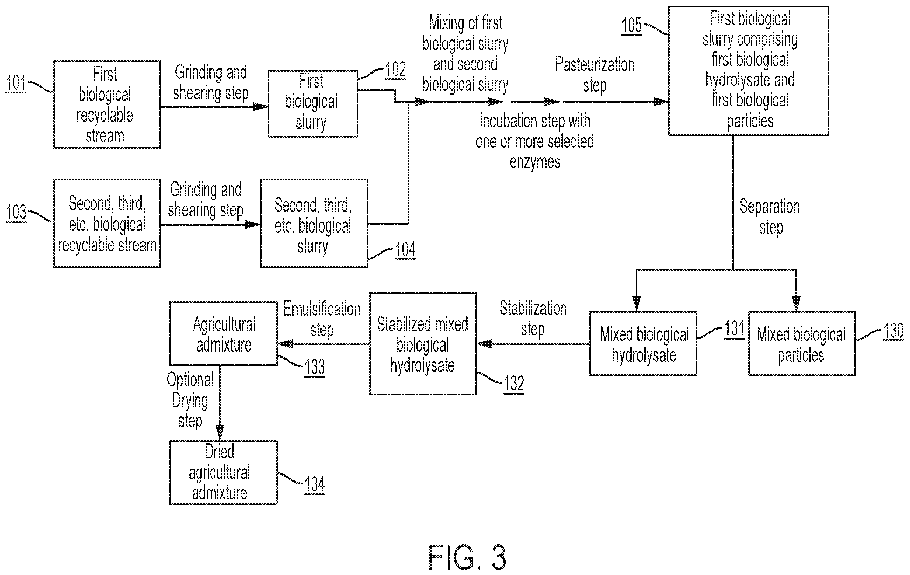

**FIG. 3** is a flowchart showing the process steps involved in one embodiment of this disclosure from selected first and second biological recyclable streams where the mixed biological hydrolysate is stabilized to form the stabilized mixed biological hydrolysate when is then emulsified and dried to form a dried form of the agricultural admixture.

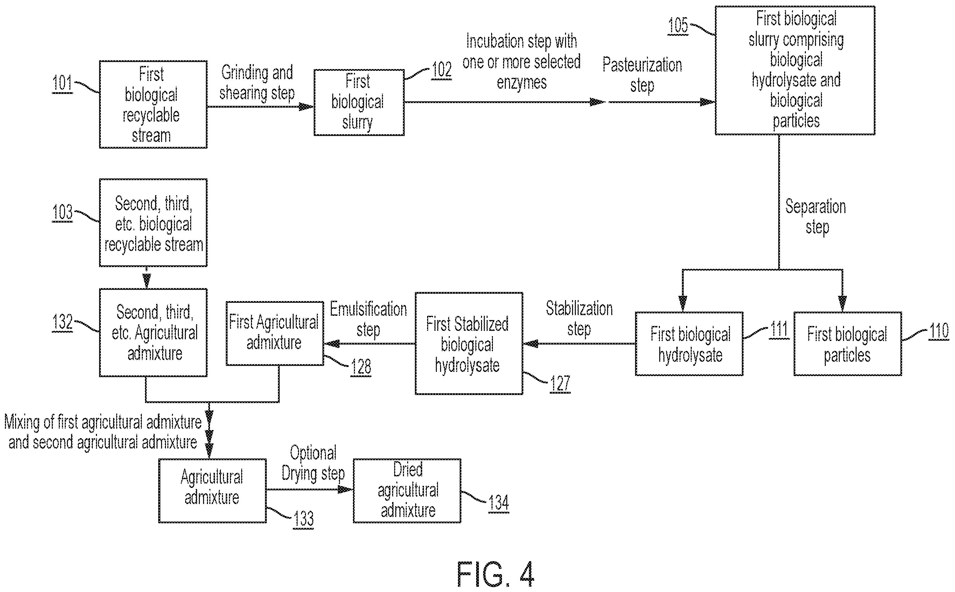

**FIG. 4** is a flowchart showing the process steps involved in one embodiment of this disclosure from selected first and second biological recyclable streams, where the agricultural admixtures are mixed together near the end of the process. A first biological recyclable stream is processed according to the steps described in FIG. 1 to produce a first agricultural admixture. A second biological recyclable stream is processed according to the steps described in FIG. 1 to produce a second agricultural admixture. The first and second agricultural admixtures are then mixed to form a mixed agricultural admixture. The mixed agricultural admixture is then optionally subject to a drying step to produce a mixed dried agricultural admixture.

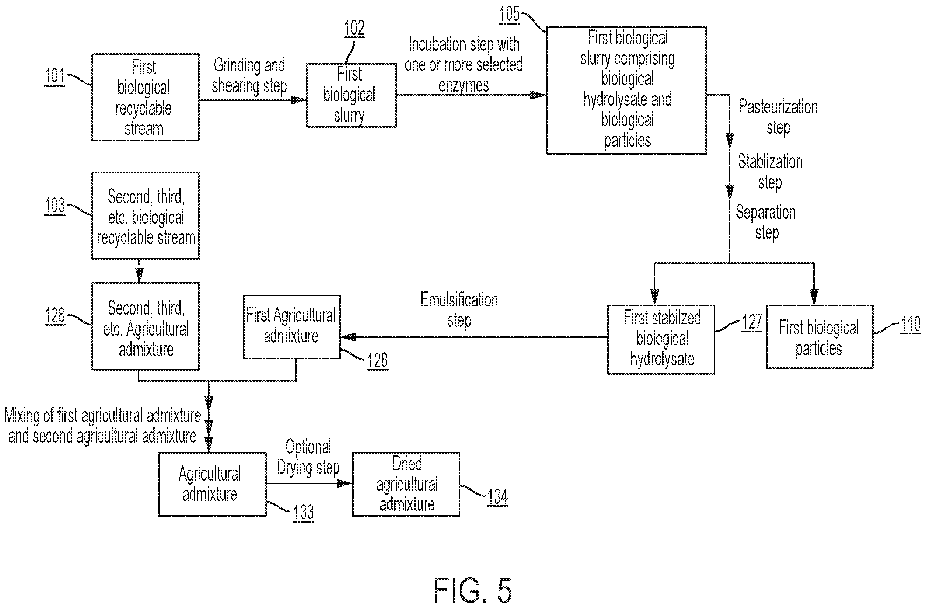

**FIG. 5** is a flowchart showing the process steps involved in one embodiment of this disclosure from selected first and second biological recyclable streams, where the stabilization step is performed after the pasteurization step and before the separation step.

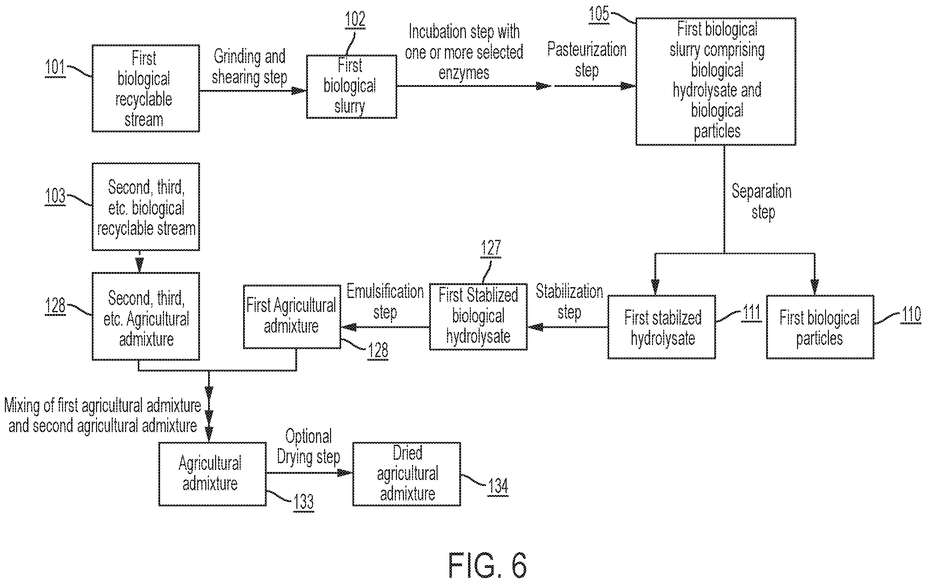

**FIG. 6** is a flowchart showing the process steps involved in one embodiment of this disclosure from selected first and second biological recyclable streams, where the stabilization step is performed on the first and second biological hydrolysates after the separation step and before the emulsification step.
**FIG. 7** is a flowchart showing the process steps involved in one embodiment of this disclosure from selected first biological recyclable streams, where the first biological particles are mixed with the dried agricultural admixture and optionally separated bread products to produce Animal Provender (V).
**FIG. 8** is a flowchart showing the process steps involved in one embodiment of this disclosure from selected first and second biological recyclable streams, where the first biological particles are mixed with the mixed dried agricultural admixture from the first and second biological recyclable streams to produce Animal Provender (VI).

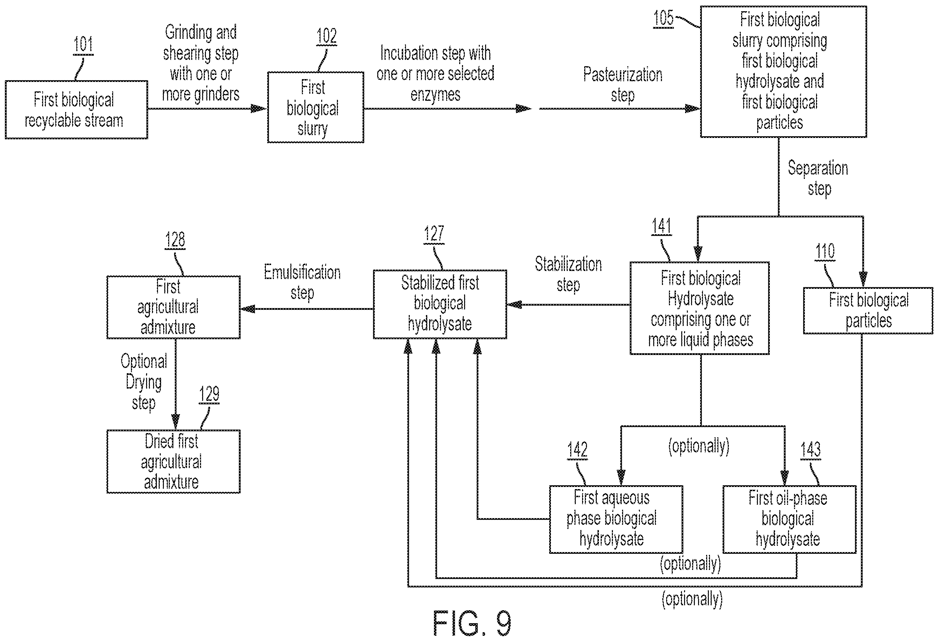

**FIG. 9** is a flowchart showing the optional steps of separating the biological hydrolysate into one or more liquid phases and solid particles. The one or more liquid phases can optionally be separated into an oil-phase and an aqueous phase. The oil-phase can be optionally mixed into the stabilized biological hydrolysate. The aqueous phase can be optionally mixed into the stabilized biological hydrolysate. The separated biological particles can be optionally mixed in the stabilized biological hydrolysate.

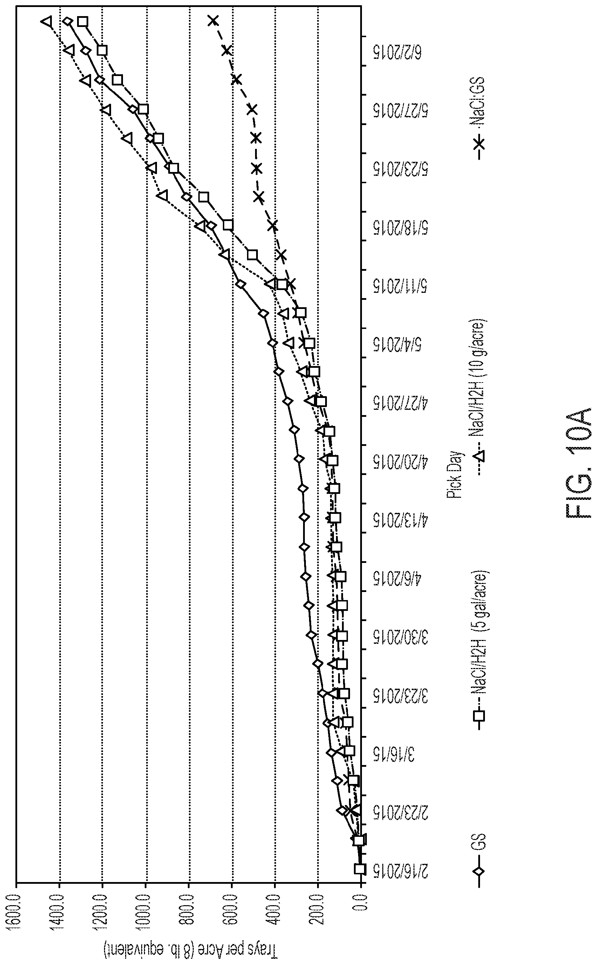

**FIG. 10A** is a chart demonstrating that the agricultural admixture compositions of this invention protects plants against the stress of high salt. The strawberry crop yield as a function of harvest time for four different sections of crop where each section is separately treated with water, 200 ppm saline water, Grower's Standard control fertilizer, Grower's Standard with an agricultural admixture of this disclosure applied in an amount of 46.77 1/ ha (5 gallons per acre) and Grower's Standard with an agricultural admixture of this disclosure applied in an amount of 93.54 l/ha (10 gallons per acre).
**FIG. 10B** is a chart demonstrating the cumulative revenue per acre of cohorts treated with agricultural admixture compositions of this invention relative to grower's standard.
**FIG. 11A** shows graphs of the average concentration of nitrate in leachate from bioassay chambers treated with bonemeal ("bone") organic fertilizers in combination with H2H. Referring to the points on day 28, the graphs for bone, H2H, bone +H2H and water are shown in descending order.
**FIG. 11B** shows graphs of the average concentration of nitrate in leachate from bioassay chambers treated with feathermeal ("feather" ("bone") organic fertilizers in combination with H2H. Referring to the points at day 28, the graphs for feather +H2H, feather, H2H, and water are shown in descending order.
**FIG. 11C** shows graphs of the average concentration of nitrate in leachate from bioassay chambers treated with bloodmeal ("blood") organic fertilizers in combination with organic fertilizers. Referring to the points at day 28, the graphs for H2H, blood, water and blood +H2H are shown in descending order.
**FIG. 12A** show bar plots of the average nitrate concentrations (in ppm) in leachate after Day 3 in experiment # 1 bioassay chambers treated with bonemeal organic fertilizer alone and in combination with H2H. Bars are standard error of measurement and asterisks denote statistical differences P < 0.05.
**FIG.12B** show bar plots of the average nitrate concentrations (in ppm) in leachate after Day 14 (FIG. 12B) in experiment # 1 bioassay chambers treated with bonemeal organic fertilizer alone and in combination with H2H. Bars are standard error of measurement and asterisks denote statistical differences P < 0.05.
**FIG. 13** shows a graph of the average ammonium concentrations in amendments from experiment #1 bioassay chambers treated with bonemeal organic fertilizer alone and in combination with H2H. Referring to the points at day 28, the graphs for water, bone + H2H, bone and H2H are shown in descending order.
**FIG. 14** shows a bar plot of the average ammonium concentrations in leachate from experiment #1 bioassay chambers treated with feather meal organic fertilizer alone and in combination with H2H. Bars are standard error of measurement and asterisks denote statistical differences P < 0.05.
**FIG. 15** show a bar plot of the average tomato plant height after 30 days in soil treated with different combinations of organic fertilizers and H2H. Bars are standard error of measurement. Bo+H2H = bonemeal with H2H. Bl+H2H = bloodmeal with H2H. Fea+H2H = feathermeal with H2H.
**FIG. 16****.** shows a bar plot of the average dry weight of tomato plant leaf and stem biomass after 30 days in soil treated with different combinations of organic fertilizers and H2H. Bars are standard error of measurement. Bo+H2H = bonemeal with H2H. Bl+H2H = bloodmeal with H2H. Fea+H2H = feathermeal with H2H.
**FIG. 17****.** shows a bar graph of comparative body weights of growing-finishing pigs fed with an agricultural admixture of this disclosure and soymeal control feeds. Control bars (blue) are on the left for each day; the bars for pigs fed the agricultural admixture of the disclosure are shown on the right (orange).
**FIG. 18****.** shows a bar plot of the average daily weight gain of growing-finishing pigs fed with an agricultural admixture of this disclosure compared to soymeal control diet. The control bars is on the left; the bar for pigs fed the agricultural admixture of the disclosure is on the right (orange).
**FIG. 19****.** shows a bar plot of body weights of nursery pigs fed with an agricultural admixture of this disclosure compared to soymeal control diet. Control bars (blue) are on the left for each day; the bars for pigs fed the agricultural admixture of the disclosure are shown on the right (orange).
**FIG. 20** shows images of representative chicks from each feed cohort: Control, 50:50, and 75:25 (Control:Ag-admixture/bread) after 11 days of feeding.
**FIG. 21** shows the mean weights per chick per each feed cohort per day. The 75:25 feed cohort had consistently higher feed uptake per bird per day.
**FIG. 22** shows the mean average weight of the chicks per each feed cohort per day in line format.
**FIG. 23** shows the average weight gained per feed cohort. The 75:25 feed cohort had consistently higher average weight gain than the other cohorts.
**FIG. 24** shows the average feed intake per bird for each feed cohort. The 75:25 feed cohort had the highest feed uptake per bird per day.
**FIG. 25** shows the average feed intake per bird in line format.
**FIG. 26** shows the feed conversion ratio per bird for each feed cohort. The 75:25 feed cohort and Control feeds had higher feed conversions than the 50:50 feed cohort. The feed conversions plateaued for all cohorts after day 10 of feeding.
**FIG. 27** shows the feed conversion ratio per each cohort in line format.
**FIG. 28** shows the digestibility of each feed cohort. Both Ag-admixture/bread feeds exhibited a higher digestibility than the Control feed.
**FIG. 29** shows a chart of the cumulative marketable production of strawberries per pick day for cohorts treated with grower's standard, grower's standard with basalt, grower's standard with basalt and H2H (an emulsified agricultural admixture produced by the methods described herein), and grower's standard with H2H. The crops were from Ventura County, California, for experiments performed in the growing season of 2017.
**FIG. 30** shows a chart of the mean weight per marketable fruit of strawberry cohorts treated with grower's standard, grower's standard with basalt, grower's standard with basalt and H2H (an emulsified agricultural admixture produced by the methods described herein), and grower's standard with H2H.
**FIG. 31** shows a chart of the cumulative revenue differential from grower's standard of strawberry cohorts treated with grower's standard, grower's standard with basalt, grower's standard with basalt and H2H (an emulsified agricultural admixture produced by the methods described herein), and grower's standard with H2H. The revenue is calculated by the dollars per acre less the gross costs, and excludes the cost of administering the test program.
**FIG. 32** shows the cumulative weight yield of marketable strawberries picked for cohorts treated with grower's standard compared to blends of grower's standard with agricultural admixtures to crops stressed with high heat (over 32.2 °C (90 °F)) during the growing season, demonstrating an improved crop yield for crops treated with the agricultural admixture-containing formulations.
FIG. 33 shows the cumulative relative revenue of marketable strawberries picked for cohorts treated with grower's standard compared to blends of grower's standard with agricultural admixtures to crops stressed with high heat (over 32.2 °C (90 °F)) during the growing season, demonstrating an improved crop yield for crops treated with the agricultural admixture-containing formulations.
**FIG. 34** shows a photograph demonstrating, *inter alia*, the consistent sizing differences of lettuce cohorts treated with agricultural admixtures produced by the processes described herein compared to conventional grower's standard fertilizer and compared to conventional fish hydrolysate fertilizer.
**FIG. 35** shows a photograph of consistent chlorophyll quantity and color of lettuce cohorts treated with agricultural admixtures produced by the processes described herein compared to conventional grower's standard fertilizer and compared to conventional fish hydrolysate fertilizer.

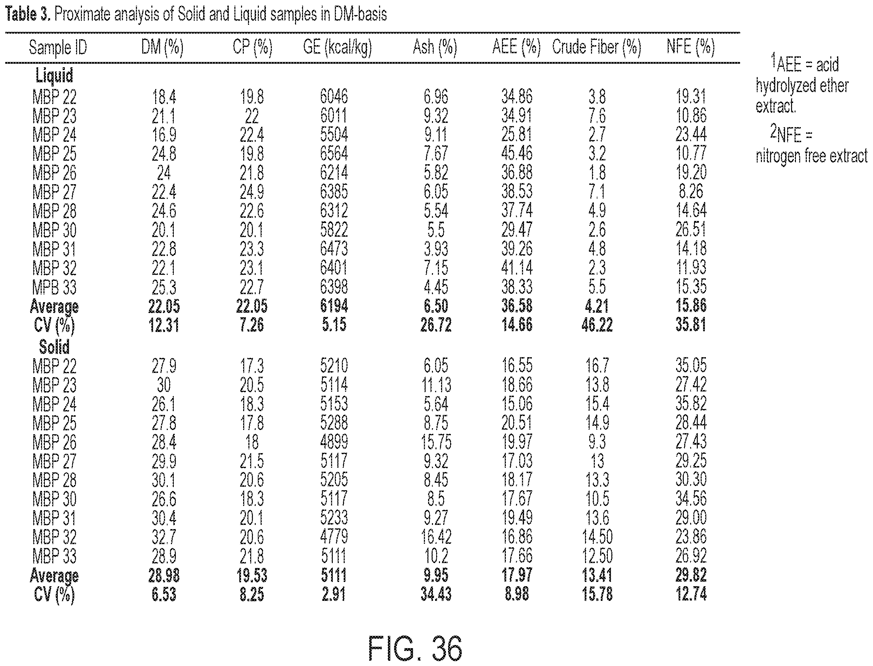

**FIG. 36** shows Table 3.

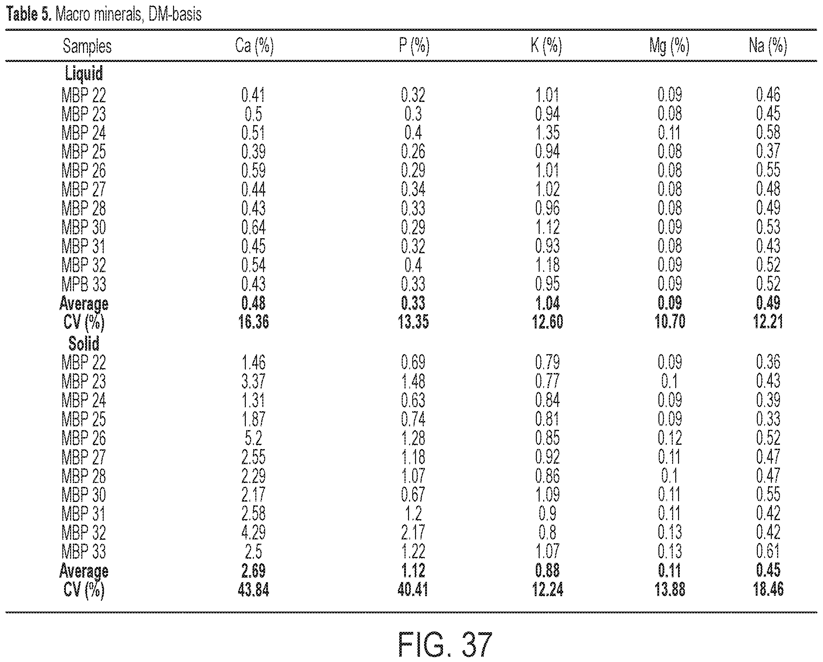

**FIG. 37** shows Table 5

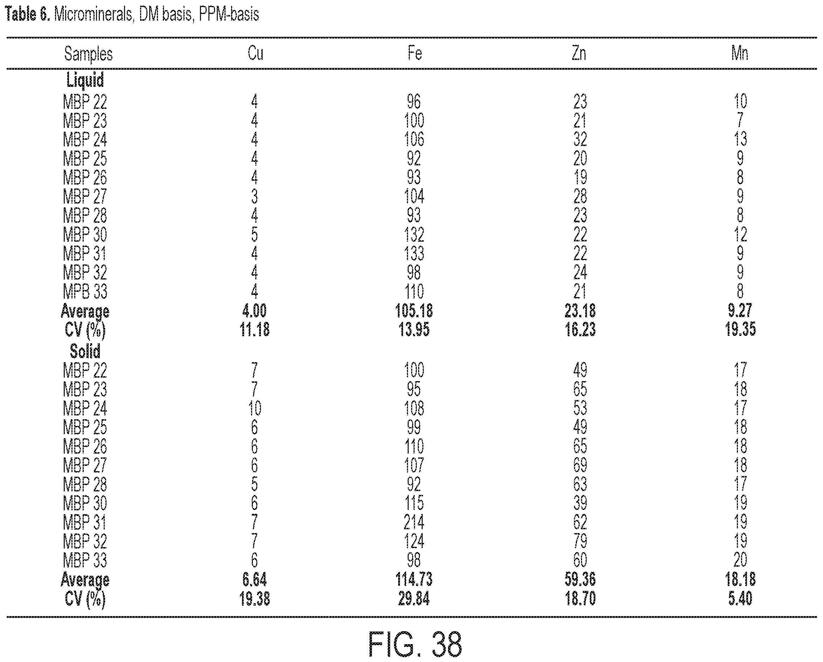

**FIG. 38** shows Table 6.

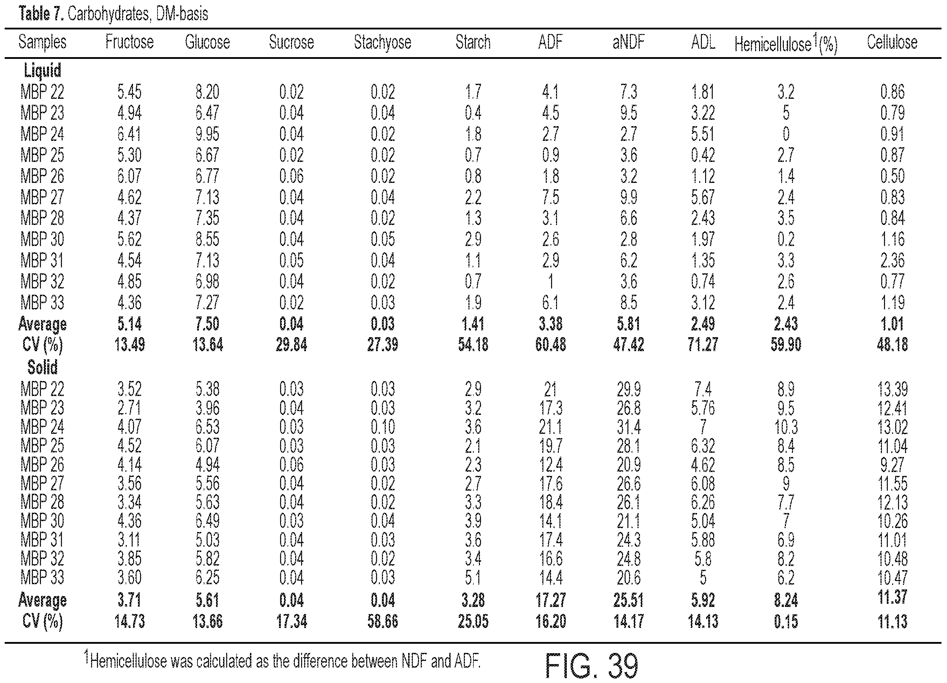

**FIG. 39** shows Table 7.

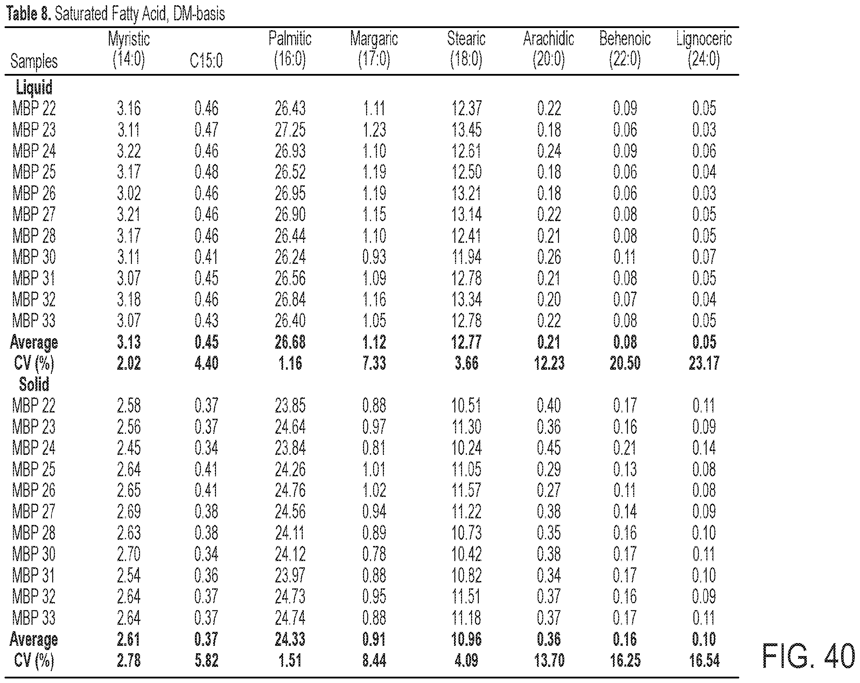

**FIG. 40** shows Table 8.

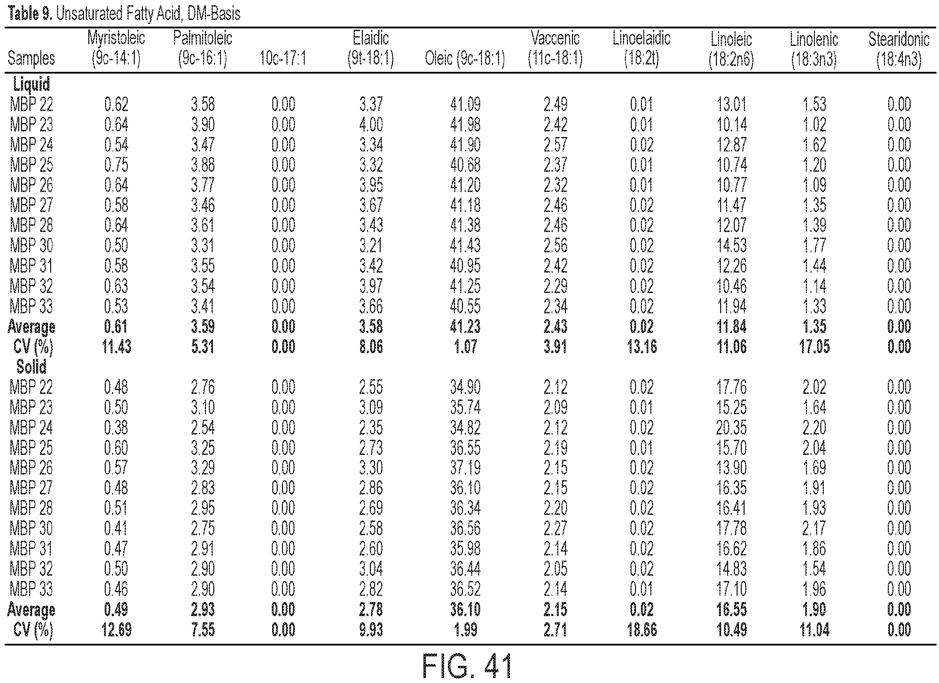

**FIG. 41** shows Table 9.

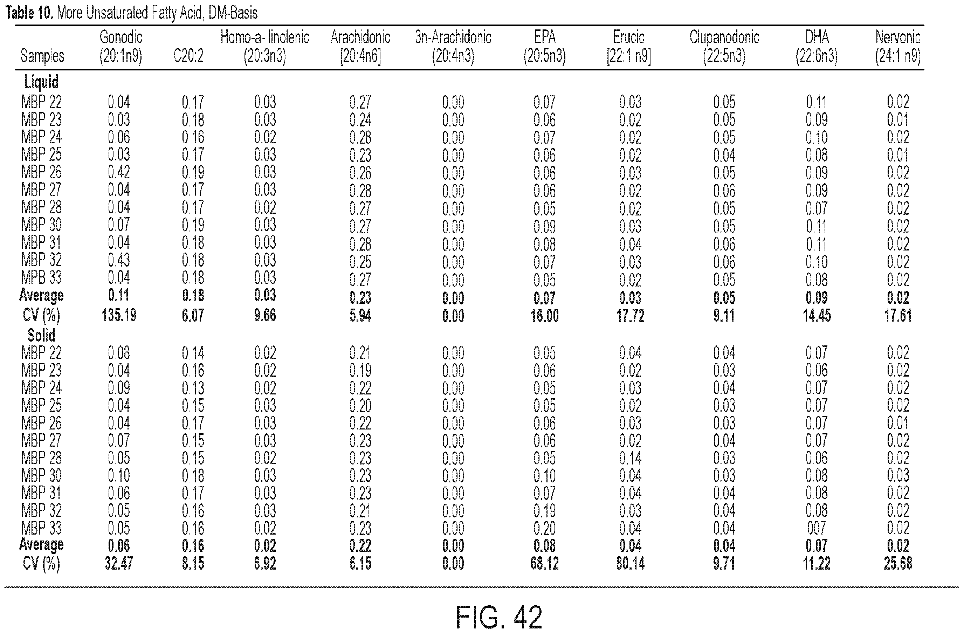

**FIG. 42** shows Table 10.

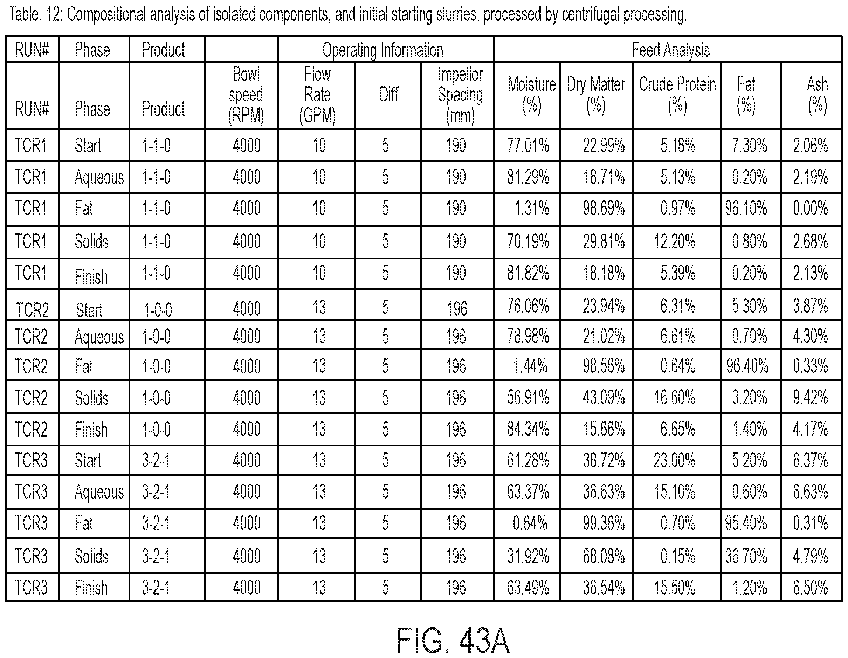

**FIG. 43A** shows Table 12.

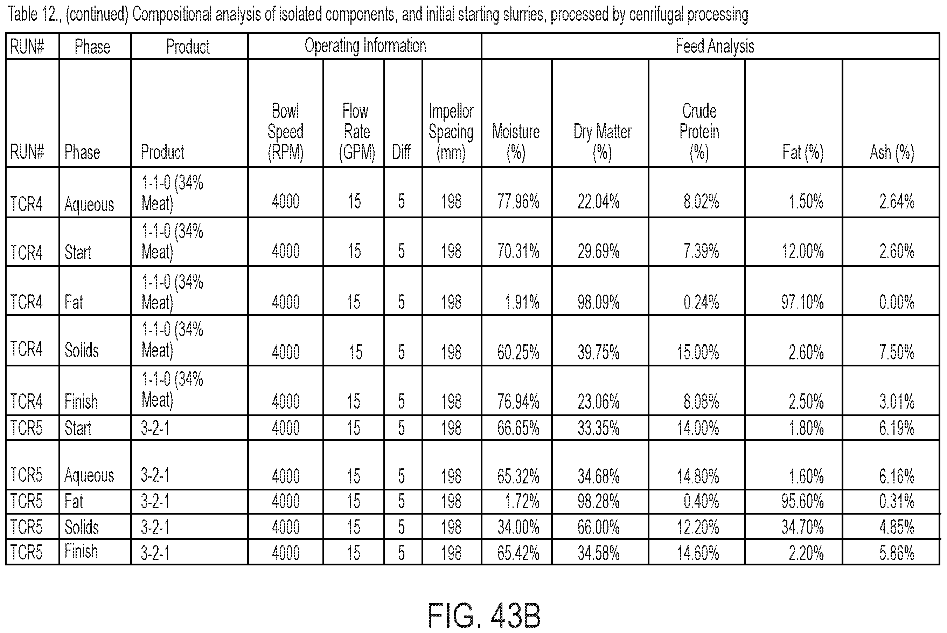

**FIG. 43B** shows Table 12, continued.

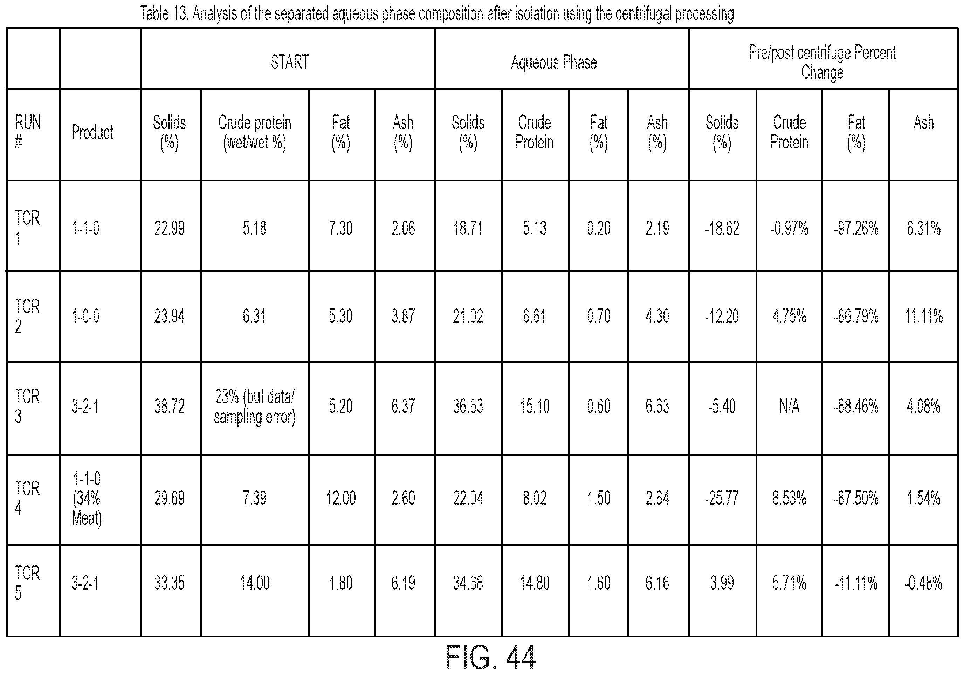

**FIG. 44** shows Table 13.

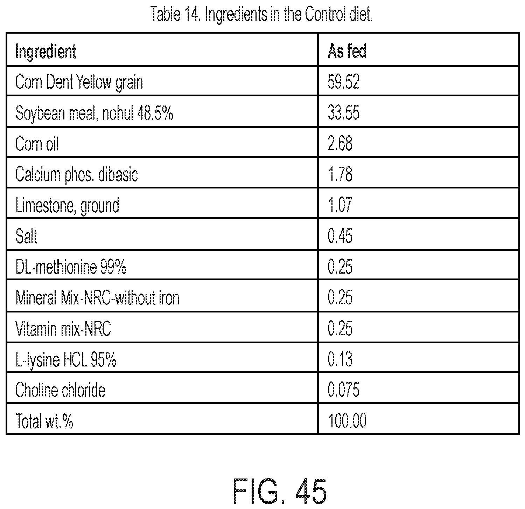

**FIG. 45** shows Table 14.

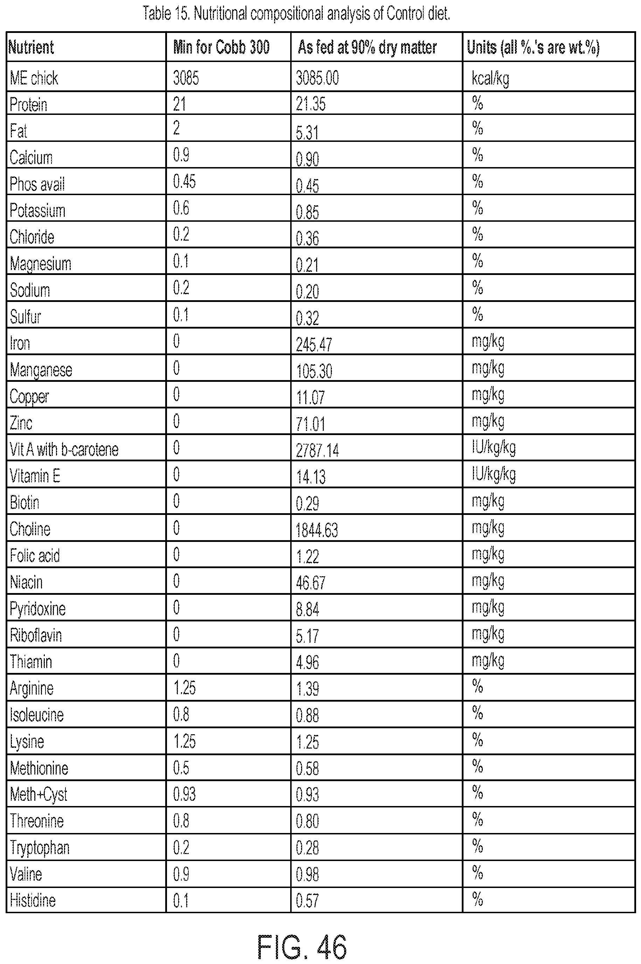

**FIG. 46** shows Table 15.

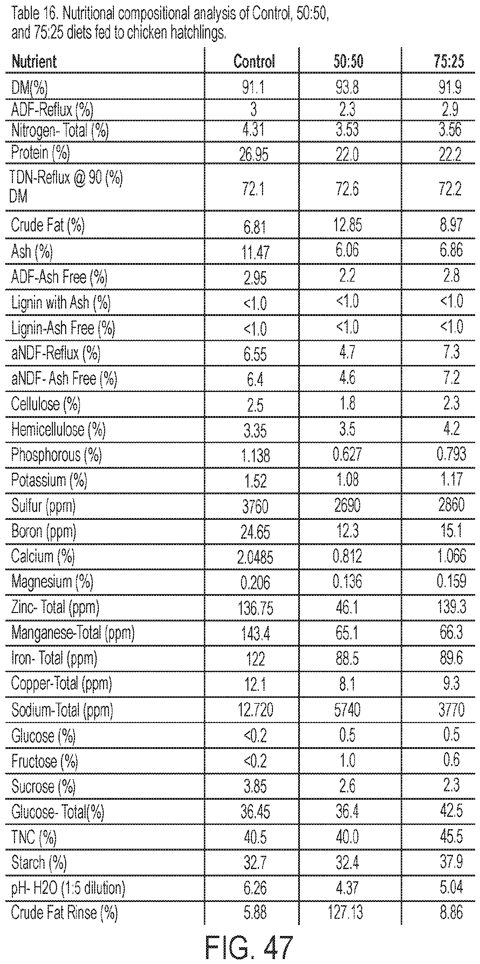

**FIG. 47** shows Table 16.

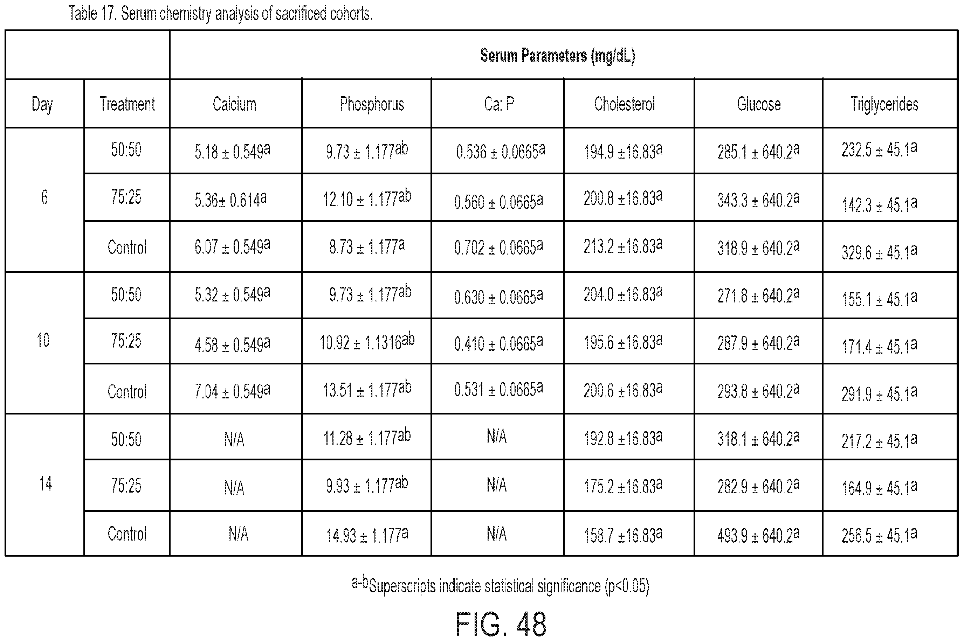

**FIG. 48** shows Table 17.
**FIG. 49** shows Table 4.

### DETAILED DESCRIPTION

### Definitions

As used herein, the term "biological recyclable stream" refers to a recyclable stream selected from: fresh food recyclables, blood meal, bakery goods, spent poultry, pomace, culled fruits and/or vegetables, and mixtures thereof.

As used herein, the term "course screen" refers to a screen or mesh to separate pasteurized solids, which can be used to produce animal provender, from the liquid pasteurized hydrolysate, and can include a variety of screening techniques. In some embodiments the course screen can be a mesh screen with pores having 18-60 mesh (a diameter of about 250 to about 1000 microns). In some embodiments, the course screen can be an 18 mesh screen with 1000 micron openings, 20 mesh screen with 841 micron openings, 25 mesh screen with 707 micron openings, 30 mesh screen with 590-595 micron openings, 35 mesh screen with 500 micron openings, 40 mesh screen with 400 micron openings, 45 mesh screen with 354 micron openings, 50 mesh screen with 297 micron openings, or 60 mesh screen with 250 micron openings, or other commercially available coarse screening technologies. A course screen may have opening so 250 microns or larger, or between any two of the recited sizes. In some aspects, the filter or mesh is made of metal, plastic, glass or ceramic. In some aspects, the plastic can be nylon.

As used herein, the term "fine screen" refers to a screen or mesh with pores having about 35 to 400 mesh (a diameter of about 500 to 27 microns). The fine screen serves to i) increase particle surface area, thereby increasing the effectiveness of the enzymes used to produce the hydrolysate; ii) assure the ability of the pasteurized hydrolysate to pass easily through the farmer's drip lines, or other similar equipment; and iii) ensure the particle sizes are appropriate for metabolism by soil organisms once the agricultural admixture is delivered to the root zone. In some embodiments, the 30 mesh screen is a vibrating screen. This separates the hydrolysate from particles too large to pass through the mesh, for example, particles having an average diameter larger than 590 microns. The hydrolysate passing through the first screen may then be further separated by filtering through a 200 mesh screen with an opening size of 74 microns. In some aspects, the incubated fresh food particles removed from the hydrolysate by screening through the 200 mesh screen have a diameter of greater than microns. In some aspects the screen may be a vibrating screen. In some embodiments the fine screen can be a mesh screen having 35 to 400 mesh may be used in the second screening step, for example, 35 mesh screen with 500 micron openings, 40 mesh screen with 400 micron openings, 45 mesh screen with 354 micron openings, 50 mesh screen with 297 micron openings, or 60 mesh screen with 250 micron openings, 70 mesh screen with 210 micron openings, 80 mesh screen with 177 micron openings, 100 mesh screen with 149 micron openings, 120 mesh screen with 125 micron openings, 140 mesh screen with 105 micron openings, 170 mesh screen with 88 micron openings, 200 mesh screen with 74 micron openings, 230 mesh screen with 63 micron openings, 270 mesh screen with 53 micron openings, 325 mesh screen with 44 micron openings or 400 mesh screen with 37 micron openings, or other commercially available fine screening technologies. The solid particles separated by the fine screen, having a diameter between about 74 microns and about 590 microns, may be recycled as a feedstock to be digested in the next batch. A fine screen may have a mesh size between any two of the recited mesh sizes. In some aspects, the filter or mesh is made of metal, plastic, glass or ceramic. In some aspects, the plastic can be nylon.

As used herein, the term "grower's standard" refers to a nitrate or ammonia based fertilizer and other fertilizing regime with nutrient requirements standardized for a given crop, in current use by the grower.

As used herein, the term "hydrolysate" refers to a product of the digestion of a selected biological recyclable stream with enzymes. The liquid may contain small particles and/or oil droplets depending on the grinders used and the mesh screen used to separate larger particles from the hydrolysate, as described herein.

As used herein, the term "agricultural admixture" refers to the composition comprising nutritional components released from one or more biological recyclable streams by digesting proteins, carbohydrates (such as sugars, starches and/or cellulosic materials), and/or fats and oils in said biological recyclable stream to produce a composition which contains, for example, amino acids, simple sugars, fatty acids and minerals, where the composition produced by the process comprises at least about 90% by weight relative to the weight of the starting material biological recyclable stream.

As used herein, the term "ground biological slurry" refers to the mixture that is formed after the first grinding step, which may be a mixture of particles and liquid.

As used herein, the term "incubated ground biological slurry" refers to the mixture that is incubated at elevated temperature formed after the first grinding step, which may be a mixture of incubated biological particles and an incubated biological hydrolysate.

As used herein, the term "incubated biological particles" refers to the particles obtained from the separated biological slurry which are separated from the incubated biological hydrolysate.

As used herein, the term "incubated biological hydrolysate" refers to the liquid hydrolysate in the ground biological slurry which is separated from the incubated biological particles.

As used herein, the term "enzyme combination" refers to two or more selected enzymes added to ground biological slurry, the processed biological hydrolysate, and/or the incubating mixture. The enzymes in an enzyme combination may be mixed together before addition to the ground biological slurry, the processed biological hydrolysate, and/or the incubating mixture, or they may be added separately to the ground biological slurry, the processed biological hydrolysate, and/or the incubating mixture.

As used herein, the term "high-shear mixer" refers to an apparatus that disperses or transports one phase or ingredient (liquid, solid, or gas) into a main continuous phase (liquid), with which it would normally be immiscible.

As used herein, the term "agitation" means a stirring action intended to increase the collisions between the enzyme molecules and the food particles. In some embodiments, agitation is produced by rotating mixing blades in the incubation vessel, at a rate of 1 to 10⁴ sec⁻¹.

As used herein, the term "shear" means a cutting action that reduces food particle size, increasing its surface area, and therefore, its interaction with enzyme molecules. In some embodiments, high shear is created by circulating the slurry through a high speed, high shear mixer throughout the digest at rates in the range of 10⁵-10⁶ sec⁻¹ or more.

In some embodiments, this disclosure relates to a process described in FIG. 1. A first biological recyclable stream 101 is subject to a grinding and shearing step to form a first biological slurry 102. An optional second, or third, or more biological recyclable stream 103 is subject to a grinding and shearing step to produce a second, or third, or more biological slurry 104. Multiple biological recyclable streams can be processed in parallel or serial and combined with any of the products described herein. The first biological slurry is then incubated with one or more selected enzymes at a temperature from 21.1 to 62.7 °C (70 °F to 145 °F). The incubated slurry is then pasteurized at a temperature greater than 71.1 °C (160 °F) to produce a biological slurry comprising biological hydrolysate and biological particles 105.

A portion or all of the biological slurry comprising biological hydrolysate and biological particles 105 can be subject to an optional drying step to produce dried solid biological slurry 106. In some embodiments, an antioxidant and/or anticaking agents 109 are added to the dried solid biological slurry. The dried solid biological slurry 106 is subjected to a milling or pelletizing step to form a milled or pelletized product 107. The milled or pelletized product 107 is then subject to an optional blending step with a carbohydrate recyclable stream. The milled or pelletized product, with or without blending with the carbohydrate recyclable stream, can be used as animal provender, as Animal Provender (I) 108. In some embodiments, an antioxidant and/or anticaking agents 109 are added to the Animal Provender (I) 108.

A portion or all of the biological slurry comprising biological hydrolysate and biological particles 105 can be separated into a biological hydrolysate 111 and biological particles 110. In some embodiments, the biological particles 110 can be recycled into the biological slurry comprising biological hydrolysate and biological particles 105 or with the first biological slurry 102 to be incubated and pasteurized again. In some embodiments, the biological particles 110 can be dewatered via a separation step to produce dewatered biological particles 112 and a recycled fraction which can be added to the biological hydrolysate 111. In some embodiments, the dewatered biological particles 112 can be used as compost, biofuel source, or as Animal Provender (IV) 113. In some embodiments, an antioxidant and/or anticaking agents 109 are added to the Animal Provender (IV) 113. In some embodiments, the biological hydrolysate 111 can be mixed with the slurry which is subject to the drying step to supplement the dried biological slurry 106 wherein the dried biological slurry 106 would have a lower relative particles content from the dilution of the added biological hydrolysate 111.

In some embodiments, the biological hydrolysate 111 is subject to a centrifugation step to reduce the fats content (oils) in the produced centrifuged biological hydrolysate 114, and to form separated centrifuged oil 115. The centrifuged oil 115 can be further separated into a food unusable oil stream 122 and a food useable oil stream 123. The food unusable oil stream 122 can be used as a biofuel source 124. In some embodiments, the food useable oil stream 123 can be used as animal provender as Animal Provender (III). In some embodiments, an antioxidant is added to the Animal Provender (III). In some embodiments, the centrifuged biological hydrolysate can be added to the 111 can be mixed with the slurry which is subject to the drying step to supplement the dried biological slurry 106 wherein the dried biological slurry 106 would have a lower relative fats content from the dilution of the added (reduced fat) centrifuged biological hydrolysate 114. In some embodiments, the centrifuged oil 115 can be mixed with the slurry which is subject to the drying step to supplement the dried biological slurry 106 wherein the dried biological slurry 106 would have a higher relative fats content due to the addition of the centrifuged oil 115.

In some embodiments, an antioxidant and/or anticaking agents 109 can be added to any of the dried, solid biological slurry, milled or pelletized product, dewatered biological particles, to any animal provender (I)-(VI), or any dried form of animal provender of this disclosure.

The centrifuged biological hydrolysate 114 can be subject to a stabilization step by adding a stabilizer to produce a stabilized aqueous hydrolysate 116. The stabilized aqueous hydrolysate 116 can be emulsified to produce an emulsified agricultural admixture 117. In some embodiments, the emulsified agricultural admixture 117 can be blended with an additive. The blended additive can include or exclude a dispersant or a mineral. The mineral can be mined basalt. The blended emulsified agricultural admixture can be used as fertilizer 118.

In some embodiments, the emulsified agricultural 117 can be concentrated to form a concentrated first agricultural admixture 119. The concentrated first agricultural admixture 119 can be used as fertilizer or animal provender 120 as Animal Provender (II).

In some embodiments, an optional additional biological recyclable stream 121 can be added to the stabilized aqueous hydrolysate 116. The optional additional biological recyclable stream 121 can be a carbohydrate recyclable stream. In some embodiments, the carbohydrate recyclable stream can be dried breadcrumbs.

In some embodiments, an optional additional biological recyclable stream 121 can be mixed with the slurry which is subject to the drying step to supplement the dried biological slurry 106 wherein the dried biological slurry 106 would have a higher content of the components of the additional biological recyclable stream 121. In some embodiments, when the additional biological recyclable stream 121 added to slurry which is subject to the drying step is a carbohydrate recyclable stream, the carbohydrate content of the dried biological slurry 106 is increased.

The inventors have discovered that by the selective addition of centrifuged oil 115, optional additional biological recyclable stream 121, biological hydrolysate 111, and/or centrifuged (reduced fat) biological hydrolysate 114 to the process steps preceding the formation of any of the animal provender forms described herein, the amino acids, solids, carbohydrates, and proteins content can be selectively obtained to produce an ideal animal provender with properties which standard animal provender forms cannot exhibit.

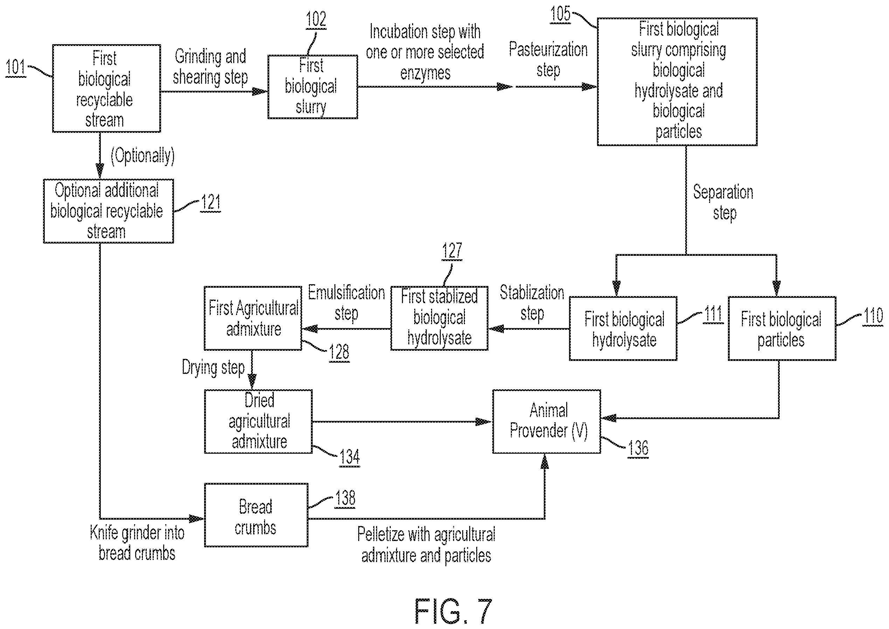

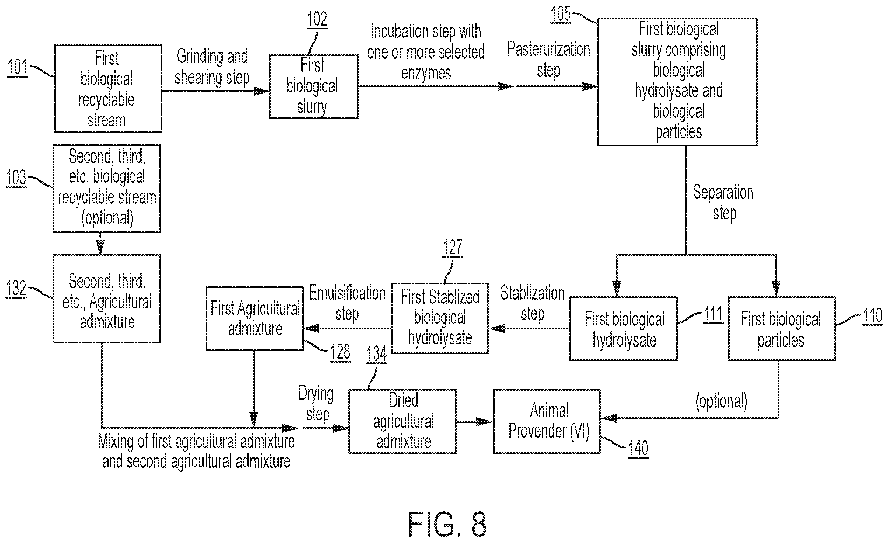

As indicated in FIG. 7, the first biological particles 110 and the dried agricultural admixture 134 can be combined, optionally with bread crumbs, to produce animal provender as Animal Provender (V). As indicated in FIG. 8, the first biological particles 110 and the dried agricultural admixture 134 formed from combining a first agricultural admixture 128 and an optional second, third, or more agricultural admixture 132, can be combined to form animal provender as Animal Provender (VI).

In some embodiments, any of the animal provenders described herein can be mixed, blended, diluted, dissolved, ground, or pulverized with any other animal provender described herein. In some embodiments, the antioxidant and/or anticaking agents can be added to any of the animal provenders described herein.

### EXAMPLES

### Example 1. Procedure to make agricultural admixture for use as a fertilizer, plant growth enhancer, or soil amendment

The following experiment demonstrates that agricultural admixtures can be processed for use as a fertilizer, plant growth enhancer, or soil amendment.

Recycled fresh food recyclables were collected from supermarkets. The fresh food recyclables from was sourced from the produce, meat, fish, bakery, & deli departments of the supermarkets, and was collected by refrigerated trucks within 2 days of being pulled off of the shelf at the supermarket. The bakery fresh food recyclable stream was isolated from the other fresh food recyclable streams and not included in the fresh food recyclable streams used to make the agricultural admixture for use as fertilizer, plant growth enhancer, or soil amendment. The collected fresh food recyclables was kept fresh by storage in specialized, insulated containers that are designed to keep the collected food fresh while awaiting pickup. Collected supermarket fresh food was processed within 24 hrs. of arrival at the production facility.

The collected fresh food recyclables was weighed and recorded separately as pounds of meat or produce. After the material was weighed, it was emptied into a central hopper and ground into a fresh food recyclables particle slurry using a Rotary Knife Grinder with a pump head.

The grinder pumped the fresh food recyclables particle slurry into a jacketed digestion vessel, where it was continuously mixed. The enzymatic digestion incubation process was carried out in this vessel for a total of 3 hours. Enzymes were introduced into the slurry, and the material was continuously heated, mixed, and further ground to maximize the efficiency of the enzymes acting on the material.

More specifically, a first enzyme combination comprising endocellulase, exocellulase and lipase was added to the fresh food recyclables slurry with constant mixing, and the temperature was increased to 37.8 °C (100 °F), for 30 minutes. An in-line high shear grinder in a recirculating line was then turned on. The high shear grinder was a high shear mixer with a disintegrating head (high RPM shearing action). A second enzyme combination comprising pectinase, protease, and α-amylase was then added, with the protease added last, and the temperature increased to 54.4 °C (130 °F) for 1.5 hours. After incubating, the incubated hydrolysate was heated to between 71.1-76.7 °C (160-170 °F) for about 30 minutes to pasteurize the hydrolysate.

The pasteurized material was then separated using mesh screens. The hydrolysate produced by incubating was first separated using a vibrating 30 mesh screen with an opening of 590 µm. The hydrolysate passing through the first screen was further separated by filtering through a 200 mesh screen with an opening size of 74 µm.

The separated liquid hydrolysate was then introduced into a tricanter centrifuge and separated into particles, fats, and an aqueous phase. The isolated aqueous phase (comprising from about 0.1 to 2.0 weight percent fats) was then emulsified/homogenized using an ultra-high shear grinder which may be a high shear multi stage mixer, to form an emulsified hydrolysate. The emulsified hydrolysate was pumped to the stabilization tank for final processing. The isolated fats were pumped into a separate storage tank for further fat processing. The isolated particles were dried at room temperature. The isolated particles were optionally pelletized for use as a separate soil amendment product.

The pasteurized aqueous hydrolysate or emulsified hydrolysate was stabilized by adding phosphoric acid to a pH of 2.8, and 0.25% potassium sorbate was then added to preserve the liquid in its pasteurized state and prevent microbial activity while in storage. This material was then sampled and checked for pH and for the presence food pathogens. Food pathogen screening required a 24 hr. incubation period, so the material was held in the stabilization tank for 24 hrs. until it cleared this check. The emulsified hydrolysate was then transferred to a storage tank.

After stabilization, the hydrolysate was also laboratory tested, to ensure that the contents are free of pathogens (including *E. coli* and *salmonella*), heavy metals and other unsuitable materials for use as a fertilizer, plant growth enhancer, or soil amendment. Individual batches were blended, to assure that the aqueous emulsified hydrolysate composition was consistent.

### Example 2. Procedure to make agricultural admixture for use as animal provender

The following experiment demonstrates that agricultural admixtures can be processed for use as animal provender.

Recycled fresh food recyclables was collected from supermarkets. The fresh food recyclables from was sourced from the produce, meat, fish, bakery, & deli departments of the supermarkets, and was collected by refrigerated trucks within 2 days of being pulled off of the shelf at the supermarket. The bakery fresh food recyclable stream was isolated from the other fresh food recyclable streams and not included in the fresh food recyclable streams used to make the agricultural admixture for use as fertilizer, plant growth enhancer, or soil amendment. The collected fresh food recyclables was kept fresh by storage in specialized, insulated containers that are designed to keep the collected food fresh while awaiting pickup. Collected supermarket fresh food was processed within 24 hrs. of arrival at the production facility.

The collected fresh food recyclables was weighed and recorded separately as pounds of meat or produce. After the material was weighed, it was emptied into a central hopper and ground into a fresh food recyclables particle slurry using a Rotary Knife Grinder with a pump head. The isolated bread fresh food recyclable stream was separately processed using a separate rotary knife grinder into bread crumbs.

The grinder pumped the fresh food recyclables particle slurry into a jacketed digestion vessel, where it was continuously mixed. The enzymatic digestion incubation process was carried out in this vessel for a total of 3 hours. Enzymes were introduced into the slurry, and the material was continuously heated, and subject to constant agitation and shear, to maximize the efficiency of the enzymes acting on the material.

More specifically, a first enzyme combination comprising endocellulase, exocellulase and lipase was added to the fresh food recyclables slurry with constant mixing, and the temperature was increased to 37.8 °C (100 °F), for 30 minutes. An in-line high shear grinder in a recirculating line was then turned on. The high shear grinder was a high shear mixer with a disintegrating head (high RPM shearing action). A second enzyme combination comprising pectinase, protease, and α-amylase was then added, with the protease added last, and the temperature increased to 54.4 °C (130 °F) for 1.5 hours. In some embodiments, the enzymes can be added simultaneously. After incubating, the incubated hydrolysate was heated to between 71.1-76.7 °C (160-170 °F) for about 30 minutes to pasteurize the hydrolysate.

The pasteurized slurry material was then directly used as animal provender after confirmation the slurry was free of pathogens, in the case of the pig trials. In the chicken trials and in the current configuration of the invention, the slurry is moved to a heated process tank, then fed into a drum dryer, milled into a powder, and, as needed: stabilized; anti-caking agent added, and pelletized.

In one optional embodiment, the pasteurized slurry material was dewatered at room temperature to form a dried provender form. In one optional embodiment, the pasteurized slurry material was mixed with the isolated bread crumbs processed by the methods described above and pelletized into a solid provender form.

In one optional embodiment, the pasteurized slurry material was then separated using mesh screens. The hydrolysate produced by incubating was first separated using a vibrating 30 mesh screen with an opening of 590 µm. The hydrolysate passing through the first screen was further separated by filtering through a 200 mesh screen with an opening size of 74 µm.

The separated liquid hydrolysate was then introduced into a tricanter centrifuge and separated into particles, fats, and an aqueous phase. The isolated aqueous phase (comprising from about 0.1 to 2.0 weight percent fats) was then emulsified/homogenized using an ultra-high shear grinder which may be a high shear multi stage mixer, to form an emulsified hydrolysate. The emulsified hydrolysate was pumped to the stabilization tank for final processing. The isolated fats were pumped into a separate storage tank for further fat processing. The isolated particles were dried at room temperature. The isolated particles were separated.

The pasteurized aqueous hydrolysate or emulsified hydrolysate was stabilized by adding 0.25% potassium sorbate to preserve the liquid in its pasteurized state and prevent microbial activity while in storage. This material was then sampled and checked for pH and for the presence food pathogens. Food pathogen screening required a 24 hr. incubation period, so the material was held in the stabilization tank for 24 hrs. until it cleared this check. The emulsified hydrolysate was then transferred to a storage tank.

After stabilization, the pasteurized aqueous hydrolysate was also laboratory tested, to ensure that the contents are free of pathogens (including *E. coli* and *salmonella*), heavy metals and other unsuitable materials for use as a fertilizer, plant growth enhancer, or soil amendment. Individual batches were blended, to assure that the aqueous emulsified hydrolysate composition was consistent.

The pasteurized aqueous hydrolysate was then dewatered to produce a dried form of animal provender.

In some optional embodiments, the bakery recyclable stream was not processed by the enzymatic digestion methods described herein and instead dried and ground into breadcrumbs. In some optional embodiments, the breadcrumbs were combined by mixing, grinding, or diluting the breadcrumbs with the dry or liquid forms of the hydrolysates described herein to produce an agricultural admixture for use as animal provender.

### Example 3. Protection against Crop Stress

The following experiment demonstrates that agricultural admixtures produced by the methods described herein can be used to enable crop irrigation under high stress crop conditions. The high stress crop conditions can include or exclude: high salinity water, high salinity soils, low soil nutrient content, low soil microbe volume, and high heat.

### High-Salinity Water Stress

A strawberry crop in Ventura County, California (United States) was divided into four separate sections, and each section was subject to separate irrigation and fertilization conditions. A first section was fertilized with Grower's Standard, a standard nitrate fertilizer to serve as a control, the composition of which is described in Table 1 below. This section was irrigated with non-saline water. A second section was fertilized with Grower's Standard and irrigated with 200 ppm NaCl. A third section was fertilized with the same amount of Grower's Standard and "H2H", an agricultural admixture of this disclosure produced from fresh food recyclables by the methods described herein. This third section was irrigated with 200 ppm NaCl. This third section was presented with an aqueous solution of the H2H at an amount of 46.77 l/ha (5 gallons per acre). A fourth section was fertilized with Grower's Standard and "H2H", and was irrigated with 200 ppm NaCl. This fourth section was presented with an aqueous solution of the H2H at an amount of 93.54 l/ha (10 gallons per acre).

**Table 1. Grower's Standard Composition**

| **Analysis** | **Description** |
|---|---|
| 20-00-11 | A/N Mopsol A/S Sol. |
| 46-00-00 | Urea |
| 00-00-62 | Muriate of Potash, Granular |
| 00-00-50 | Sulphate of Potash |
| 20% (wt.) | Iron Sulphate |
| 9.8% (wt.) | Magnesium Sulphate (Epsom Salt) |
| 32% (wt.) | Manganese Sulphate |
| 00-00-60 | Muriate of Potash, Soluable |

The results are depicted in Table 2, below:

**Table 2. Summary of results of high salinity irrigation using the agricultural admixtures of this disclosure.**

| | **Trial: Measure H2H buffering effect on 200ppm NaCl in irrigation water on strawberries** | |
|---|---|---|
| **#** | **Protocol** | **Results** |
| 1 | Grower's Standard - No salt | Control |
| 2 | GS + 200ppm NaCl | Salt cuts yield by 50% |
| 3 | GS w/NaCl + H2H (5 g/a) | Comparable to GS/No Salt |
| 4 | GS w/NaCl + H2H (10 g/a) | Highest Production Overall within the experiment |

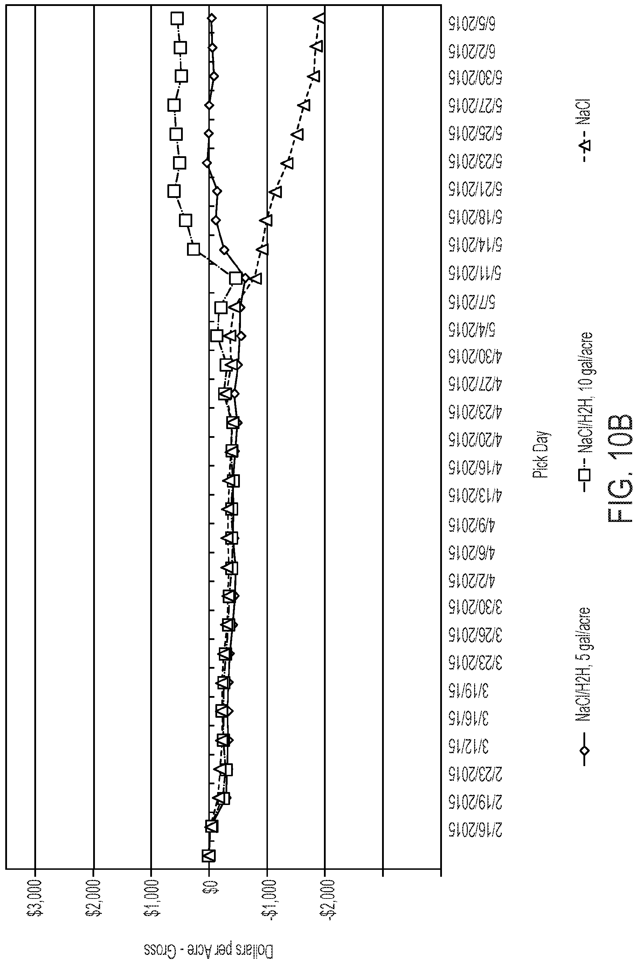

The results shown in FIG. 10 indicate that irrigating with high salinity water cuts the crop yield by 50% when the crop is only fertilized with the control fertilizer. The treatment with grower's standard with no salt is depicted by the (pink) square box line. The treatment with the grower's standard + 200 ppm NaCl is depicted by the (purple) x-x-x line. However, irrigating with 200 ppm NaCl to crops fertilized with the control and H2H at an amount of 5 gallons per acre, the crop yield is equivalent to the control crop treated with normal water (no saline added) (see (blue) line with diamonds), e.g., a high crop yield compared to that obtained using grower's standard fertilizer (200% of the yield obtained with standard fertilizer). Surprisingly, the highest crop yield was observed for the fourth section which was irrigated with 200 ppm NaCl and fertilized with the control fertilizer and H2H at an amount of 10 gallons per acre. (See top (green) line with triangles). The results demonstrate both (1) that the agricultural admixture of this disclosure can be applied to a crop irrigated with high salinity water to achieve a crop yield equivalent to a crop treated with normal water and a control fertilizer, and (2) the dose-response of the crop yield as a function of the agricultural admixture amount clearly demonstrates the effect of the agricultural admixture on crop yield under standard and high salinity conditions. The results herein demonstrate that the agricultural admixtures can be used to increase crop yields for crops under high stress conditions, where the high stress can include or exclude high salinity water or high salinity soil.

### High Temperature Stress

Strawberry cohorts were administered with grower's standard, grower's standard (at half the application rate of the control) with H2H (administered at a rate of 5 gal/acre per treatment day) ("H2H-low"), grower's standard (at half the application rate as control) with H2H (administered at a rate of 7.5 gal/acre per treatment day) ("H2H-mid"), or grower's standard (at half the application rate as control) with H2H (administered at a rate of 10 gal/acre per treatment day) ("H2H-high") to growing crops exposed to high temperature of over 32.2 °C (90 °F) during the growing season.

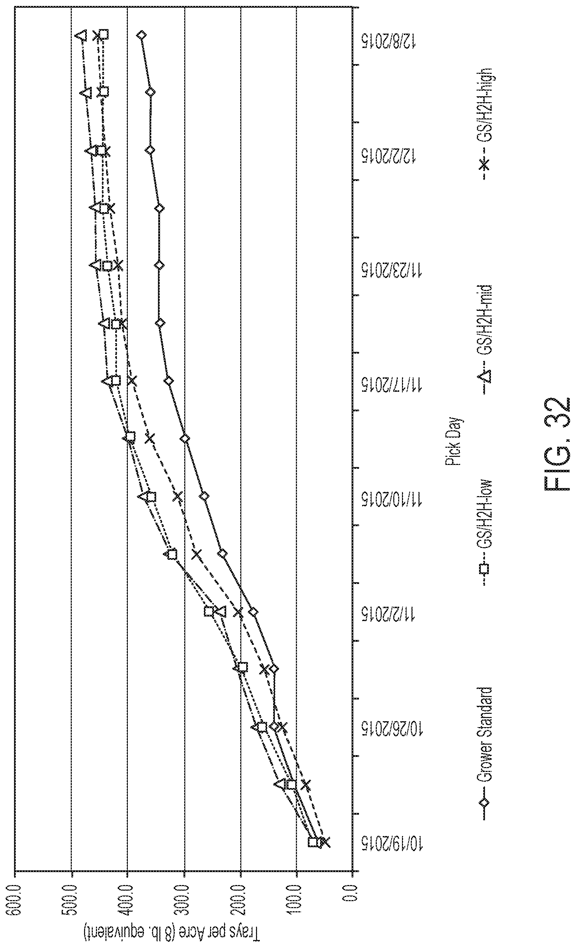

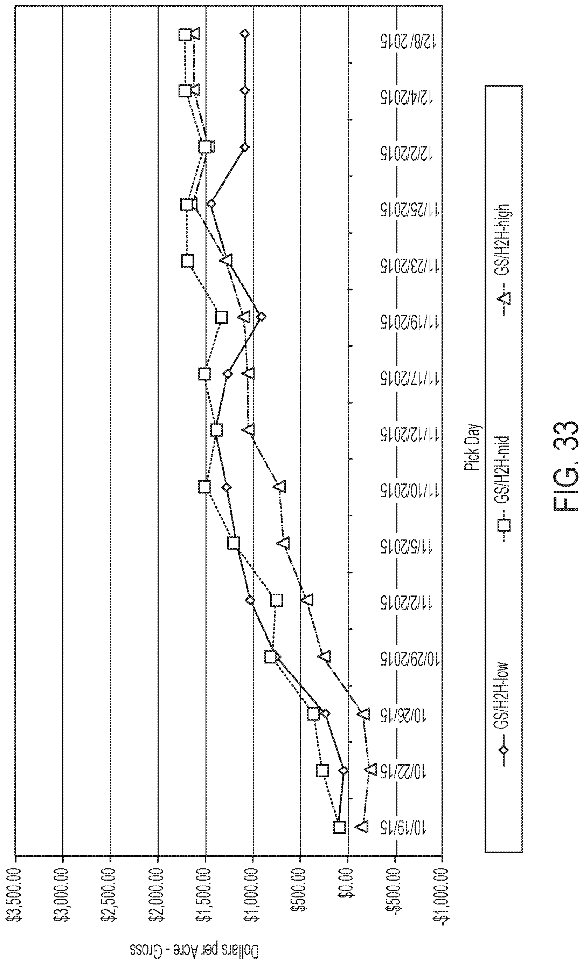

As shown in FIG. 32, cohorts treated with H2H and grower's standard consistently yielded a higher cumulative fruit pick per day than cohorts treated with grower's standard alone. As show in FIG. 33, cohorts treated with H2H and grower's standard consistently yielded a higher cumulative per-acre crop revenue difference compared to cohorts treated with grower's standard alone. The results demonstrate that the agricultural admixtures described herein can increase crop yield when applied to crops under high-stress conditions.

### Example 4. Analysis and proof of batch to batch consistency of agricultural admixtures

To demonstrate the batch to batch consistency of the agricultural admixture made by the processes described herein, eleven separate batches were analyzed for their composition. Table 3 lists the proximate analysis of the solid and liquid samples in dry-matter (DM) basis. The dry matter percentage (DM%), crude protein percentage (CP%), gross energy (GE), ash weight after ashing (ash%), acid hydrolyzed ether extract composition (AEE%) which is equivalent to the fats content, crude fiber perentage, and nitrogen free extract (NFE) which is equivalent to the carbohydrates content, were all measured on a percentage by weight (wt.%), for both the liquid hydrolysate and the separated solids. The compositional analysis was assessed by known methods: DM - Method 930.15; AOAC International, 2007, ash - Method 942.05; AOAC International, 2007, crude fat -Method 954.02; AOAC International, 2007, crude protein **(CP)** by combustion - (Method 990.03; AOAC International, 2007) on an Elementar Rapid N-cube protein/nitrogen apparatus (Elementar Americas Inc., Mt. Laurel, NJ), amino acids - Method 982.30 E (A, B, and C); AOAC International, 2007, crude fiber - Method 978.10; AOAC International, 2007, acid detergent fiber (ADF) and acid detergent lignin - Method 973.18; AOAC International, 2007, neutral detergent fiber (NDF) (Holst, D.O., 1973. Holst filtration apparatus for Van Soest detergent fiber analysis, J. AOAC. 56, 1352-1356), sugar profile (fructose, glucose, sucrose, lactose, maltose) - by the methods described in Churmas, S.C., 1982. Carbohydrates, in: Zweig, G., Sherma, J. ed., Handbook of Chromatography, CRC Press, Boca Raton, FL, pp. 209-254; and Kakeki, K., Honda, S., 1989. Silyl ethers of carbohydrates, in: Biermann, C.J., McGinnis, G.D. ed., Analysis of Carbohydrates by GLC and MS, CRC Press, Boca Raton, FL, pp. 43-85, oligosaccharides (stachyose, verbascose; Churmas, 1982, *supra*), minerals (Cu, Fe, Zn, Mn, Ca, P, K, Mg, Na, S, Cl) - by Inductive Coupled Plasma-Optical Emission Spectoscopy [ICP-OES; Method 985.01 (A, B, and C); AOAC International, 2007]. All samples were also analyzed for fatty acid profiles by gas-liquid chromatography according to Methods 965.49 and 996.06 (AOAC International, 2007). The concentration of nitrogen free extract (NFE) was calculated as the difference between DM and the summation of AEE, ash, CF, and CP. Gross energy (GE) was calculated using the equation: GE = 17.6 + 0.0617*CP + 0.2193*EE +0.0387*CF - 0.1867*Ash (Sauvant, D., Perez, J. M., Tran, G., 2002. Tables of composition and nutritional value of primary materials destined for stock animals: pigs, poultry, cattle, sheep, goats, rabbits, horses, fish. INRA Editions). The concentration of hemicellulose was calculated as the difference between NDF and ADF.

The intra-batch CV (coefficient of variance) was found to be less than 36% for all of the parameters, with the exception of crude fiber percentage which has a high CV from the low values. All percentages listed herein for the compositional analysis are by weight percent. For the liquid samples, the DM% ranged from 16.9 to 25.3%, the CP ranged from 19.18 to 25.3%, the GE ranged from 5504 to 6564 kcal.kg, the Ash amount ranged from 3.93 to 9.32%, the AEE ranged from 25.81 to 41.14%, the crude fiber ranged from 1.8 to 7.6%, and the NFE ranged from 8.26 to 26.51%. For the solid samples, the DM% ranged from 26.1 to 32.7%, the CP ranged from 17.3 to 21.8%, the GE ranged from 4779 to 5288 kcal.kg, the Ash amount ranged from 6.05 to 16.42%, the AEE ranged from 15.06 to 20.51%, the crude fiber ranged from 9.3 to 16.7%, and the NFE ranged from 23.86 to 35.82%.

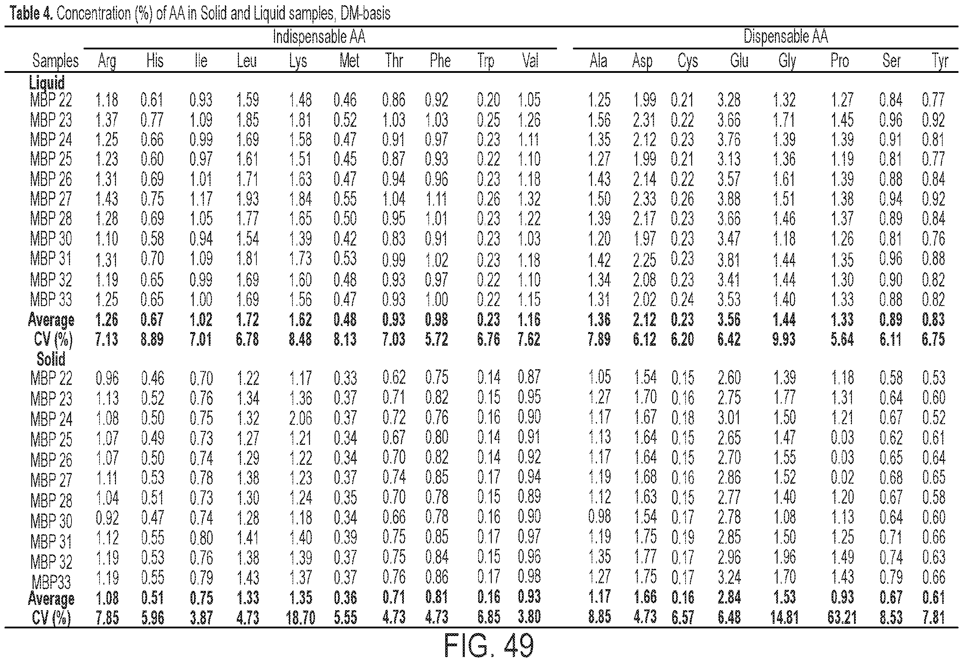

As shown in FIG. 49, Table 4 lists the weight concentration of amino acids in the solid (separated particles), and liquid (agricultural admixture) samples made by the processes described herein. The intra-batch CV was no greater than 8.89%, indicating very consistent batch to batch amino acid content. For the liquid samples, the wt% of arginine ranged from 1.1 to 1.43%, of histidine ranged from 0.58 to 0.77 %, of isoleucine ranged from 0.93 to 1.17%, of leucine ranged from 1.54 to 1.93%, of lysine ranged from 1.39 to 1.84%, of methionine ranged from 0.42 to 0.55%, of threonine ranged from 0.83 to 1.04%, of phenylalanine ranged from 0.92 to 1.11%, of tryptophan ranged from 0.92 to 1.11%, of valine ranged from 1.03 to 1.32%, of alanine ranged from 1.20 to 1.56%, of asparagine ranged from 1.97 to 2.33%, of cysteine ranged from 0.21 to 0.26%, of glutamic acid ranged from 3.13 to 3.88%, of glycine ranged from 1.18 to 1.71%, of proline ranged from 1.19 to 1.45%, of serine ranged from 0.81 to 0.96%, and of tyrosine ranged from 0.76 to 0.92%. For the solid samples, the wt% of arginine ranged from 0.92 to 1.19%, of histidine ranged from 0.46 to 0.55 %, of isoleucine ranged from 0.73 to 0.8%, of leucine ranged from 1.22 to 1.43%, of lysine ranged from 1.17 to 2.06%, of methionine ranged from 0.33 to 0.39%, of threonine ranged from 0.62 to 0.76%, of phenylalanine ranged from 0.75 to 0.86%, of tryptophan ranged from 0.14 to 0.17%, of valine ranged from 0.87 to 0.98%, of alanine ranged from 0.98 to 1.35%, of asparagine ranged from 1.54 to 1.77%, of cysteine ranged from 0.15 to 0.19%, of glutamic acid ranged from 2.60 to 3.24%, of glycine ranged from 1.08 to 1.96%, of proline ranged from 1.03 to 1.49%, of serine ranged from 0.58 to 0.79%, and of tyrosine ranged from 0.52 to 0.66%.

The compositional analysis of the liquid and solid compositions indicates that each comprises nutrients which can be used to promote biological growth, including nematode growth for crop yield enhancement, and animal provender.

Tables 5 and 6 list the mineral content of the agricultural admixtures made by the processes described herein. The intra-batch CV was found to be less than 16.4%, indicating very consistent batch to batch mineral content. For the liquid samples, the wt.% of calcium ranged from 0.39 to 0.64%, of phosphorous ranged from 0.26 to 0.4%, of potassium ranged from 0.93 to 1.35%, of magnesium ranged from 0.08 to 0.11%, and of sodium ranged from 0.37 to 0.58%. For the liquid samples, the concentrations (in ppm, parts-per-million) of copper ranged from 3 to 5 ppm, of iron ranged from 92 to 133 ppm, of zinc ranged from 19 to 32 ppm, and of manganese ranged from 7 to 13 ppm. For the solid samples, the wt.% of calcium ranged from 1.31 to 5.2%, of phosphorous ranged from 0.63 to 2.17%, of potassium ranged from 0.77 to 1.09%, of magnesium ranged from 0.09 to 0.13%, and of sodium ranged from 0.33 to 0.61%. For the solid samples, the concentrations (in ppm, parts-per-million) of copper ranged from 5 to 10 ppm, of iron ranged from 92 to 214 ppm, of zinc ranged from 49 to 79 ppm, and of manganese ranged from 17 to 20 ppm.

Table 7 lists the carbohydrates content (wt.%) of the agricultural admixtures made by the processes described herein. Each weight percent listed of carbohydrates is the weight percent of dry matter of the NFE component of the admixture. With the exception of the starch content, the intra-batch CV was found to be less than 30%, indicating very consistent batch to batch mineral content. For the liquid samples, the acid detergent fiber (ADF), which comprises cellulose, lignin, and other insoluble fibers but not hemicellulose, ranged between 0.9 and 6.1%, with an intra-batch CV of 60.48%; the ash-free neutral detergent fiber (aNDF) content ranged between 2.7 and 8.5%, with an intra-batch CV of 47.42%; the acid detergent lignin (ADL) ranged between 0.42 and 5.51% with a CV of 71.27%; the hemicellulose content ranged between 0 and 5%, the cellulose content ranged between 0.77 and 2.36%; the fructose content ranged between 4.36 and 6.41%; the glucose content ranged between 6.47 and 9.95%; the sucrose content ranged between 0.02 and 0.06 %; the stachyose content ranged between 0.02 and 0.05%; and the starch content ranged between 0.4 to 7.5%. For the solid samples, the acid detergent fiber (ADF), which comprises cellulose, lignin, and other insoluble fibers but not hemicellulose, ranged between 12.7 and 21.1%, with an intra-batch CV of 16.2%; the ash-free neutral detergent fiber (aNDF) content ranged between 20.6 and 31.4%, with an intra-batch CV of 14.17%; the acid detergent lignin (ADL) ranged between 4.62 and 7.4% with a CV of 14.13%; the hemicellulose content ranged between 6.2 and 10.3%, the cellulose content ranged between 9.27 and 13.39%; the fructose content ranged between 2.71 and 4.52%; the glucose content ranged between 3.96 and 6.49%; the sucrose content ranged between 0.03 and 0.06 %; the stachyose content ranged between 0.02 and 0.1%; and the starch content ranged between 2.1 to 5.1%.

Tables 8, 9, and 10 list the saturated fatty acids content on a weight percentage (wt.%) basis of the total fats contents (AEE%) on a dry-matter basis of the agricultural admixtures made by the processes described herein. With the exception of gonodic acid, the intra-batch CV was found to be less than 23.17 %, indicating very consistent batch to batch saturated fatty acids content. For the liquid samples, the wt.% of myristic (14:0) ranged from 3.07 to 3.22%, of C15:0 ranged from 0.41 to 0.48%, of palmitic (16:0) ranged from 26.24 to 27.25%, of margaric (17:0) ranged from 0.93 to 1.23%, of stearic (18:0) ranged from 11.94 to 13.45%, of arachidic (20:0) ranged from 0.18 to 0.26%, of behenoic (22:0) ranged from 0.18 to 0.26%, of lignoceric (24:0) ranged from 0.03 to 0.07%, of myristoleic (9c-14:1) ranged from 0.5 to 0.75%, of palmitoleic (9c-16:1) ranged from 3.31 to 3.90%, of 10c-17:1 was 0%, of elaidic (9t-18:1) ranged from 3.21 to 4.0%, of oleic (9c-18:1) ranged from 40.55 to 41.98%, of vaccenic (11c-18:1) ranged from 2.29 to 2.57%, of linoelaidic (18:2t) ranged from 0.01 to 0.02%, of linoleic (18:2n6) ranged from 10.14 to 14.53%, of linolenic (18:3n3) ranged from 1.02 to 1.77%, of gonodic (20:1n9) ranged from 0.03 to 0.43%, of C20:2 ranged from 0.16 to 0.19%, of homo-a-linolenic (20:3n3) ranged from 0.02 to 0.03%, of arachidonic (20:4n6) ranged from 0.23 to 0.28%, of EPA (22:ln9) ranged from 0.02 to 0.04%, of clupanodonic (22:5n3) ranged from 0.04 to 0.06%, of DHA (22:6n3) ranged from 0.07 to 0.11%, and of nervonic (24:1n9) ranged from 0.01 to 0.02%. For the solid samples, the wt.% of myristic (14:0) ranged from 2.45 to 2.7%, of C15:0 ranged from 0.34 to 0.41%, of palmitic (16:0) ranged from 23.84 to 24.76%, of margaric (17:0) ranged from 0.78 to 1.02%, of stearic (18:0) ranged from 10.24 to 11.57%, of arachidic (20:0) ranged from 0.29 to 0.45%, of behenoic (22:0) ranged from 0.11 to 0.21%, of lignoceric (24:0) ranged from 0.08 to 0.14%, of myristoleic (9c-14: 1) ranged from 0.38 to 0.60%, of palmitoleic (9c-16:1) ranged from 2.54 to 3.10%, of elaidic (9t-18:1) ranged from 2.35 to 3.09%, of oleic (9c-18:1) ranged from 37.19 to 34.90%, of vaccenic (11c-18:1) ranged from 2.05 to 2.27%, of linoelaidic (18:2t) ranged from 0.01 to 0.02%, of linoleic (18:2n6) ranged from 13.9 to 20.35%, of linolenic (18:3n3) ranged from 1.54 to 2.20%, of gonodic (20:1n9) ranged from 0.04 to 0.1%, of C20:2 ranged from 0.13 to 0.18%, of homo-a-linolenic (20:3n3) ranged from 0.02 to 0.03%, of arachidonic (20:4n6) ranged from 0.19 to 0.23%, of EPA (22:ln9) ranged from 0.05 to 0.2%, of clupanodonic (22:5n3) ranged from 0.03 to 0.04%, of DHA (22:6n3) ranged from 0.06 to 0.08%, and of nervonic (24:1n9) ranged from 0.01 to 0.03%.

The low CV indicates that the processes described herein can produce agricultural admixtures with consistent composition profiles.

### Example 5. Blending of basalt rock with agricultural admixtures for obtaining enhanced crop yields

Basalt rock was mixed with the agricultural admixtures described herein after processing the agricultural admixture to produce a mineral-enriched agricultural admixture. Alternatively, the basalt rock was administered to the crop separately from the administration of the agricultural admixtures described herein. The inventors have surprisingly discovered that basalt rock comprises reduced iron (Fe(I) or Fe(II)) which can reduce organic compounds in the agricultural admixtures described herein to ammonia gas (NH₃) or ammonium salts (NH₄⁺) resulting in high nitrogen fertilizer compositions. The high nitrogen fertilizer compositions from the basalt-blended agricultural admixtures described herein produced an increase in microbial activity thereby stimulating crop yield.

The strawberries (cv. *Portola*) utilized for this experiment were grown in a conventional field setting in Oxnard, California. This trial was set up as a completely randomized block trial of one rate of H2H 3-2-1 organic fertilizer alone and in combination with an earlier basalt material application and compared to the basalt alone overlaid on a grower standard compared to a grower standard, with completely randomized data collection of four replicates maintained during the growing season. All treatments received conventional in-season applications of nitrogen, phosphorus and potassium fertilizers. All H2H materials were applied in the growers' below ground drip tape during the season. The basalt material was hand spread prior to bed formation. Basalt in the basalt:Grower's standard formulation was applied at a rate of 1235.55 kg/ha (0.5 tons per acre). Basalt in the basalt: Grower's standard:H2H formulation was applied at a rate of 1235.55 kg/ha (0.5 tons per acre). When H2H was applied, it was applied at a rate of 93.54 l/ha (10 gallons per acre) per treatment. All cohorts were treated with the same levels of grower's standard.

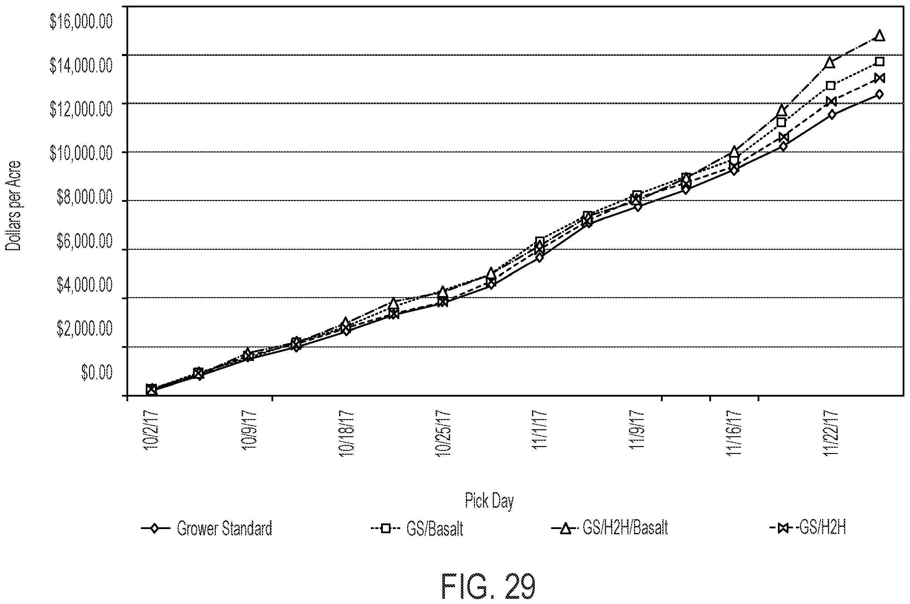

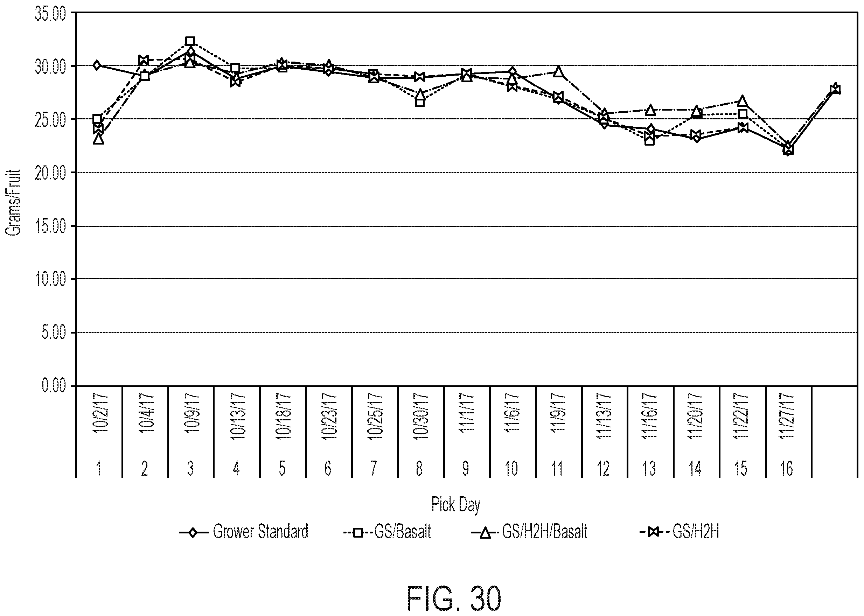

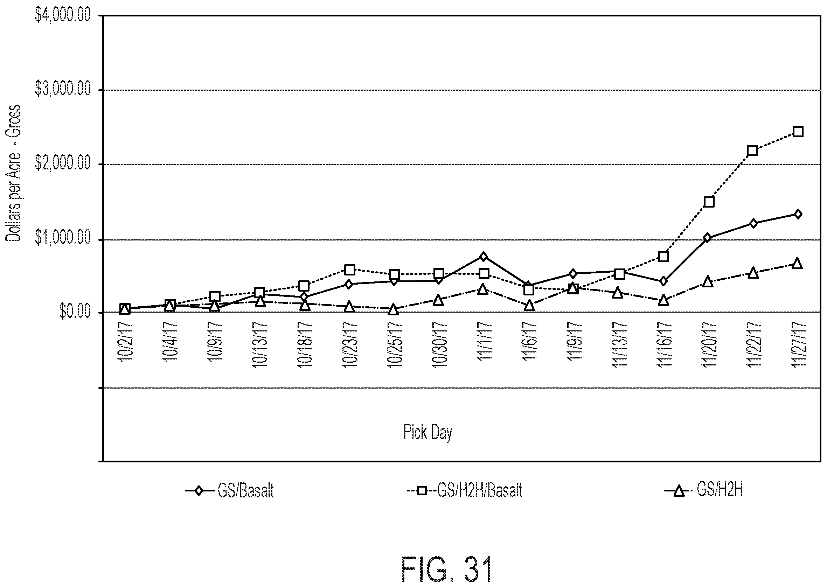

As shown in FIG. 29, H2H with basalt and grower's standard exhibited a marketable increase in production in calibrated trays per acre for all treatments for each pick day both on a daily and cumulative basis. The treatments of basalt in combination with H2H 3-2-1, and basalt alone, produced the most extrapolated flats of strawberries during the trial period with the most flats on average for the pick period of 2037 and 1942 flats per 0.40 ha (acre), compared to the grower standard at 1778 flats per 0.40 ha (acre). A different perspective of how the rated production affected final grower returns is shown in FIG. 31 which shows the daily marketable returns based on USDA Shipping Point Market Prices found at HTTP:\\marketnews.usda.gov/portal (as of 2017 growing season) for each pick day. This data is represented as the net revenue after costs of approximately $6.00 per tray were removed (*e.g*., costs attributable to picking labor, carton and tray costs, transportation to the cooler, and cooling costs associated with picking the strawberries). Based on this data numerically cumulative seasonal increase to the grower's return was seen by the use of basalt with H2H and grower's standard over the other treatments. FIG. 29 shows the daily market utilization for the berries picked during the season, that is the percent of marketable berries to the total weight of berries picked, with significant differences noted for all treatments over the grower standard with utilization averaging between 80.6% and 84.4% on average. The best utilization was seen with treatments GS:H2H:basalt, to GS:basalt, to GS:H2H in descending order. FIG. 30 shows the net differential in returns to the farm for each pick day for the treatment programs over the grower standard, which in this case was quite different for all of the treatments with GS:H2H:basalt at $2427, GS:basalt at $1341 per acre, and GS:H2H following at $667 per acre.

The results demonstrate that the utilization of the agricultural admixture products produced by the methods described herein in conjunction with a grower standard program adds value to the grower's production. The results also demonstrate the synergistic effects of how the H2H products (agricultural admixture) compared to a basalt based product, and the basalt in combination with the H2H products. Basalt alone provides a superior yield over the H2H 3-2-1, and only when an agricultural admixture is combined with basalt are additional fruit weight yield and increased revenue observed.

In some embodiments, the amount of agricultural admixture described herein is 5 to 50 weight percent of the resulting mixture, preferably about 10 weight percent, on a dry matter basis. In some embodiments, the basalt rock application rate is 560.43 to 2241.7 kg/ha (500 to 200 pounds of basalt per acre) per growing season. In some embodiments, the agricultural admixture application rate is 18.93 to 378.54 litres (5 gallons to 100 gallons) per ton of basalt rock dust, to be composted prior to application of the mixed compost, to be applied annually on organic crops and/or organic dairy alfalfa or hay or rangeland. In some embodiments, the agricultural admixture is applied once, twice, or thrice per growing season. The basalt rock mixed with the agricultural admixtures described herein can be certified for use in organic farming. The basalt rock mixed with the agricultural admixtures described herein are applied to feed pastures for organic dairies. The basalt rock mixed with the agricultural admixtures described herein are applied to rangeland for free range beef and chicken production. The mineral-enriched agricultural admixture can increase crop yield, or increase forage volume (the volume of food available to animals feeding in rangeland or feed pastures) in regenerative agriculture by 5 to 25 percent, including 5, 6, 7, 8, 9, 10, 11, 12, 13, 14, 15, 16, 17, 18, 19, 20, 21, 22, 23, 24 percent.

### Example 6. Processing soybeans with lettuce culls to produce a high nitrogen content organic nutrient admixture

Organic soy meal may be hydrolyzed in water (9:1 water:soy, by weight) at a temperature range of 60 °C - 82.2 °C (140 - 180 °F) and adding an alpha amylase and a protease, under conditions of constant shear, using the systems described herein, at a pH of 4.5, for about 3 hours, to create a soy meal slurry. After incubating the soy meal slurry, the slurry is screened with coarse and fine filters, and centrifuged using a tricanter centrifuge, to further remove solids, to yield a high nitrogen soy hydrolysate. Alternatively, organic soy meal and recycled vegetables (which can include or exclude lettuce, spinach, kale, cabbage, and other leafy greens and brassicas) are added to the incubation tank at a weight ratio ranging from about 20:1 to about 5:1 (of recycled vegetables:soy, by weight). The recycled vegetables may be used in lieu of water to hydrolyze the soy meal, given their high water content. The recycled vegetable-soy meal biological recyclable stream is hydrolyzed at a temperature range of 60 °C - 82.2 °C (140 - 180 °F) and adding an alpha amylase, a protease, and a cellulose, under conditions of constant shear, using the systems described herein, at a pH of 3.5 - 7.0, for about 3 hours. After incubating the soy meal-recycled vegetable slurry, the slurry is screened with coarse and fine filters, and centrifuged using a tricanter centrifuge, to further remove solids, to yield a high nitrogen soy hydrolysate. The resulting finished product is safe for use as a fertilizer to increase crop yields in organic vegetable production.

### Example 7. Synergistic Effects on Plant Growth from combined treatment of agricultural admixture and bone, blood, and feather meal

Application of labile forms of carbon, including the agricultural admixtures made by the processes described herein was found to stimulate microbial activity, resulting in rapid breakdown of amendments, including bone, blood, and feather meal. The rapid breakdown of amendments was found to yield faster nitrogen mineralization compared to the absence of said agricultural admixtures. Faster nitrogen mineralization resulted in faster plant growth.

Although bone, blood, and feather meal are known to stimulate microbial beneficial soil microorganisms (Quilty J., et al., Soil Res., 49, 1-26 (2011), prior to this disclosure no information was known about the combination of bone, blood, or feather meal with the agricultural admixtures described herein on the effects of soil microbe, chemistry, and plant growth.

Two experiments were conducted, one with soil only to measure the mineralization rates, and one with tomato seedlings to measure effects of plant growth. Three amendments were evaluated: a bone meal mix (Nature Safe^{™} 7-12-0), a blood meal mix (Nature Safe^{™} 8-5-5) and a feather meal mix (Nature Safe^{™}13-0-0). Each amendment was assayed for increased plant growth and mineralization rates, alone and in combination with an agricultural admixture made by the methods described herein ("H2H"). Controls included H2H alone, and water. The rates of the amendment additions were adjusted to normalize the nitrogen application in all cases. The H2H was diluted 10:1 in water and applied from a rate of 467.70 1/ha (50 gallons per acre).
There were eight soil treatment conditions assigned as follows:
Bone meal only
Feather meal only
Blood meal only
Water only
Bone meal + H2H
Feather meal + H2H
Blood meal + H2H
H2H only

The listed N-P-K (nitrogen-phosphorous-potassium) contents of the amendments, with normalized nitrogen content, and amount of normalized amendment added per experiment (performed in a tube) are summarized in Table 11.
(1 pound = 0.45kg )

**Table 11. Summary of soil amendment test conditions.**

| | N-P-K ratio | Amount of amendment required for 1 pound of nitrogen | Amount of amendment added per experiment |
|---|---|---|---|
| Bone meal | 7-12-0 | 14.3 lbs | 88 mg |
| Blood meal | 8-5-5 | 12.5 lbs | 77 mg |
| Feather meal | 13-0-0 | 7.7 lbs | 47 mg |

Soil was collected from an unamended irrigated soil previously planted with almonds. Soil was mixed thoroughly by hand at field moisture levels, and stored in a cold room (4-6 degrees Celsius) until the beginning of the experiments. Before the experiments, the moisture content of the soil was adjusted to 40% water holding capacity.

Bioassay chambers were prepared from PVC columns (31.5 cm length, 4 cm diameter), and were capped on one end with a 6 mm diameter hole, with a mesh covering the hole to prevent soil loss. The holes were fitted with a removable stopper. The PVC columns and mesh were thoroughly washed with water prior to the introduction of soil. The PVC columns were then allowed to drip dry. Each of the eight treatment conditions was replicated five times, for a total of experiments (with one tube per experiment, for 40 tubes). For each treatment, a batch of soil was prepared, mixed with the treatment condition, and divided into individual chambers. The H2H was applied at a rate of 467.70 1/ha ( 50 gallons per acre ), diluted 10:1 in water, scaled down to the surface area of the chambers (12.6 cm²), such that 0.06 ml of H2H was applied in 0.6 ml of water per tube. All experiments were performed at room temperature. The experiments were performed twice.

To measure leachate from each chamber, 100 ml of ddH2O (double distilled water) was added the day of application (day 1), and also at days 3, 7, 14, 28, and at 2 months. For each measurement, the bottoms of the columns were unstopped and allowed to drain for 2 hours. Samples were stored in 15 ml plastic scintillation vials at -20 degrees C until analysis for inorganic nitrogen. Nitrate and ammonium concentrations were determined by colorimetric analysis and comparison to standard curves per well-understood methods (Keeney et al., Nitrogen - inorganic forms. In A.L. Page (ed.), Methods of Soil Analysis, part 2. Agron. Monogr., 2nd ed. ASA and SSSA, Madison, WI, p. 643-698, 1982).

Tomato seedlings of the "Rutgers" variety were planted in soils amended as described above, with five replicates of each treatment combination in four-inch diameter pots. Seedlings were maintained on growbenches in the laboratory for four weeks at room temperature after which plant size metrics including plant height, dry weight, root length, and root biomass were measured.

### Leachate Analysis Results

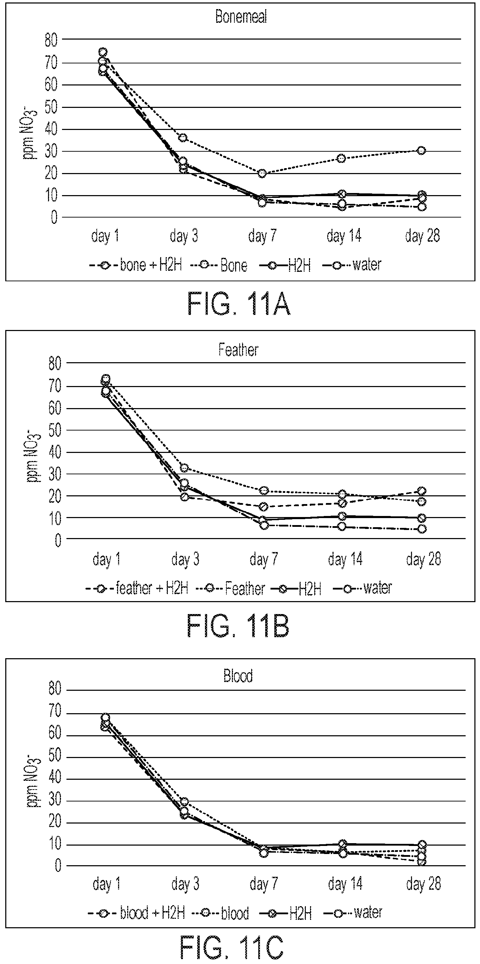

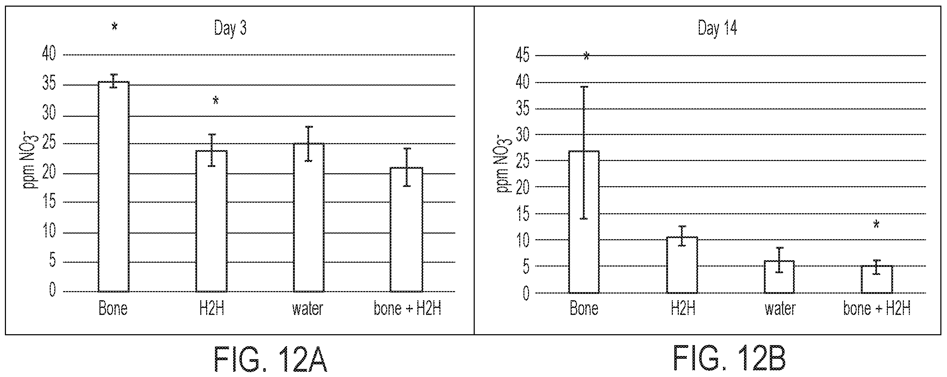

The nitrate content of leachate from all treatments started high and decreased rapidly, but no striking differences were seen in the rate of decrease over time between the treatments, although the amendments themselves tended to be a little higher (FIG. 11). There were some differences between the treatments for individual dates. For example, in the first experiment at day 3, bonemeal alone had significantly higher nitrate ppm than H2H in combination with bonemeal (P<0.01, t=-4.0, FIG. 12) a trend that was repeated until day 14 (P=0.04, t=2.8). The decreases in nitrate seen during this time were coupled with increases in ammonium with the H2H and bonemeal treatment, which is described further below.

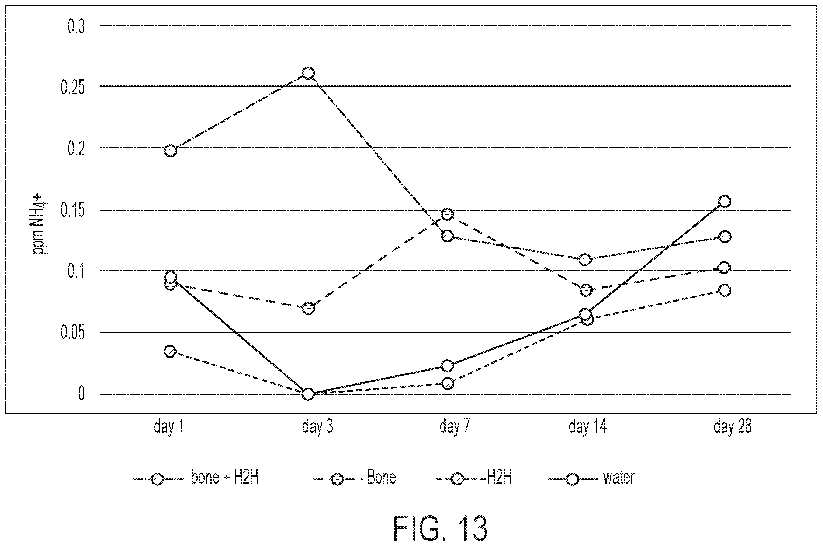

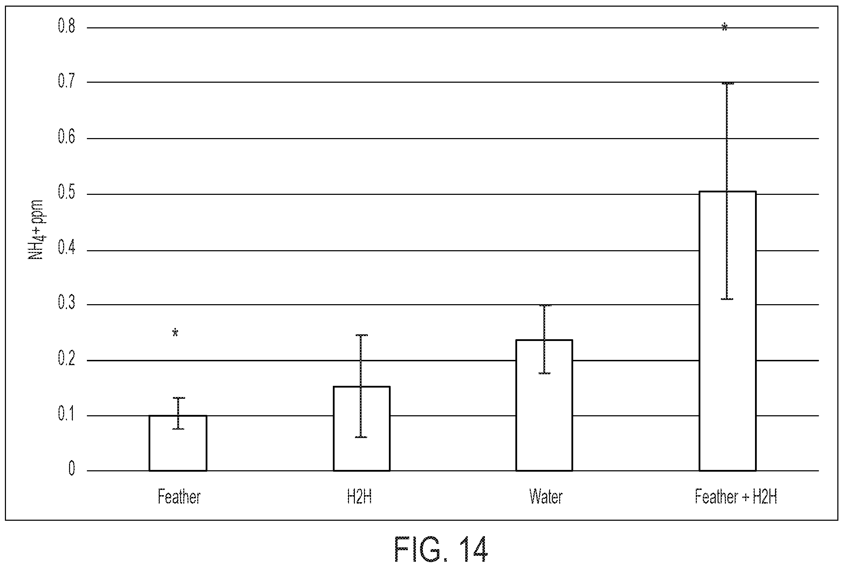

Although ammonium concentrations were lower than nitrate, reaching only about 5 ppm, they showed a larger variation between the treatments. For example, bonemeal in combination H2H had higher concentrations of NH₄⁺ (ammonium) than bonemeal alone, both at day 1 (P=0.03) and day 3 (P<0.01) in experiment #2 (FIG. 13). A similar trend was observed in the first experiment, except at day 7 (P=0.09), although the trend was not as strong. The increases in ammonium seen with the H2H and bone treatment coincided with a reduction in nitrate. H2H similarly increased ammonium leachate for the feather meal amendment (FIG. 14), with feather in combination with H2H having higher concentrations of ammonium than feather alone at day 14 (P=0.05).

Rapid mineralization of the nitrogen from the organic fertilizers (amendments with H2H) was observed within the first two weeks, with mineralization after that proceeding more slowly. Without being bound by theory, enzymatic hydrolysis of urea and simple proteins in the amendments releases nitrogen into the soil.

The synergistic increases seen in the ammonium concentrations from the combination of amendments with H2H suggest increased microorganism activity which is consistent with the hypothesis that H2H can increase nutrient availability by simulating the soil food web. The combination of low nitrate with higher ammonium in H2H treated soil may indicate that more mineralization was happening due to ammonification (the production of ammonium) rather than nitrification (the production of nitrate).

### Enhanced Plant Growth Results

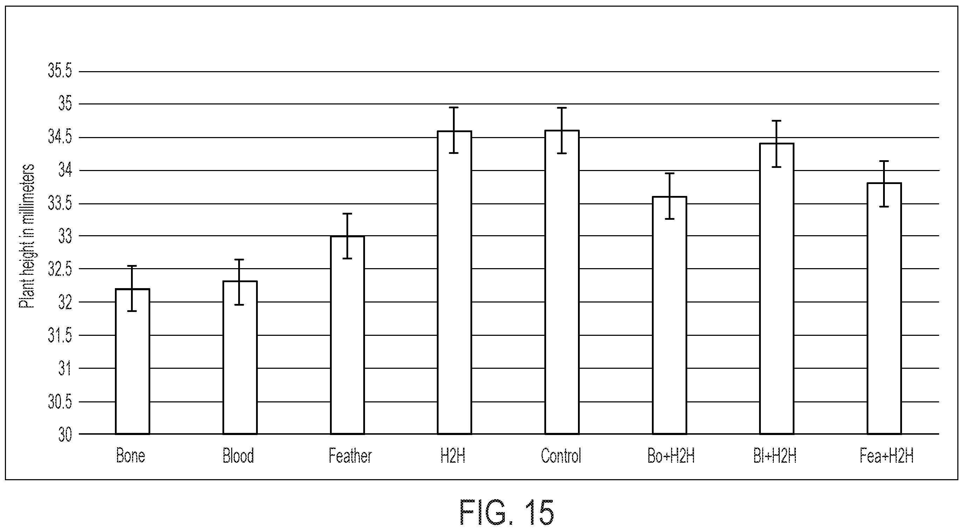

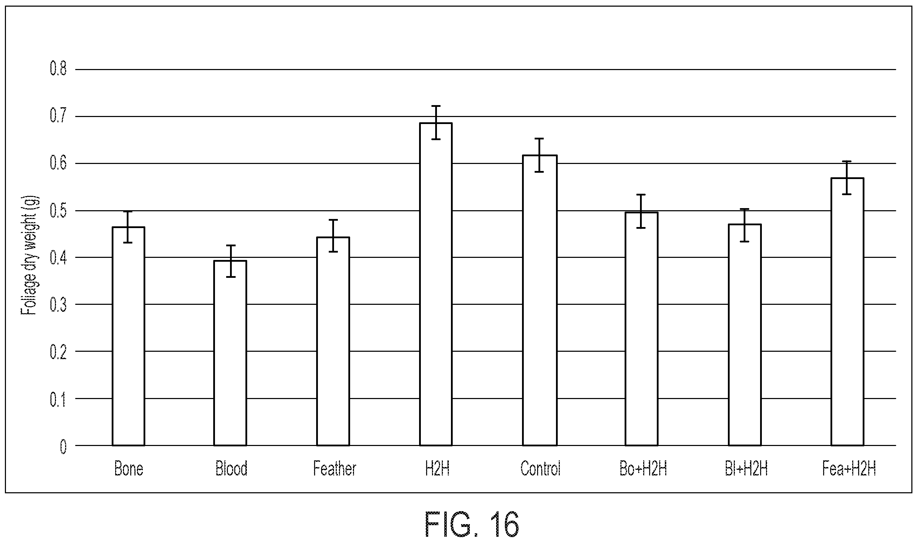

After 30 days, the H2H and Control treatments had grown the most (FIG. 15). The above ground biomass of H2H treated plants was 49% higher than bonemeal treated plants, 80% higher than bloodmeal treated plants, and 56% higher than feathermeal treated plants, while the controls were 34%, 62% and 40% higher respectively. The inhibitory effect of the amendments on growth was surprisingly discovered to have been overcome by the H2H. All plants grew more in the amendment combination treatments with H2H, compared to the amendments alone. A similar trend was seen in total aboveground plant biomass (FIG. 16). Root biomass and root length differed little between the treatments, and the root shoot ratio, a measure of how much energy the plant is allocating to belowground versus aboveground biomass, did not show large differences.

Without being bound by theory, the counterintuitive decreased growth seen with the H2H-amendment combination treatments compared to Controls may have resulted from breakdown of more labile forms of nitrogen into urea early in the experiment inhibited plant growth. Without being bound by theory, the release of ammonia from such amendments could have a temporary toxic effect on sensitive microbes, although the effect likely depends on soil type and application rate. Such inhibition of microbes could have slowed the nitrification process, limiting nitrogen available to plants, slowing their growth. All amendment types, however, were observed to yield increased plant growth when combined with H2H, compared to the amendments alone.

### Example 8. Use of agricultural admixtures as animal provender - measurements of animal weight growth

Growing-finishing pigs were fed either a solid diet of corn-soybean meal ("solid diet") or started on a diet comprising an exemplary liquid slurry form of an agricultural admixture of this disclosure, comprising liquid and particulates ("agricultural admixture diet") before switching to the solid diet. It was observed that the liquid form of the agricultural admixture of this disclosure, made from biological recyclable waste streams could be used as a sufficient feed source for pigs.

It was discovered that the compositional analysis of the hydrolysates produced by the methods described herein are very close to the ideal protein profile for growing pigs. As described in Example 4, the indispensable amino acid profiles of some embodiments of the hydrolysates produced by the methods described herein are consistent across batches. It was discovered that both dried and liquid (and mixed) hydrolysates provide a balanced amino acid profile to growing pigs with optimal growth and reduced nitrogen excretion. Reduced nitrogen excretion affords a larger volume of pigs per unit area, because high nitrogen excretion pollutes runoff water, nearby air quality, and soil quality. In addition, it was discovered that the hydrolysates of this disclosure include appropriate amounts of minerals and nutrients for use as animal provender, including Calcium, Phosphorous, Copper, Iron, and Manganese. In some embodiments, the hydrolysates can be further supplemented with other minerals when used as the exclusive source of animal provender, including or excluding calcium, phosphorous, zinc, and arsenic. Furthermore, the hydrolysates made by the methods described herein contain higher amounts of disaccharides and oligosaccharides but less starch compared to corn. The results indicate that the hydrolysates are expected to provide more energy as animal provender than corn because high starch and fiber content is known to reduce digestibility of amino acids, energy, and other nutrients (Zhang, W., et al., 2013. The effects of dietary fiber level on nutrient digestibility in growing pigs, J. Anim. Sci. Biotechnol. 4, 17).

In the growing-finishing pig trial, 64 pigs were split into the solid diet group or the agricultural admixture diet group. Pigs were monitored in three phases-the first phase of 35 to 60 kg weight pigs (2 weeks); the second phase of 60 to 90 kg weight pigs (for two weeks), and the third phase of 90 to 120 kg weight pigs. In the study of growing-finishing pigs, the pigs fed the agricultural admixture diet were switched to the solid diet during the third phase of the trialthe 90 to 120 kg weight phase. Measurements were obtained, including growth performance, daily weight gain, feed intake, feed efficiency and carcass quality.

In the trial with nursery pigs, 108 pigs were split into two groups-one group was fed a corn-soybean meal diet, while the other group was fed the agricultural admixture diet for phase 1 (2 weeks), and then switched to the solid diet for the second phase (2 weeks). Measurements were obtained, including growth performance, daily weight gain, feed intake, feed efficiency and frequency of diarrhea.

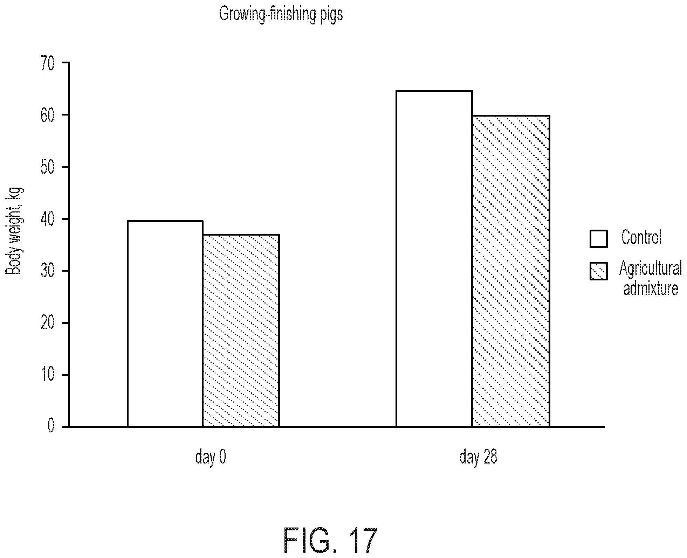

FIG. 17 shows that the agricultural admixture made from recyclables was a suitable feed for growing-finishing pigs, yielding similar weigh gains to the cornmeal-soy diet (weight gains that were not significantly different from the corn-soybean control animals). This demonstrates that the admixtures of this disclosure can be used to provide sustainably grown, healthy livestock.

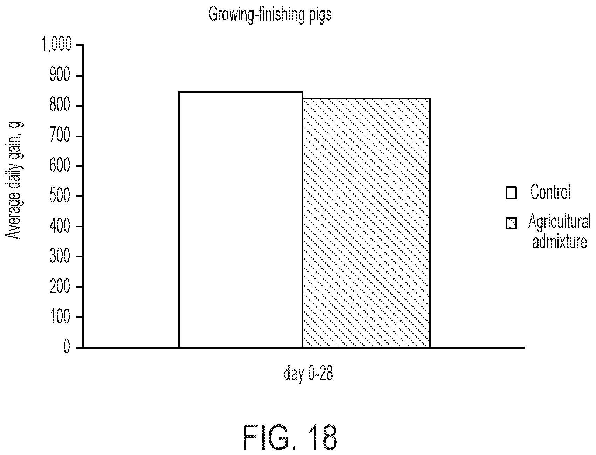

In addition, FIG. 18 shows that the hydrolysate feed also yielded approximately the same average daily weight gain to the cornmeal-soy diet.

Although the pigs fed hydrolysate gained marginally less weight by day 28, supplementing the hydrolysate as described herein, for example, by adding carbohydrates and/or de-watering the hydrolysate into a sold pelleted product will increase weight gain in hydrolysate fed pigs compared to pigs fed traditional solid feeds such as the cornmeal /soy feed. Animals fed the liquid admixtures had larger stomachs than the control animals, indicating that consumption of calories from the liquid diet was limited by the size of the animals' stomach. The animals produced less manure and had less diarrhea when fed the pre-digested composition. In addition, feeding pigs the nutrient rich compositions of this disclosure yields pigs with leaner meat, reduced diarrhea, and/or other health benefits such as lower incidence of infections and/or disease.

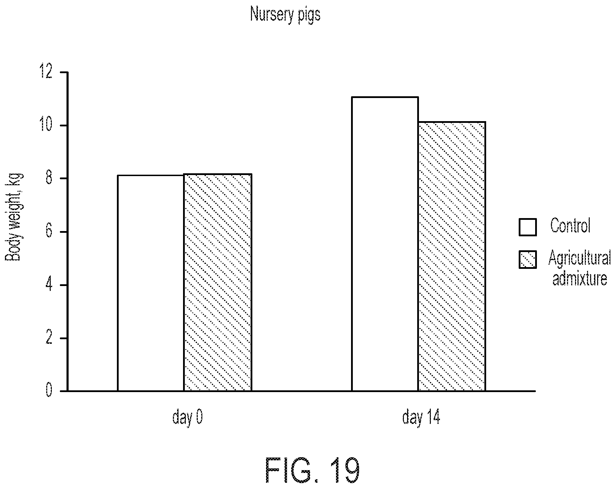

The results from the nursery pigs show that the hydrolysate diet was a suitable feed, yielding similar weigh gains to the cornmeal-soy diet, as shown in FIG. 19.

In addition, pigs fed with the hydrolysate feed had reduced diarrhea levels. Thus, in some embodiments, feeding livestock such as pigs the hydrolysate admixture improves animal health.

Furthermore, it was discovered that the hydrolysate comprised a high level of unsaturated fatty acids, which was included in the animal feed provender. The animals fed with a diet comprising unsaturated fatty acids from the hydrolysates described herein are expected to exhibit a high amount of unsaturated fatty acids post-slaughter. In non-ruminant animals, fatty acid profiles in tissues reflect the fatty acid profiles in their provender. Provender which is enriched with unsaturated fatty acids may in some embodiments could increase the concentration of unsaturated fatty acids in pork (*See* Nguyen, L.Q. et al., Mathematical relationships between the intake of n-6 and n-3 polyunsaturated fatty acids and their contents in adipose tissue of growing pigs, Meat Sci. 65, 1399-1406 (2003); Mitchaothai, J. et al., Effect of dietary fat type on meat quality and fatty acid composition of various tissues in growing-finishing swine, Meat Sci. 76, 95-101 (2007)), thereby indirectly enhancing the health of pork consumers.

In some embodiments, hydrolysates of different fat compositions can be fed to the animals at different growth stages. In some embodiments, hydrolysates made with reduced fat levels using the tricanter centrifuge according to the methods described herein can be fed to weanling pigs. Hydrolysates made with non-reduced fat levels according to the methods described herein can be fed to pigs at later stages of growth, preferably during the growing-finishing period to increase provender energy density and diet palatability (Kerr, B.J. et al., Characteristics of lipids and their feeding value in swine diets, J. Anim. Sci. Biotechnol. 6, 30 (2015)).

In some embodiments, additional nutrients can be added to the hydrolysate to increase weight gain of the animals for use of the agricultural hydrolysate as animal provender to customize the carbohydrate and sugar balance in the animal provender.

In some embodiments, additional carbohydrates may be added to the hydrolysate. Carbohydrates may be supplied, for example, by adding bakery goods, or hydrolyzed bakery goods. In some embodiments, bread crumbs, soymeal, distiller's grains, and/or almond hulls may be added to the hydrolysate for use as feed supplements. Distiller's grains can include or exclude: barley, corn, rice, and hops. In some embodiments, the hydrolysate can be in a dewatered (essentially dry) or liquid form when combined with the additional carbohydrate source. In some embodiments, a supplement comprising from 20%, 25%, 30%, 35%, 40%, 45%, 50%, 55%, 60%, or 65%, or any range of carbohydrate percentages between any two of the recited percentages, may be added to the agricultural admixtures. In some embodiments, the carbohydrate supplemented agricultural admixture can be dewatered and pelleted. In some embodiments particulates from the biological recyclables, for example, particulates obtained by filtering the hydrolysate or from the tricanter centrifuge, may be added to the hydrolysate. In some embodiments the particulate matter may be high in protein.

In some embodiments, the agricultural admixtures fed to weaning pigs may be supplemented with particulates high in protein, while the hydrolysate fed to growing-finishing pigs may be supplemented with carbohydrate. In some embodiments, the agricultural admixture fed to either weanling pigs or growing-finishing pigs may be supplemented with fats, for example saturated and/or unsaturated fats. Supplementing the agricultural admixture with either carbohydrates, fats or proteins includes any process that increases the percentage of carbohydrates or proteins in the hydrolysate by more than 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40%, or by any range of percentages between any two of the recited percentages.

### Example 9. High-conversion animal provender using liquid agricultural admixtures

The agricultural admixture produced by the methods described herein comprising a constituent which can include or exclude: proteins and/or peptides, fats, fiber, and carbohydrates, can be used as a pre-digested feedstock for animals. The inventors have recognized that the pre-digested feedstock has a higher mass conversion rate of feed to animal weight compared to a standard feed product. Standard animal provender product comprises undigested corn, soy, alfalfa, and/or oats.

The agricultural admixtures of this disclosure can be used as a high-conversion rate animal provender. Animals (pigs and/or chickens that are usually fed a diet of corn & soy meal) can be fed the liquid or dried agricultural admixtures of this disclosure to gain weight with increased food use efficiency (*i.e.*, an increased conversion rate of food into animal weight). In some aspects, the animals produce less manure and have less diarrhea when fed the pre-digested composition. Accordingly, approximately 100% of the biological recyclable stream processed according to the methods of this disclosure can be efficiently utilized.

### Example 10. High-conversion chicken-feed using dried agricultural admixtures

The agricultural admixture produced by the methods described was used for hatchling chicken feed to demonstrate the enhanced conversion rate of the agricultural admixture relative to a control diet comprising soy and cornmeal.

The control diet met or exceeded the Cobb recommendations for chicken hatchlings. The control diet ingredients are listed in Table 14, assuming 90 wt.% dry matter. The composition of the control diet is listed on Table 15.

The control diet was mixed with the agricultural admixture ("H2H") and bread at weight ratios of 100-0-0 ("control"); 50-25-25 ("50-50"), and 75-12.5-12.5 ("75-25"). The nutrient composition of the three diets is listed in Table 16.

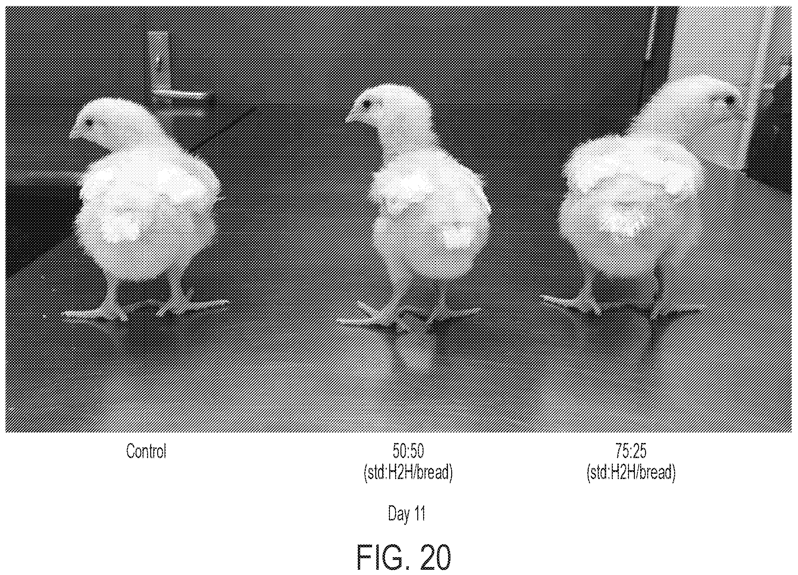

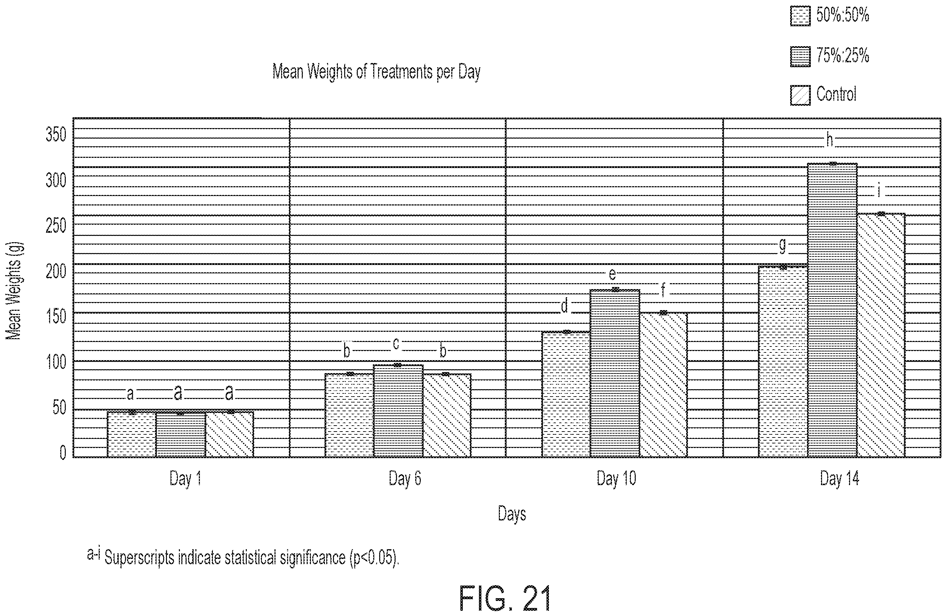

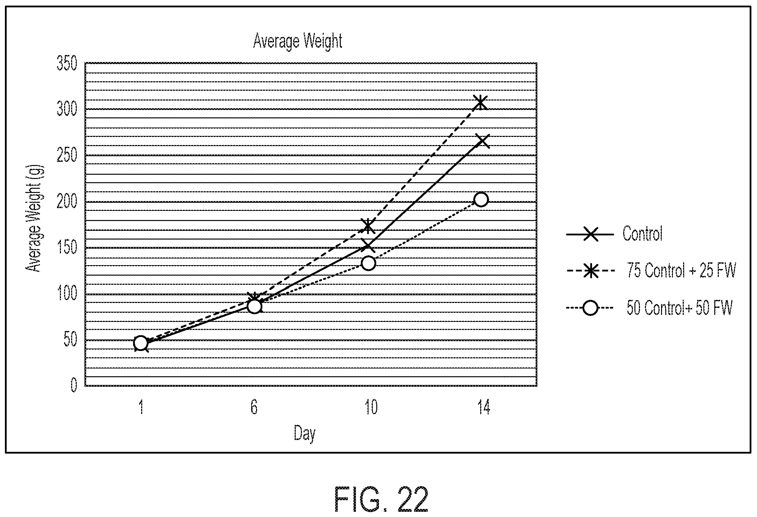

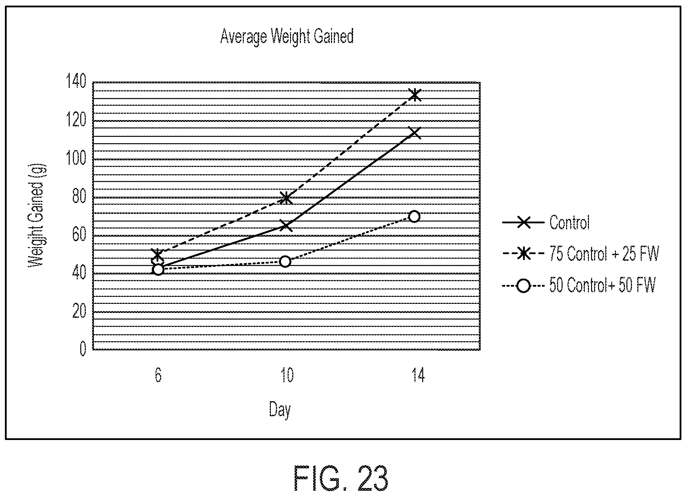

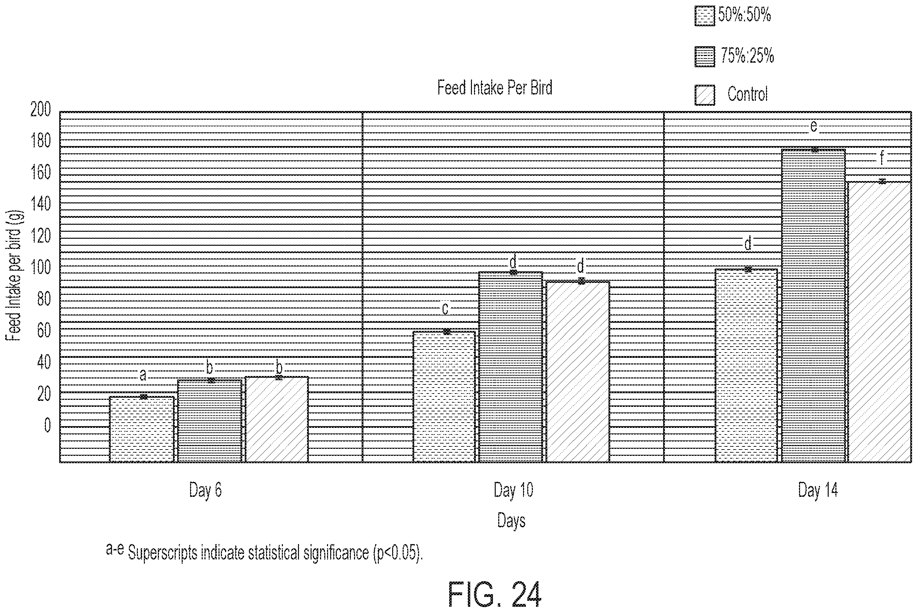

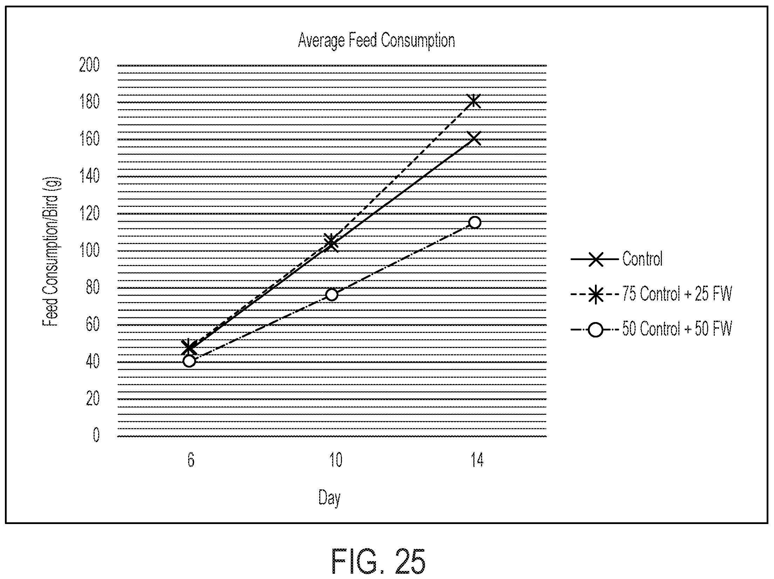

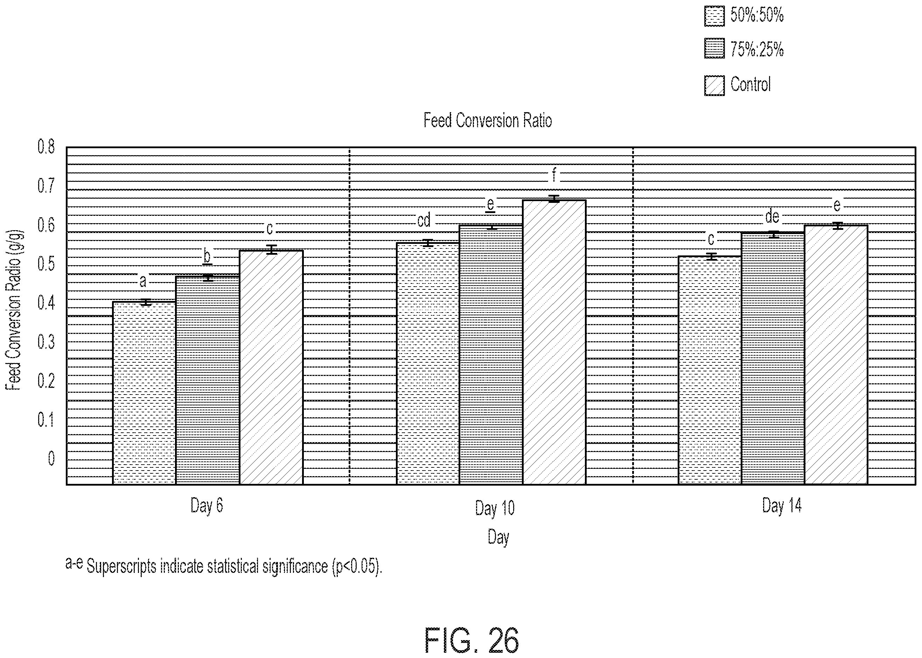

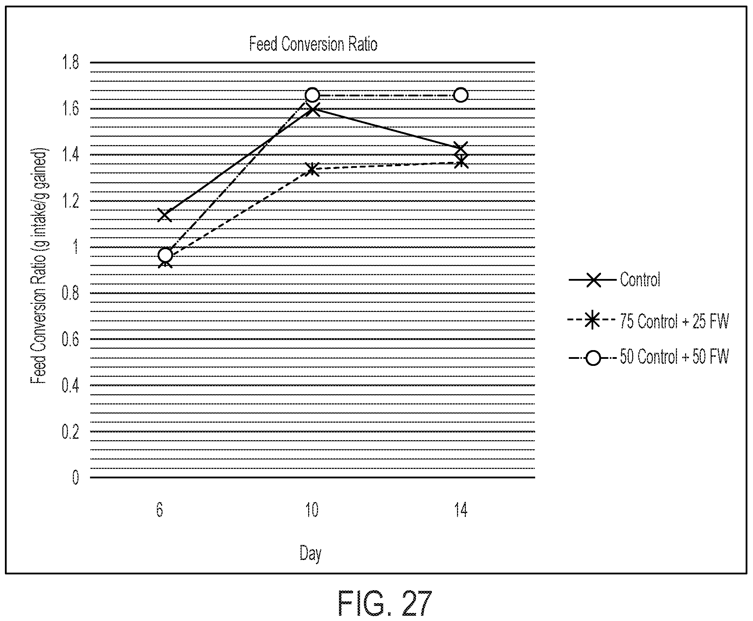

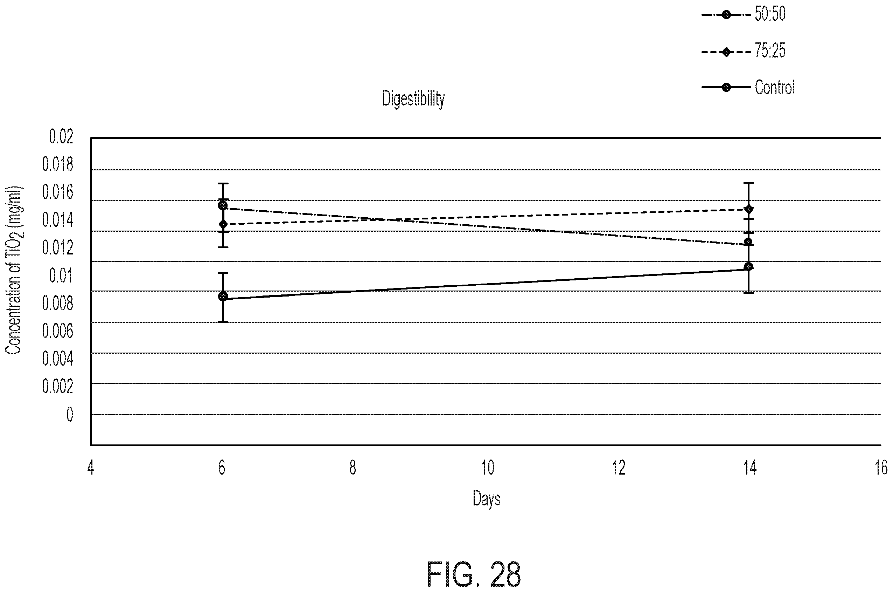

Three cohorts comprising 144 hatchling chicks (broiler) per cohort were fed a diet of 50:50, 75:25, or strict control feed for their first 14 days. The animals were allowed to eat *ad libitum.* The chicks were divided into six chicks per cage, with 72 total cages. One chick from each cage was sampled on days 6, 10, and 14, to determine the effects of the feed diets on hatchling growth and feed conversion uptake. Representative sizes of the diet treatment cohorts at 11 days of feeding is shown in FIG. 20. FIG. 20 shows that cohorts fed with the 75:25 (Control :Ag-admixture/bread) achieved the largest overall animal volume and breast meat volume. FIG. 21 shows that the cohorts fed with the 75:25 diet had the highest weight-per-bird ("weights of treatment"). FIG. 22 shows that the average weight of the cohorts fed with 75:25 was consistently higher than that of the cohort fed with Control feed or the 50:50 feed. FIG. 23 shows that the cohort fed with the 75:25 feed exhibited the most weight gain compared to the Control feed or the 50:50 feed. One of the reasons the cohort fed with the 75:25 feed gained the most weight was that this cohort consistently had the highest per-bird feed update (FIG. 24, and FIG. 25). The difference in the feed conversion ratio, however, was less pronounced between the 75:25 and Control feeds because the mass of the cohort fed with the 75:25 feed was larger and closer to full maturity, so the feed conversion plateaued after 10 days of feeding (FIG. 26 and FIG. 27). The feed conversion ratio indicates that the cohort fed with the 75:25 diet yielded more output when fed the same amount of food than Control or 50:50 diet. The Control cohort was trending towards the same feed conversion ratio as the 75:25 cohort at day 14 of feeding. The digestibility of the feed was measured using known methods in the art (F. Short, et al., Animal Feed Science and Technology, 1996, 59: 215-221). The digestibility of both Ag-admixture cohorts (75:25 and 50:50) was consistently higher than the Control feed cohort (FIG. 28).

The serum chemistry of the sacrificed cohorts was analyzed, as shown in Table 17. The results indicate that the cohorts treated with Ag-admixtures and bread exhibited higher cholesterol levels than the Control feed cohort, but lower Glucose and Triglycerides content after 14 days of feeding.

The results indicate that a proper balance of fat content and pH in the feed differences most likely led increased food uptake, which when combined with the higher feed conversion ratio of the Ag-admixture/bread feeds, led to the observed increased weight gain. Thus, the inventors have demonstrated that compositional control of the animal provender, such as Animal Provender (I), produced by the methods described herein including selective fats removal or addition enables production of an animal provender which results in a surprisingly large animal weight difference compared to animals fed with a control diet.

### Example 11. Agricultural admixtures from Brassica as natural pesticide

The liquid hydrolysate obtained from the agricultural admixtures described herein are useful for suppressing or inhibiting soil-pest growth. Feedstocks comprising brassica spp. yield high levels of the soil-pest inhibitor isothiocyanate from the hydrolysis of glucosinolates present in the brassica spp. Glucosinolates are derived from amino acids and are stored in the vacuoles of cells of all tissue types within the plant (M. Morra, et al., Soil Biology and Biochemistry, 2002, 34:1683-1690) . After tissue damage from the grinding and shearing and cellulase activity induced by the processes and enzymes described herein, glucosinolates are cleaved by added thioglucosidase (myrosinase; EC 3.2.1.1), producing many products including isothiocyanates, nitriles, and thiocyanates. Isothiocyanates are biologically active, disrupting cellular components, including those of soil-pests by denaturing protein structure.

A feedstock comprising *Brassica juncea* (mustard green) is processed using the methods described herein, where the processing enzymes include a cellulase to break down the cellular structure and optionally a thioglucosidase to maximize glucosinolate hydrolysis which results in isothiocyanate release.

In some embodiments, the feedstock can comprise one or more *brassica* species, including those described herein.

Soil which was not used for one grow season is divided into three or more parts. One part is treated with water as a control. One other soil part is treated with inorganic fertilizer (Grower's Standard) as another control. Another soil part is treated with the agricultural admixture from *brassica spp.* feedstock. Another soil part is treated with the agricultural admixture from *brassica spp.* feedstock combined with inorganic fertilizer (Grower's standard). Each soil part can be done in solo or in replicates. Tomato (cv. Rhodade) seedlings are added to each soil part. To each soil part is then added a measured amount of *P. neglectus* nematodes. The levels of nematodes in the soil are measured using the Baermann funnel method. General agronomic practices are implemented to raise the seedlings. Each soil sample with tomato seedling is treated separately with water, water with inorganic fertilizer, water with agricultural admixture from *brassica spp.* feedstock and inorganic fertilizer, and water with agricultural admixture from *brassica spp.* feedstock. The nematode populations are monitored before nematode introduction, at 1 day after nematode introduction, 2 days after nematode introduction, 3 days after nematode introduction, 1 week after nematode introduction, and 2 weeks after nematode introduction. The soil-pest nematode populations can decrease in soil samples treated with agricultural admixtures treated with *brassica spp.* feedstocks.

### Example 12. Centrifugal processing of agricultural admixtures to separate agricultural admixtures into higher value product streams

The processes to make the agricultural admixtures described herein further include the use of centrifugal processing to separate the hydrolyzed slurry into higher value product streams. Hydrolysate slurries prepared from fresh food streams were separated into aqueous, fat, and solid phases using a tricanter centrifuge (Flottwegg Separator (Germany)). The hydrolysate slurries tested had N-P-K levels of 1-0-0, 1-1-0 (made from high fish content), 3-2-1 (made from high fish content), and 1-1-0 made from 34% red meats. The use of the tricanter centrifuge allowed the slurry to be separated into an aqueous phase, an oil (fat) phase, and a solids phase. The fat contents were reduced from 6-12 % (wt.) in the slurry to 0.2-1.4% in the isolated aqueous phase using the tricanter centrifuge. In some embodiments, the isolated aqueous phase was able to be subsequently dewatered by the methods described herein. In some embodiments, the isolated fats were further separated into high-titer fats and low-titer fats.

The use of centrifugal processing enabled control of the amounts of fats, dry matter, crude protein, and ash in the separated products, as shown in Table 12.

Table 13 shows the mass percent change of the separated aqueous phase composition compared to the slurry after isolation using the centrifugal processing.

### Example 13. Crop Quality Improvement

An emulsified agricultural admixture was prepared as described herein. The admixture was reduced in fats content to less than 1.5% using the tricanter centrifuge. The admixture was blended with a dispersant to enable facile emulsification and delivery through drip-line irrigation.

Nine single plant replicates of romaine lettuce (cv. Green towers) were transplanted as plugs into a non-fertilized soilless growing media. The cohorts were drenched three days after transplanting and again 2 weeks thereafter with 93.541/ha (10 gallons per acre) of H2H 3-2-1 and an organic fish hydrolysate fertilizer.

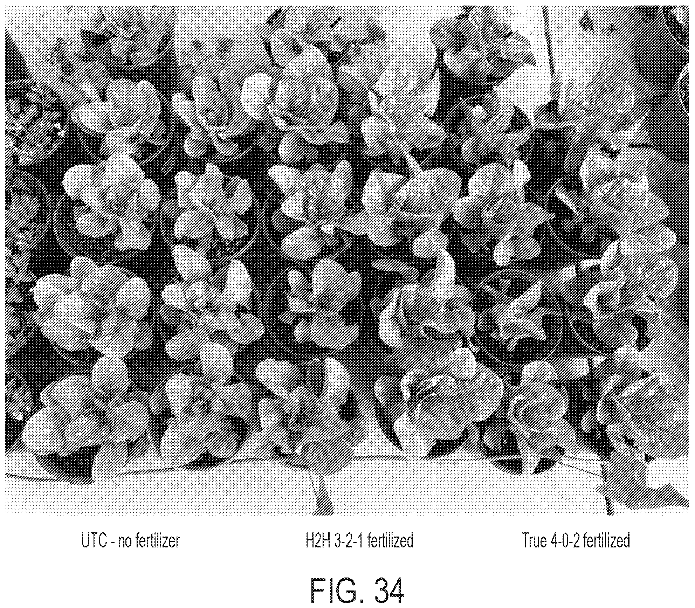

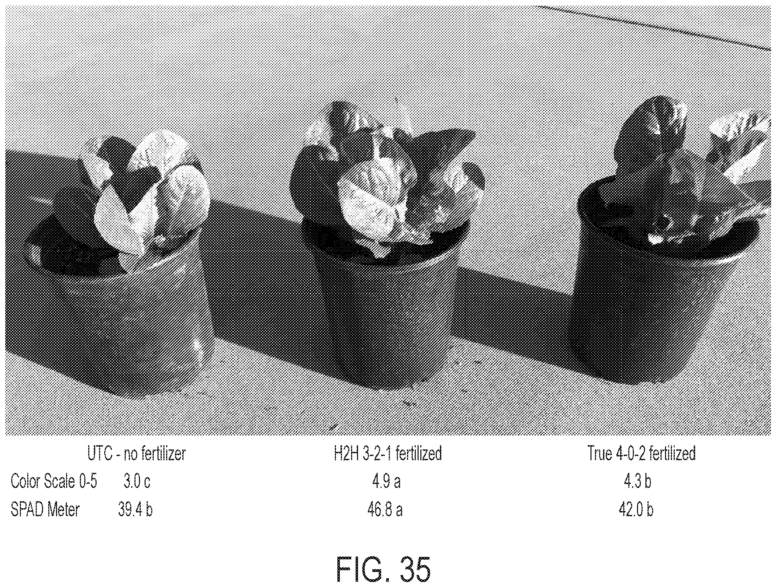

As shown in FIG. 34, the lettuce at 4 weeks after transplanting treated with H2H were consistently larger and greener than cohorts treated with no fertilizer or fish hydrolysate fertilizer. As shown in FIG. 35, the cohorts treated with H2H exhibited a higher color (averaging 4.9) as measured by a 0-5 color scale (with 0 the lowest, and 5 the highest), and also exhibited a higher chlorophyll content (relative chlorophyll content as analyzed with a Minolta SPAD meter) of 46.8, compared to no fertilizer (3.0 and 39.4, respectively) or fish hydrolysate (4.3 and 42.0, respectively). The results clearly demonstrate that the emulsified agricultural admixture with tailored properties exhibits a significant crop size and quality difference compared to a standard inorganic fertilizer or conventional fish hydrolysate fertilizer.

## Claims

1. A process for producing an agricultural admixture from a selected biological recyclable stream, the process comprising the steps of:
(a) providing a biological recyclable stream using a collection system;
(b) grinding the biological recyclable stream using a first grinder and optionally a second grinder to produce a ground biological slurry;
(c) adding to said ground biological slurry one or more selected enzymes;
(d) increasing the temperature of the first ground biological slurry from ambient temperature to at least one temperature between about 35 °C and about 60 °C (about 95 °F and about 140 °F) and incubating the first ground biological slurry under constant agitation and shear at two or more temperatures between about 35 °C and about 60 °C (about 95 °F and about 140 °F), thereby producing an incubated first biological slurry comprising first incubated biological particles and a first incubated biological hydrolysate;
(e) pasteurizing the incubated ground biological slurry to kill pathogens;
(f) separating the first incubated hydrolysate into a first incubated biological hydrolysate and first incubated biological particles using one or a plurality of size-based separation methods; and
(g) reducing the fat content of the pasteurized first incubated hydrolysate to a range of between 1 to 4 wt% by tricanter centrifugation to form a centrifuged biological hydrolysate.

2. The process of claim 1, further comprising the steps of:
(i) stabilizing the centrifuged biological hydrolysate to form a stabilized aqueous hydrolysate; and
(ii) emulsifying the stabilized aqueous hydrolysate to form an emulsified agricultural admixture, and optionally adding a dispersant to the emulsified agricultural admixture.

3. The process of claim 2 wherein:
(a) said process further comprises the step of concentrating the emulsified agricultural admixture; optionally wherein concentrating the liquid agricultural admixture is performed using a vibratory filter, vacuum drum, vacuum evaporator, drum dryer, spray dryer, paddle dryer, rotary dryer, or extruder;
(b) stabilizing the centrifuged biological hydrolysate by adding a stabilization agent selected from: inorganic acid, organic acid, organic preservative, or inorganic preservative;
(c) emulsifying the stabilized aqueous hydrolysate with a high-shear mixer; or
(d) said process further comprises adding a second or more biological recyclable stream to the stabilized aqueous hydrolysate.

4. The process of claim 1, wherein:
(a) the process further comprises the step of separating the first incubated biological particles into dewatered biological particles and a recycled liquid fraction;
(b) the separated first incubated biological particles are added to a second or more biological recyclable waste stream which is processed according to the steps of claim 1;
(c) step (g) of claim 1 further comprises separating the centrifuged oil into food usable oil and a food unusable oil;
(d) the one or a plurality of size-based separation methods comprises the use of a coarse filter, a fine filter, or both;
(e) said process further comprises the step of adding an anti-oxidant, anti-caking agent, or both, to the dried, solid biological slurry, the milled or pelletized product, or an animal provender; or
(f) said process further comprises the step of adding amino acids to the resulting products.

5. The process of claim 1 or claim 2, wherein the process further comprises:
(a) performing the steps of claim 1 or claim 2, respectively, with a second biological hydrolysate,
(b) combining a centrifuged biological hydrolysate produced from the first biological hydrolysate with a centrifuged biological hydrolysate produced from the second biological hydrolysate.

6. The centrifuged biological hydrolysate produced by the process of claim 1.

7. The emulsified agricultural admixture produced by the process of claim 2.

8. The dewatered biological particles formed by the process of claim 4(a).

9. The concentrated emulsified agricultural admixture produced by the process of claim 3(a).

10. The process of claim 1, wherein:
(a) the selected enzyme is selected from: at least one enzyme to digest proteins, at least one enzyme to digest fats and lipids, and at least one enzyme to digest cellulosic material or at least one enzyme to digest other carbohydrates; optionally wherein the selected enzyme is selected from the group consisting of: xylanase, asparaginase, cellulase, hemicellulase, glumayase, beta-glumayase (endo-1,3(4)-), urease, protease, lipase, amylase, phytase, phosphatase, aminopeptidase, amylase, carbohydrase, carboxypeptidase, catalase, chitinase, cutinase, cyclodextrin glycosyltransferase, deoxyribonuclease, esterase, alpha-galactosidase, beta-galactosidase, glucoamylase, alpha-amylase, alpha-glucosidase, beta-glucosidase, haloperoxidase, invertase, laccase, mannosidase, oxidase, glucose oxidase, pectinolytic enzyme, pectinesterase, peptidoglutaminase, peroxidase, polyphenoloxidase, proteolytic enzyme, protease, ribonuclease, thioglucosidase and transglutaminase; or
(b) the biological recyclable stream is selected from: blood or blood meal, bone or bone meal, feather or feather meal, manure, culled vegetable or fruit recyclables, vegetables containing oils, grape pomace, tomato pomace, olive pomace, fresh food recyclables, fish recyclables, carbohydrate recyclables, bread crumbs, bakery waste, nut hulls, almond hulls, walnut hulls, pistachio hulls, soymeal, pomace, and distiller's grains and bakery recyclables.

11. An agricultural admixture of claim 1, wherein the agricultural admixture is produced by grinding a biological recyclable stream to produce a ground biological slurry, heating and incubating the ground biological slurry with one or more selected enzymes with constant agitation and shear, pasteurizing the incubated mixture to produce a biological hydrolysate, reducing the fats content in the biological hydrolysate aqueous phase to a range of 1 to 4 wt%, and stabilization of the aqueous phase by adding a stabilizer selected from an inorganic acid, an organic acid, an inorganic preservative, or an organic preservative, to produce a stabilized agricultural admixture.

12. The process of claim 1, further comprising the steps of:
(h) drying the centrifuged biological hydrolysate to form a dried, solid biological slurry; and
(i) milling the solid biological slurry to form a powdered, dried biological slurry or pelletizing the dried, solid biological slurry to form dried biological slurry pellets.

13. The process of claim 12, further comprising the steps of combining or blending the powdered, dried biological slurry or dried biological slurry pellets with a carbohydrate recyclable stream to form animal provender.

14. A method of increasing animal weight, or increasing the conversion rate of animal provender into animal weight, the method comprising providing to the animal a formulation comprising the animal provender produced by the process of claim 13.

## Patentansprüche

1. Vorgang zum Produzieren eines landwirtschaftlichen Zusatzstoffes aus einem ausgewählten biologisch wiederverwertbaren Strom, wobei der Vorgang die Schritte umfasst:
(a) Bereitstellen eines biologisch wiederverwertbaren Stroms unter Verwendung eines Sammelsystems;
(b) Mahlen des biologisch wiederverwertbaren Stroms unter Verwendung einer ersten Mühle und optional einer zweiten Mühle, um eine gemahlene biologische Aufschlämmung zu produzieren;
(c) Zugeben eines oder mehrerer ausgewählter Enzyme zu der gemahlenen biologischen Aufschlämmung;
(d) Erhöhen der Temperatur der ersten gemahlenen biologischen Aufschlämmung von Umgebungstemperatur auf wenigstens eine Temperatur zwischen etwa 35 °C und etwa 60 °C (etwa 95 °F und etwa 140 °F) und Inkubieren der ersten gemahlenen biologischen Aufschlämmung unter konstantem Rühren und Scheren bei zwei oder mehr Temperaturen zwischen etwa 35 °C und etwa 60 °C (etwa 95 °F und etwa 140 °F), wobei dadurch eine inkubierte erste biologische Aufschlämmung produziert wird, die erste inkubierte biologische Partikel und ein erstes inkubiertes biologisches Hydrolysat umfasst;
(e) Pasteurisieren der inkubierten gemahlenen biologischen Aufschlämmung, um Krankheitserreger abzutöten;
(f) Trennen des ersten inkubierten Hydrolysats in ein erstes inkubiertes biologisches Hydrolysat und erste inkubierte biologische Partikel unter Verwendung eines oder einer Mehrzahl von auf Größe basierenden Trennverfahren; und
(g) Reduzieren des Fettgehalts des pasteurisierten ersten inkubierten Hydrolysats auf einen Bereich zwischen 1 und 4 Gew.-% durch Tricanterzentrifugation, um ein zentrifugiertes biologisches Hydrolysat auszubilden.

2. Vorgang nach Anspruch 1, das ferner die Schritte umfasst:
(i) Stabilisieren des zentrifugierten biologischen Hydrolysats, um ein stabilisiertes wässriges Hydrolysat auszubilden; und
(ii) Emulgieren des stabilisierten wässrigen Hydrolysats, um einen emulgierten landwirtschaftlichen Zusatzstoff auszubilden, und optionales Zugeben eines Dispersionsmittels zu dem emulgierten landwirtschaftlichen Zusatzstoff.

3. Vorgang nach Anspruch 2, wobei:
(a) der Vorgang ferner den Schritt des Konzentrierens des emulgierten landwirtschaftlichen Zusatzstoffes umfasst; optional wobei das Konzentrieren des emulgierten landwirtschaftlichen Zusatzstoffes unter Verwendung eines Vibrationsfilters, einer Vakuumtrommel, eines Vakuumverdampfers, eines Trommeltrockners, eines Sprühtrockners, eines Schaufeltrockners, eines Rotationstrockners oder eines Extruders durchgeführt wird;
(b) Stabilisieren des zentrifugierten biologischen Hydrolysats durch Zugeben eines Stabilisierungsmittels, ausgewählt aus: anorganischer Säure, organischer Säure, organischem Konservierungsmittel oder anorganischem Konservierungsmittel;
(c) Emulgieren des stabilisierten wässrigen Hydrolysats mit einem Hochschermischer; oder
(d) der Vorgang ferner das Zugeben eines zweiten oder mehrerer biologisch wiederverwertbarer Ströme zu dem stabilisierten wässrigen Hydrolysat umfasst.

4. Vorgang nach Anspruch 1, wobei:
(a) der Vorgang ferner den Schritt des Trennens der ersten inkubierten biologischen Partikel in entwässerte biologische Partikel und eine recycelte flüssige Fraktion umfasst;
(b) die getrennten ersten inkubierten biologischen Partikel einem zweiten oder mehreren biologischen wiederverwertbaren Abfallströmen zugegeben werden, die nach den Schritten von Anspruch 1 verarbeitet werden;
(c) Schritt (g) von Anspruch 1 ferner das Trennen des zentrifugierten Öls in ein für Lebensmittel verwendbares Öl und ein für Lebensmittel unbrauchbares Öl umfasst;
(d) das eine oder eine Mehrzahl von größenbasierten Trennverfahren die Verwendung eines Grobfilters, eines Feinfilters oder beider umfasst;
(e) der Vorgang ferner den Schritt des Zugebens eines Antioxidationsmittels, eines Antibackmittels oder von beidem zu der getrockneten, festen biologischen Aufschlämmung, dem gemahlenen oder pelletierten Produkt oder einem Tierfuttermittel umfasst; oder
(f) der Vorgang ferner den Schritt des Zugebens von Aminosäuren zu den resultierenden Produkten umfasst.

5. Vorgang nach Anspruch 1 oder 2, wobei der Vorgang ferner umfasst:
(a) Durchführen der Schritte nach Anspruch 1 beziehungsweise Anspruch 2 mit einem zweiten biologischen Hydrolysat,
(b) Kombinieren eines zentrifugierten biologischen Hydrolysats, das aus dem ersten biologischen Hydrolysat produziert wurde, mit einem zentrifugierten biologischen Hydrolysat, das aus dem zweiten biologischen Hydrolysat produziert wurde.

6. Zentrifugiertes biologisches Hydrolysat, das nach dem Vorgang von Anspruch 1 produziert wird.

7. Emulgierter landwirtschaftlicher Zusatzstoff, der nach dem Vorgang von Anspruch 2 produziert wird.

8. Entwässerte biologische Partikel, die durch den Vorgang nach Anspruch 4(a) ausgebildet werden.

9. Konzentrierter emulgierter landwirtschaftlicher Zusatzstoff, der nach dem Vorgang des Anspruchs 3(a) produziert wird.

10. Vorgang nach Anspruch 1, wobei:
(a) das ausgewählte Enzym ausgewählt ist aus: wenigstens einem Enzym zum Verdauen von Proteinen, wenigstens einem Enzym zum Verdauen von Fetten und Lipiden und wenigstens einem Enzym zum Verdauen von zellulosehaltigem Material oder wenigstens einem Enzym zum Verdauen anderer Kohlenhydrate; optional wobei das ausgewählte Enzym aus der Gruppe ausgewählt ist, bestehend aus: Xylanase, Asparaginase, Cellulase, Hemicellulase, Glumayase, beta-Glumayase (endo-1,3(4)-), Urease, Protease, Lipase, Amylase, Phytase, Phosphatase, Aminopeptidase, Amylase, Carbohydrase, Carboxypeptidase, Katalase, Chitinase, Cutinase, Cyclodextrin, Glykosyltransferase, Deoxyribonuklease, Esterase, Alpha-Galaktosidase, Beta-Galactosidase, Glucoamylase, Alpha-Amylase, Alpha-Glucosidase, Beta-Glucosidase, Haloperoxidase, Invertase, Laccase, Mannosidase, Oxidase, Glucoseoxidase, pektinolytischem Enzym, Pektinesterase, Peptidoglutaminase, Peroxidase, Polyphenoloxidase, proteolytischem Enzym, Protease, Ribonuklease, Thioglucosidase und Transglutaminase; oder
(b) der biologisch verwertbare Strom ausgewählt ist aus: Blut oder Blutmehl, Knochen oder Knochenmehl, Federn oder Federmehl, Dung, ausgelesenem Gemüse oder wiederverwertbarem Obst, ölhaltigem Gemüse, Traubentrester, Tomatentrester, Oliventrester, frischen Lebensmittelwertstoffen, Fischwertstoffen, Kohlenhydratwertstoffen, Brotkrümel, Bäckereiabfällen, Nussschalen, Mandelschalen, Walnussschalen, Pistazienschalen, Sojamehl, Trester, Brennereikörnern und Backwarenwertstoffen.

11. Landwirtschaftlicher Zusatzstoff nach Anspruch 1, wobei der landwirtschaftliche Zusatzstoff durch Mahlen eines biologisch Stromwertstoffs, um eine gemahlene biologische Aufschlämmung zu produzieren, Erwärmen und Inkubieren der gemahlenen biologischen Aufschlämmung mit einem oder mehreren ausgewählten Enzymen unter ständigem Rühren und ständiger Scherung, Pasteurisieren der inkubierten Mischung, um ein biologisches Hydrolysat zu produzieren, Reduzieren des Fettgehalts in der wässrigen Phase des biologischen Hydrolysats auf einen Bereich von 1 bis 4 Gew.-% und Stabilisieren der wässrigen Phase durch Zugeben eines Stabilisators, der aus einer anorganischen Säure, einer organischen Säure, einem anorganischen Konservierungsmittel oder einem organischen Konservierungsmittel ausgewählt ist, um einen stabilisierten landwirtschaftlichen Zusatzstoff zu produzieren.

12. Vorgang nach Anspruch 1, der ferner die Schritte umfasst:
(h) Trocknen des zentrifugierten biologischen Hydrolysats, um eine getrocknete, feste biologische Aufschlämmung auszubilden; und
(i) Mahlen der festen biologischen Aufschlämmung, um eine pulverisierte, getrocknete biologische Aufschlämmung auszubilden, oder Pelletieren der getrockneten, festen biologischen Aufschlämmung, um getrocknete biologische Aufschlämmungspellets auszubilden.

13. Vorgang nach Anspruch 12, ferner umfassend die Schritte des Kombinierens oder Vermischens der pulverisierten, getrockneten biologischen Aufschlämmung oder der getrockneten biologischen Aufschlämmungspellets mit einem wiederverwertbaren Kohlenhydratstrom, um Tierfutter auszubilden.

14. Verfahren zum Erhöhen von Tiergewicht oder Erhöhen der Umwandlungsrate eines Tierfutters in Tiergewicht, wobei das Verfahren das Bereitstellen einer Formulierung an das Tier umfasst, die das durch den Vorgang nach Anspruch 13 produzierte Tierfutter umfasst.

## Revendications

1. Procédé de production d'un mélange agricole à partir d'un flux biologique recyclable sélectionné, le procédé comprenant les étapes consistant à :
(a) fournir un flux biologique recyclable à l'aide d'un système de collecte ;
(b) broyer le flux biologique recyclable à l'aide d'un premier broyeur et éventuellement d'un second broyeur pour produire une bouillie biologique broyée ;
(c) ajouter à ladite bouillie biologique broyée une ou plusieurs enzymes sélectionnées ;
(d) augmenter la température de la première bouillie biologique broyée de la température ambiante à au moins une température entre environ 35 °C et environ 60 °C (environ 95 °F et environ 140 °F) et incuber la première bouillie biologique broyée sous des conditions constantes d'agitation et de cisaillement à deux ou plusieurs températures entre environ 35 °C et environ 60 °C (environ 95 °F et environ 140 °F), produisant ainsi une première bouillie biologique incubée comprenant des premières particules biologiques incubées et un premier hydrolysat biologique incubé ;
(e) pasteuriser la bouillie biologique broyée incubée pour tuer les agents pathogènes ;
(f) séparer le premier hydrolysat incubé en un premier hydrolysat biologique incubé et en premières particules biologiques incubées en utilisant un ou plusieurs procédés de séparation basés sur la taille ; et
(g) réduire la teneur en matières grasses du premier hydrolysat incubé pasteurisé à une plage comprise entre 1 et 4 % en poids par centrifugation au tricanter pour former un hydrolysat biologique centrifugé.

2. Procédé selon la revendication 1, comprenant en outre les étapes consistant à :
(i) stabiliser l'hydrolysat biologique centrifugé pour former un hydrolysat aqueux stabilisé ; et
(ii) émulsionner l'hydrolysat aqueux stabilisé pour former un mélange agricole émulsifié, et éventuellement ajouter un dispersant au mélange agricole émulsifié.

3. Procédé selon la revendication 2, dans lequel :
(a) ledit procédé comprend en outre l'étape de concentration du mélange agricole émulsifié ; éventuellement dans lequel la concentration du mélange agricole liquide est effectuée à l'aide d'un filtre vibrant, d'un tambour sous vide, d'un évaporateur sous vide, d'un séchoir à tambour, d'un séchoir par pulvérisation, d'un séchoir à pales, d'un séchoir rotatif ou d'une extrudeuse ;
(b) stabiliser l'hydrolysat biologique centrifugé en ajoutant un agent de stabilisation choisi parmi : un acide inorganique, un acide organique, un conservateur organique ou un conservateur inorganique ;
(c) émulsionner l'hydrolysat aqueux stabilisé avec un mélangeur à cisaillement élevé ; ou
(d) ledit procédé comprend en outre l'ajout d'un second ou plusieurs flux biologiques recyclables à l'hydrolysat aqueux stabilisé.

4. Procédé selon la revendication 1, dans lequel :
(a) le procédé comprend en outre l'étape de séparation des premières particules biologiques incubées en particules biologiques déshydratées et en une fraction liquide recyclée ;
(b) les premières particules biologiques incubées séparées sont ajoutées à un second ou plusieurs flux de déchets biologiques recyclables qui sont traités selon les étapes de la revendication 1 ;
(c) l'étape (g) de la revendication 1 comprend en outre la séparation de l'huile centrifugée en une huile alimentaire utilisable et en une huile alimentaire inutilisable ;
(d) l'un ou plusieurs procédés de séparation basés sur la taille comprennent l'utilisation d'un filtre grossier, d'un filtre fin ou des deux ;
(e) ledit procédé comprend en outre l'étape consistant à ajouter un antioxydant, un agent anti-mottant, ou les deux, à la bouillie biologique solide séchée, au produit broyé ou granulé, ou à une nourriture animale ; ou
(f) ledit procédé comprend en outre l'étape d'addition d'acides aminés aux produits résultants.

5. Procédé selon la revendication 1 ou la revendication 2, dans lequel le procédé comprend en outre les étapes consistant à :
(a) effectuer les étapes de la revendication 1 ou de la revendication 2, respectivement, avec un second hydrolysat biologique,
(b) combiner un hydrolysat biologique centrifugé produit à partir du premier hydrolysat biologique avec un hydrolysat biologique centrifugé produit à partir du second hydrolysat biologique.

6. Hydrolysat biologique centrifugé produit par le procédé selon la revendication 1.

7. Mélange agricole émulsifié produit par le procédé selon la revendication 2.

8. Particules biologiques déshydratées formées par le procédé selon la revendication 4(a).

9. Mélange agricole émulsifié concentré produit par le procédé selon la revendication 3(a).

10. Procédé selon la revendication 1, dans lequel :
(a) l'enzyme sélectionnée est sélectionnée parmi : au moins une enzyme pour digérer les protéines, au moins une enzyme pour digérer les graisses et les lipides, et au moins une enzyme pour digérer la matière cellulosique ou au moins une enzyme pour digérer d'autres glucides ; éventuellement dans lequel l'enzyme sélectionnée est sélectionnée dans le groupe constitué par : xylanase, asparaginase, cellulase, hémicellulase, glumayase, bêta-glumayase (endo-1,3(4)-), uréase, protéase, lipase, amylase, phytase, phosphatase, aminopeptidase, amylase, carbohydrase, carboxypeptidase, catalase, chitinase, cutinase, cyclodextrine glycosyltransférase, désoxyribonucléase, estérase, alpha-galactosidase , bêta-galactosidase, glucoamylase, alpha-amylase, alpha-glucosidase, bêta-glucosidase, haloperoxydase, invertase, laccase, mannosidase, oxydase, glucose oxydase, enzyme pectinolytique, pectinestérase, polyphénoloxydase, enzyme protéolytique, protéase, ribonucléase, thioglucosidase et transglutaminase ; ou
(b) le flux biologique recyclable est sélectionné parmi : le sang ou la farine de sang, les os ou la farine d'os, les plumes ou la farine de plumes, le fumier, les légumes ou fruits recyclables, les légumes contenant des huiles, le marc de raisin, le marc de tomate, le marc d'olive, les aliments frais recyclables, les poissons recyclables, les glucides recyclables, la chapelure, les déchets de boulangerie, les coques de noix, les coques d'amandes, les coques de noyer, les coques de pistache, la farine de soja, le marc, les drêches de distillerie et les produits recyclables de boulangerie.

11. Mélange agricole selon la revendication 1, dans lequel le mélange agricole est produit en broyant un flux biologique recyclable pour produire une bouillie biologique broyée, en chauffant et en incubant la bouillie biologique broyée avec une ou plusieurs enzymes sélectionnées avec une agitation et un cisaillement constants, en pasteurisant le mélange incubé pour produire un hydrolysat biologique, en réduisant la teneur en matières grasses dans la phase aqueuse de l'hydrolysat biologique à une plage de 1 à 4 % en poids, et en stabilisant la phase aqueuse en ajoutant un stabilisant choisi parmi un acide inorganique, un acide organique, un conservateur inorganique, ou un conservateur organique, pour produire un mélange agricole stabilisé.

12. Procédé selon la revendication 1, comprenant en outre les étapes consistant à :
(h) sécher l' hydrolysat biologique centrifugé pour former une bouillie biologique solide séchée; et
(i) broyer la bouillie biologique solide pour former une bouillie biologique séchée en poudre ou granuler la bouillie biologique solide séchée pour former des granulés de bouillie biologique séchée.

13. Procédé selon la revendication 12, comprenant en outre les étapes de combinaison ou de mélange de la bouillie biologique séchée en poudre ou des granulés de bouillie biologique séchée avec un flux de glucides recyclables pour former de la nourriture pour animaux.

14. Procédé d'augmentation du poids animal, ou d'augmentation du taux de conversion de la nourriture animale en poids animal, le procédé comprenant la fourniture à l'animal d'une formulation comprenant la nourriture animale produite par le procédé selon la revendication 13.
